# EUROPEAN PATENT SPECIFICATION

(11) **EP 1 476 138 B1**
(45) Date of publication and mention of the grant of the patent: **07.12.2011**
(21) Application number: 03743147.5
(22) Date of filing: 21.02.2003
(51) Int. Cl.: A61K 9/22, A61K 9/32, A61K 9/36

(54) **MODIFIED RELEASE FORMULATIONS OF AT LEAST ONE FORM OF TRAMADOL**
FORMULIERUNGEN MIT MODIFIZIERTER FREISETZUNG VON MINDESTENS EINER FORM VON TRAMADOL
FORMULATIONS A LIBERATION MODIFIEE D'AU MOINS UNE FORME DE TRAMADOL

(30) Priority: 21.02.2002 US 357851 P
(43) Date of publication of application: 17.11.2004
(73) Proprietor: BIOVAIL LABORATORIES INTERNATIONAL SRL, Collymore Rock Saint Michael (BB)
(72) Inventor: SETH, Pawan, Irvine, CA 92612 (US); MAES, Paul, J., Toronto, Ontario M5R 2V1 (CA)
(74) Representative: Gates, Marie Christina Esther
(86) International application number: PCT/US2003/004866
(87) International publication number: WO 2003/072025

(56) References cited:
- EP-A- 1 020 186
- US-B1- 6 254 887

## Description

### FIELD OF INVENTION

The present invention relates to modified release formulations for oral administration, to processes for their preparation and to their medical use. In particular, the present invention relates to modified release formulations of at least one form of tramadol, selected from the group consisting of tramadol, racemic mixtures thereof, enantiomers thereof, pharmaceutically acceptable salts thereof and combinations thereof.

### BACKGROUND OF THE INVENTION

Tramadol, which was first described in United States Patent No. 3,652,589, is a class of analgesic cycloalkanol-substituted phenol esters having a basic amine group in the cycloalkyl ring and having the chemical name *trans*-(±)-2-[(dimethylamino)methyl]-1-(3-methoxyphenyl)cyclohexanol. Tramadol is believed to produce an analgesic effect through a mechanism that is neither fully opioid-like nor non-opioid-like because clinical data suggests that tramadol lacks many of the typical side effects of opioid antagonists such as respiratory depression, constipation, tolerance and abuse liability but can produce hot flashes and sweating. Due to the combination of non-opioid and opioid activity, tramadol is a very unique analgesic and many attempts have been made to prepare oral formulations of the drug.

Conventional or immediate release preparations in the form of tablets, capsules, drops and suppositories containing tramadol, or more particularly its hydrochloride salt, have been commercially available for many years for use in the treatment of moderate to severe pain. The clinical efficacy of immediate release tramadol preparations has been well established in numerous single dose and multiple dose studies, with 70% to 90% of patients obtaining satisfactory pain relief depending on the etiology of the pain. Immediate release tramadol preparations have demonstrated efficacy in obstetrical, gynecologic, orthopedic, abdominal and oral surgery. Immediate release tramadol preparations have also been studied in long-term clinical trials in patients with chronic pain of varying etiology, including low-back pain, osteoarthritis, cancer pain, neuropathic pain and orthopedic pain.

Immediate release tramadol preparations, however, do not provide a controlled release of the tramadol. For example, an immediate release oral formulation of tramadol is commercially available in the United States, from McNeil Pharmaceuticals under the tradename ULTRAM® as tramadol hydrochloride tablets. The 53rd Edition of the Physician's Desk Reference, copyright 1999, p. 2255, states that peak plasma levels of tramadol for the ULTRAM® product occur at about 1.6 hours after a single oral dose (100 mg) and at about 2.3 hours after multiple oral dosing (100 mg q.i.d). The short elimination half-life of tramadol necessitates dosing of patients with immediate release tramadol preparations every 4-6 hours in order to maintain optimal levels of analgesia in chronic pain.

To overcome the difficulties associated with the required dosing frequency of immediate release tramadol preparations, various attempts have been made to formulate tramadol into modified release formulations. For example, see United States Patent Nos. 5,395,626, 5,474,786, 5,645,858, 5,478,577, 5,591,452, 6,254,887, 5,601,842, 5,580,578, 5,639,476, 5,811,126, 5,849,240, 5,891,471, 5,965,163, 5,958,452, 5,965,161, 5,478,577, 5,580,578, 5,648,096, 5,672,360, 5,811,126, 5,879,705, 5,968,551, 5,980,941, 6,068,858, 6,077,532, 6,077,533 and 6,254,887. Such modified release tramadol preparations purport to control the rate of release of tramadol within the gastrointestinal tract, with the purported result that tramadol is delivered at a specific, predetermined rate.

EP 1 020 186 discloses tablet cores comprising tramadol-hydrochloride coated with ethylcellulose.

### SUMMARY OF THE INVENTION

It is an object of the present invention to prepare a modified release pharmaceutical composition comprising at least one form of tramadol, according to claim 1.

It is a further object of the present invention to prepare a modified release pharmaceutical composition comprising at least one form of tramadol wherein the composition is suitable for oral administration to patients which provides effective relief from pain.

Further and other objects of the present invention will be realized by those skilled in the art from the following summary of the invention and detailed description of embodiments thereof.

In accordance with one aspect of the present invention, there is provided a modified release pharmaceutical composition for oral administration, suitable for once daily dosing, comprising at least one form of tramadol selected from the group consisting of tramadol, racemic mixtures thereof, enantiomers thereof, pharmaceutically acceptable salts thereof and combinations thereof, in combination with at least one pharmaceutically acceptable excipient, wherein the pharmaceutical composition when orally administered to a patient, induces a statistically significant lower mean fluctuation index in the plasma than an immediate release composition of the at least one form of tramadol while maintaining bioavailability substantially equivalent to that of the immediate release composition.

In accordance with another aspect of the present invention, there is provided a modified release pharmaceutical composition for oral administration, suitable for once daily dosing, comprising at least one form of tramadol selected from the group consisting of tramadol, racemic mixtures thereof enantiomers thereof, pharmaceutically acceptable salts thereof and combinations thereof, in combination with at least one pharmaceutically acceptable excipient, wherein the pharmaceutical composition when orally administered to a patient, produces a mean maximum plasma concentration (Cₘₐₓ) of the at least one form of tramadol that is lower than that produced by an immediate release pharmaceutical composition of the at least one form of tramadol, and the area under the concentration-time curve (AUC) and the mean minimum plasma concentration (Cₘᵢₙ) are substantially equivalent to that of the immediate release pharmaceutical composition.

In accordance with another aspect of the present invention, there is provided a modified release pharmaceutical composition for oral administration, suitable for once daily dosing, comprising at least one form of tramadol selected from the group consisting of tramadol, racemic mixtures thereof, enantiomers thereof, pharmaceutically acceptable salts thereof and combinations thereof, in combination with at least one pharmaceutically acceptable excipient, wherein the composition, when orally administered to a patient, produces a mean maximum plasma concentration (Cₘₐₓ) of the at least one form of tramadol having both a maximum concentration (Cₘₐₓ) and an area under a plasma concentration vs. time curve (AUC) within the range from about -20% to about +25% of that produced by an immediate release pharmaceutical composition of the at least one form of tramadol.

In an embodiment of the present invention, the at least one form of tramadol is tramadol hydrochloride and the immediate release pharmaceutical composition is the subject of the United States Food and Drug Administration Approved New Drug Application number N20281, N75963, N75980, N75974, N76003, N75968, N75983, N76100, N75986, N75960, N75982, N75977, N75981, or N75962.

In accordance with another aspect of the present invention, there is provided a modified release pharmaceutical composition for oral administration, suitable for once daily dosing, comprising at least one form of tramadol selected from the group consisting of tramadol, racemic mixtures thereof, enantiomers thereof, pharmaceutically acceptable salts thereof and combinations thereof, in combination with at least one pharmaceutically acceptable excipient, the composition exhibiting an *in vitro* dissolution profile (measured using the USP Basket Method at 75 rpm in 900 ml 0.1 N HCl at 37 °C) such that after 2 hours, from about 0% up to about 30% (by weight) of the at least one form of tramadol is released, after 4 hours, from about 5% to about 22% (by weight) of the at least one form of tramadol is released, after 6 hours, from about 15% to about 38% (by weight) of the at least one form of tramadol is released, after 8 hours, more than about 40% (by weight) of the at least one form of tramadol is released.

In accordance with another aspect of the present invention, there is provided a modified release pharmaceutical composition for oral administration, suitable for once daily dosing, comprising at least one form of tramadol selected from the group consisting of tramadol, racemic mixtures thereof, enantiomers thereof, pharmaceutically acceptable salts thereof and combinations thereof, in combination with at least one pharmaceutically acceptable excipient, the composition exhibiting an *in vitro* dissolution profile (measured using the USP Basket Method at 75 rpm in 900 ml 0.1 N HCl at 37 °C) such that after 2 hours, from about 2% to about 10% (by weight) of the at least one form of tramadol is released, after 4 hours, from about 12% to about 20% (by weight) of the at least one form of tramadol is released, after 6 hours, from about 30% to about 38% (by weight) of the at least one form of tramadol is released, after 8 hours, from about 48% to about 56% (by weight) of the at least one form of tramadol is released, after 10 hours, from about 64% to about 72% (by weight) of the at least one form of tramadol is released, and after 12 hours, more than about 76% (by weight) of the at least one form of tramadol is released.

In accordance with another aspect of the present invention, there is provided a modified release pharmaceutical composition for oral administration, suitable for once daily dosing, comprising:
(i) a core comprising at least one form of tramadol selected from the group consisting of tramadol, racemic mixtures thereof, enantiomers thereof, pharmaceutically acceptable salts thereof and combinations thereof and at least one pharmaceutically acceptable excipient; and
(ii) a coating comprising at least one water-insoluble, water-permeable film-forming polymer, at least one plasticizer and at least one water-soluble polymer.

In accordance with another aspect of the present invention, there is provided a modified release pharmaceutical composition comprising:
(i) a core comprising at least one form of tramadol selected from the group consisting of tramadol, racemic mixtures thereof, enantiomers thereof, pharmaceutically acceptable salts thereof, and combinations thereof and at least one pharmaceutically acceptable excipient; and
(ii) a coating comprising at least one water-insoluble, water-permeable film-forming polymer, at least one plasticizer and at least one water-soluble polymer, wherein the proportion of the at least one water-insoluble, water-permeable film-forming polymer varies from about 20% to about 90% of the coating dry weight, the proportion of the at least one plasticizer varies from about 5% to about 30% of the coating dry weight, and the proportion of the at least one water-soluble polymer varies from about 10% to about 75% of the coating dry weight.

In accordance with another aspect of the present invention, there is provided a modified release pharmaceutical composition for oral administration, suitable for once daily dosing, comprising:
(i) a core comprising at least one form of tramadol selected from the group consisting of tramadol, racemic mixtures thereof, enantiomers thereof, pharmaceutically acceptable salts thereof and combinations thereof and at least one pharmaceutically acceptable excipient; and
(ii) a coating comprising at least one water-insoluble, water-permeable film-forming polymer, at least one plasticizer and at least one water-soluble polymer, wherein the composition exhibits an *in vitro* dissolution profile (measured using the USP Basket Method at 75 rpm in 900 ml 0.1 N HCl at 37 °C) such that after 2 hours, from about 0% up to about 30% (by weight) of the at least one form of tramadol is released, after 4 hours, from about 5% to about 22% (by weight) of the at least one form of tramadol is released, after 6 hours, from about 15% to about 38% (by weight) of the at least one form of tramadol is released, and after 8 hours, more than about 40% (by weight) of the at least one form of tramadol is released.

In accordance with another aspect of the present invention, there is provided a modified release pharmaceutical composition for oral administration, suitable for once daily dosing, comprising:
(i) a core comprising at least one form of tramadol selected from the group consisting of tramadol, racemic mixtures thereof, enantiomers thereof, pharmaceutically acceptable salts thereof and combinations thereof and at least one pharmaceutically acceptable excipient; and
(ii) a coating comprising at least one water-insoluble, water-permeable film-forming polymer, at least one plasticizer and at least one water-soluble polymer, wherein the composition exhibits an *in vitro* dissolution profile (measured using the USP Basket Method at 75 rpm in 900 ml 0.1 N HCl at 37 °C) such that after 2 hours, from about 2% to about 10% (by weight) of the at least one form of tramadol is released, after 4 hours, from about 12% to about 20% (by weight) of the at least one form of tramadol is released, after 6 hours, from about 30% to about 38% (by weight) of the at least one form of tramadol is released, after 8 hours, from about 48% to about 56% (by weight) of the at least one form of tramadol is released, after 10 hours, from about 64% to about 72% (by weight) of the at least one form of tramadol is released, and after 12 hours, more than about 76% of the at least one form of tramadol is released.

In accordance with another aspect of the present invention, there is provided a modified release pharmaceutical composition for oral administration, suitable for once daily dosing, comprising:
(i) a core comprising at least one form of tramadol selected from the group consisting of tramadol, racemic mixtures thereof, enantiomers thereof, pharmaceutically acceptable salts thereof, and combinations thereof and at least one pharmaceutically acceptable excipient; and
(ii) a coating comprising at least one water-insoluble, water-permeable film-forming polymer, at least one plasticizer and at least one water-soluble polymer, wherein the proportion of the at least one water-insoluble, water-permeable film-forming polymer varies from about 20% to about 90% of the coating dry weight, the proportion of the at least one plasticizer varies from about 5% to about 30% of the coating dry weight, and the proportion of the at least one water-soluble polymer varies from about 10% to about 75% of the coating dry weight, and wherein the composition exhibits an *in vitro* dissolution profile (measured using the USP Basket Method at 75 rpm in 900 ml 0.1 N HCl at 37 °C) such that after 2 hours, from about 0% up to about 30% (by weight) of the at least one form of tramadol is released, after 4 hours, from about 5% to about 22% (by weight) of the at least one form of tramadol is released, after 6 hours, from about 15 % to about 38 % (by weight) of the at least one form of tramadol is released, and after 8 hours, more than about 40% (by weight) of the at least one form of tramadol is released.

In accordance with another aspect of the present invention, there is provided a modified release pharmaceutical composition for oral administration, suitable for once daily dosing, comprising:
(i) a core comprising at least one form of tramadol selected from the group consisting of tramadol, racemic mixtures thereof, enantiomers thereof, pharmaceutically acceptable salts thereof, and combinations thereof and at least one pharmaceutically acceptable excipient; and
(ii) a coating comprising at least one water-insoluble, water-permeable film-forming polymer, at least one plasticizer and at least one water-soluble polymer, wherein the proportion of the at least one water-insoluble, water-permeable film-forming polymer varies from about 20% to about 90% of the coating dry weight, the proportion of the at least one plasticizer varies from about 5% to about 30% of the coating dry weight, and the proportion of the at least one water-soluble polymer varies from about 10% to about 75% of the coating dry weight, and wherein the composition exhibits an in vitro dissolution profile (measured using the USP Basket Method at 75 rpm in 900 ml 0.1 N HCl at 37°C) such that after 2 hours, from about 2% to about 10% (by weight) of the at least one form of tramadol is released, after 4 hours, from about 12% to about 20% (by weight) of the at least one form of tramadol is released, after 6 hours, from about 30% to about 38% (by weight) of the at least one form of tramadol is released, after 8 hours, from about 48% to about 56% (by weight) of the at least one form of tramadol is released, after 10 hours, from about 64% to about 72% (by weight) of the at least one form of tramadol is released, and after 12 hours, more than about 76% (by weight) of the at least one form of tramadol is released.

In accordance with another aspect of the present invention, there is provided a modified release pharmaceutical composition for oral administration, suitable for once daily dosing, comprising at least one form of tramadol selected from the group consisting of tramadol, racemic mixtures thereof, enantiomers thereof, pharmaceutically acceptable salts thereof and combinations thereof, in combination with at least one pharmaceutically acceptable excipient, wherein the pharmaceutical composition, when orally administered to a patient, provides a mean maximum plasma concentration (Cₘₐₓ) of the at least one form of tramadol from about 80 ng/ ml to about 500 ng/ ml.

In accordance with another aspect of the present invention, there is provided a modified release pharmaceutical composition for oral administration, suitable for once daily dosing, comprising at least one form of tramadol selected from the group consisting of tramadol, racemic mixtures thereof, enantiomers thereof, pharmaceutically acceptable salts thereof and combinations thereof, in combination with at least one pharmaceutically acceptable excipient, wherein the pharmaceutical composition, when orally administered to a patient, provides a time to mean peak plasma concentration (Tₘₐₓ) of the at least one form of tramadol ranging from about 4 hours to about 14 hours.

In accordance with another aspect of the present invention, there is provided a modified release pharmaceutical composition for oral administration, suitable for once daily dosing, comprising at least one form of tramadol selected from the group consisting of tramadol, racemic mixtures thereof, enantiomers thereof, pharmaceutically acceptable salts thereof and combinations thereof, in combination with at least one pharmaceutically acceptable excipient, wherein the pharmaceutical composition, when administered to a patient, provides a plasma concentration time curve with an area under the curve (AUC) ranging from about 1000 ng.hr/ml to about 10000 ng.hr/ml.

In an embodiment of the present invention, the at least one form of tramadol is present in the pharmaceutical composition in an amount effective for the management of moderate to moderately severe pain.

In an embodiment of the present invention, the pharmaceutical composition comprises from about 25 mg to about 800 mg or more of the at least one form of tramadol.

In an embodiment of the present invention, the pharmaceutical composition comprises from about 50 mg to about 400 mg or more of the at least one form of tramadol.

In an embodiment of the present invention, the pharmaceutical composition comprises from about 100 mg to about 300 mg or more of the at least one form of tramadol.

In an embodiment of the present invention, the at least one form of tramadol is tramadol hydrochloride.

In an embodiment of the present invention, the core is in a form selected from the group consisting of a granule, a spheroid, a microsphere, a bead, a seed, a pellet, a microtablet, a tablet, a capsule, and combinations thereof.

In an embodiment of the present invention, the core is in the form of a tablet.

In an embodiment of the present invention, the modified release pharmaceutical composition is suitable for once daily dosing.

In an embodiment of the present invention, the at least one pharmaceutically acceptable excipient is selected from the group consisting of at least one release rate controlling pharmaceutically acceptable carrier, at least one diluent, at least one lubricant, at least one binder, at least one filler and combinations thereof.

In an embodiment of the present invention, the at least one pharmaceutically acceptable excipient is at least one diluent.

In an embodiment of the present invention, the at least one diluent is selected from the group consisting of lactose, microcrystalline cellulose, mannitol and combinations thereof.

In an embodiment of the present invention, the at least one pharmaceutically acceptable excipient is at least one lubricant.

In an embodiment of the present invention, the at least one lubricant is selected from the group consisting of stearic acid, magnesium stearate, glyceryl behenate, talc, sodium stearyl fumarate and combinations thereof.

In an embodiment of the present invention, the at least one lubricant is sodium stearyl fumarate.

In an embodiment of the present invention, the at least one pharmaceutically acceptable excipient is at least one binder.

In an embodiment of the present invention, the at least one binder is selected from the group consisting of modified starch, gelatin, polyvinylpyrrolidone, polyvinyl alcohol and combinations thereof.

In an embodiment of the present invention, the at least one binder is polyvinyl alcohol.

In an embodiment of the present invention, the at least one pharmaceutically acceptable excipient is at least one filler.

In an embodiment of the present invention, the at least one filler is selected from the group consisting of lactose, microcrystalline cellulose and combinations thereof.

In an embodiment of the present invention, the at least one filler is microcrystalline cellulose.

There are at least three types of modified release pharmaceutical compositions in the pharmaceutical art; namely those that are delayed release, those that are extended release, and those that are both delayed and extended release. Delayed release pharmaceutical compositions are often designed to prevent drug release in the upper part of the gastrointestinal tract. Modified release coatings used to prepare this type of pharmaceutical composition are commonly called enteric coatings in the pharmaceutical art. Extended release pharmaceutical compositions are designed to extend drug release over a period of time, a result which is often achieved by the application of a sustained or controlled release coating.

In an embodiment of the present invention, the modified release pharmaceutical composition is an extended release pharmaceutical composition.

In an embodiment of the present invention, the at least one pharmaceutically acceptable excipient is at lease one release rate controlling pharmaceutically acceptable carrier.

In an embodiment of the present invention, the at least one release rate controlling pharmaceutically acceptable carrier may be incorporated into a matrix along with the at least one form of tramadol and/or may be applied as a release rate controlling coating.

In an embodiment of the present invention, the matrix is a normal release matrix having a coating which provides for modified release of the at least one form of tramadol.

In an embodiment of the present invention, the at least one release rate controlling pharmaceutically acceptable carrier is at least one sustained release pharmaceutically acceptable carrier.

In an embodiment of the present invention, the at least one sustained release pharmaceutically acceptable carrier is at least one solid sustained release pharmaceutically acceptable carrier.

In an embodiment of the present invention, the at least one solid sustained release pharmaceutically acceptable carrier is at least one solid sustained release pharmaceutically-acceptable polymer.

In an embodiment of the present invention, the at least one solid sustained release pharmaceutically-acceptable polymer is selected from the group consisting of at least one hydrophilic water-soluble polymer, at least one hydrophobic water-insoluble polymer and combinations thereof.

In an embodiment of the present invention, the modified release coating is semi-permeable.

In an embodiment of the present invention, the modified release coating comprises at least one water-insoluble, water-permeable film-forming polymer.

In an embodiment of the present invention, the at least one water-insoluble, water-permeable film-forming polymer is ethylcellulose.

In an embodiment of the present invention, the modified release coating further comprises at least one water-soluble polymer.

In an embodiment of the present invention, the at least one water-soluble polymer is polyvinylpyrrolidone.

In an embodiment of the present invention, the modified release coating further comprises at least one plasticizer.

In an embodiment of the present invention, the at least one plasticizer is dibutyl sebacate.

In an embodiment of the present invention, the at least one water-insoluble, water-permeable film-forming polymer is ethylcellulose, the at least one water-soluble polymer is polyvinylpyrrolidone and the at least one plasticizer is dibutyl sebacate.

In an embodiment of the present invention, the modified release pharmaceutical composition is in the form of a tablet.

In accordance with another aspect of the present invention, there is provided a modified release pharmaceutical composition comprising:
(i) a core comprising at least one form of tramadol selected from the group consisting of tramadol, enantiomers thereof, pharmaceutically acceptable salts thereof and combinations thereof, polyvinyl alcohol, silicon dioxide and sodium stearyl fumarate; and
(ii) a coating comprising ethylcellulose, polyvinylpyrrolidone and dibutyl sebacate, wherein the proportion of ethylcellulose varies between about 20% and about 90% of the coating dry weight, the proportion of dibutyl sebacate varies between about 5% and about 30% of the coating dry weight, and the proportion of polyvinylpyrrolidone varies between about 10% and about 75% of the coating dry weight, and wherein the composition exhibits an *in vitro* dissolution profile (measured using the USP Basket Method at 75 rpm in 900 ml 0.1 N HCl at 37 °C) such that after 2 hours, from about 0% up to about 30% of the at least one form of tramadol is released, after 4 hours, from about 5% to about 22% of the at least one form of tramadol is released, after 6 hours, from about 15% to about 38% of the at least one form of tramadol is released, after 8 hours, more than about 40% of the at least one form of tramadol is released.

In accordance with another aspect of the present invention, there is provided a modified release pharmaceutical composition comprising:
(i) a core comprising at least one form of tramadol selected from the group consisting of tramadol, enantiomers thereof, pharmaceutically acceptable salts thereof and combinations thereof, polyvinyl alcohol, silicon dioxide and sodium stearyl fumarate; and
(ii) a coating comprising ethylcellulose, polyvinylpyrrolidone and dibutyl sebacate, wherein the proportion of ethylcellulose varies between about 20% and about 90% of the coating dry weight, the proportion of dibutyl sebacate varies between about 5% and about 30% of the coating dry weight, and the proportion of polyvinylpyrrolidone varies between about 10% and about 75% of the coating dry weight, and wherein the composition exhibits an *in vitro* dissolution profile (measured using the USP Basket Method at 75 rpm in 900 ml 0.1 N HCl at 37 °C) such that after 2 hours, from about 2% to about 10% of the at least one form of tramadol is released, after 4 hours, from about 12% to about 20% of the at least one form of tramadol is released; after 6 hours, from about 30% to about 38% of the at least one form of tramadol is released, after 8 hours, from about 48% to about 56% of the at least one form of tramadol is released, after 10 hours, from about 64% to about 72% of the at least one form of tramadol is released, and after 12 hours, more than about 76% of the at least one form of tramadol is released.

In an embodiment of the present invention, the pharmaceutical composition is further coated with an immediate release coating comprising at least one form of tramadol.

### BRIEF DESCRIPTION OF THE DRAWINGS

The present invention will be further understood from the following description with references to the drawings in which:
Figure 1 compares the *in vitro* dissolution profiles of 100 mg Tramadol HCl ER Tablets formulated according to Lot Nos. 2159, 2162 and 2165.
Figure 2 illustrates the mean plasma Tramadol concentrations (ng/ml) over time following once a day Tramadol HCl ER Tablet (100 mg x 2) formulated according to Lot Nos. 2159, 2162 and 2165 vs Ultram^{®} (50 mg x 2) q.i.d.
Figure 3 illustrates the mean plasma Desmethyltramadol concentrations (ng/ml) following once a day Tramadol HCl ER Tablet (100 mg x 2) formulated according to Lot Nos. 2159, 2162 and 2165 vs Ultram^{®} (50 mg x 2) q.i.d.
Figure 4 illustrates the mean plasma Tramadol concentrations on Day 1 following once a day Tramadol HCl ER Tablets (100 mg x 2) formulated according to Lot# 2162 for 5 Days vs. Ultram^{®} (50 mg x 2) q.i.d.
Figure 5 illustrates the mean plasma Tramadol concentrations on Day 5 following once a day Tramadol HCl ER Tablets (100 mg x 2) formulated according to Lot# 2162 for 5 Days vs. Ultram^{®} (50 mg x 2) q.i.d.
Figure 6 illustrates the mean plasma Desmethyltramadol concentrations on Day 1 following once a day Tramadol HCl ER Tablets (100 mg x 2) formulated according to Lot# 2162 for 5 Days vs. Ultram^{®} (50 mg x 2) q.i.d.
Figure 7 illustrates the mean plasma Desmethyltramadol concentrations on Day 5 following once a day Tramadol HCl ER Tablets (100 mg x 2) formulated according to Lot# 2162 for 5 Days vs. Ultram^{®} (50 mg x 2) q.i.d.
Figure 8 illustrates the mean plasma Tramadol concentrations on Day 1 following once a day Tramadol HCl ER Tablets (100 mg x 3) formulated according to Lot Nos. 2162 and 2165 for 5 Days vs. Ultram^{®} (50 mg x 2) t.i.d.
Figure 9 illustrates the mean plasma Tramadol concentrations on Day 5 following once a day Tramadol HCl ER Tablets (100 mg x 3) formulated according to Lot Nos. 2162 and 2165 for 5 Days vs. Ultram^{®} (50 mg x 2) t.i.d.
Figure 10 illustrates the mean plasma Desmethyltramadol concentrations on Day 1 following once a day Tramadol HCl ER Tablets (100 mg x 3) formulated according to Lot Nos. 2162 and 2165 for 5 Days vs. Ultram^{®} (50 mg x 2) t.i.d.
Figure 11 illustrates the mean plasma Desmethyltramadol concentrations on Day 1 following once a day Tramadol HCl ER Tablets (100 mg x 3) formulated according to Lot Nos. 2162 and 2165 for 5 Days vs. Ultram^{®} (50 mg x 2) t.i.d.
Figure 12 illustrates the *in vitro* dissolution profiles of Tramadol HCl 100 mg ER Tablets formulated according to Lot Nos.1-4.
Figure 13 illustrates the *in vitro* dissolution profile of a 200 mg Tramadol HCl ER Tablet formulated according to Example 4.
Figure 14 illustrates the *in vitro* dissolution profiles of 200 mg Tramadol HCl ER Tablets formulated according to Lot Nos. 5 to 7.
Figure 15 illustrates the comparison of the mean tramadol plasma concentration-time profiles resulting from the oral administration of Tramadol HCl 100 mg ER tablets (2 x 100 mg once a day) and Tramadol HCl 200 mg ER tablets (1 x 200 mg once a day) formulated according to an embodiment of the present invention.
Figure 16 illustrates the comparison of the mean M1 plasma concentration-time profiles resulting from the oral administration of Tramadol HCl 100 mg ER tablets (2 x 100 mg once a day) and Tramadol HCl 200 mg ER tablets (1 x 200 mg once a day) formulated according to an embodiment of the present invention.
Figure 17 illustrates the mean plasma Tramadol concentrations (ng/ml) over time after two 100 mg Tramadol HCl ER Tablets formulated according to an embodiment of the present invention or after one 200 mg Tramadol HCl ER Tablet formulated according to an embodiment of the present invention following a 10 hour overnight fast.
Figure 18 illustrates the mean plasma M1 concentrations (ng/ml) over time after two 100 mg Tramadol HCl ER Tablets formulated according to an embodiment of the present invention or after one 200 mg Tramadol HCl ER Tablet formulated according to an embodiment of the present invention following a 10 hour overnight fast.
Figure 19 illustrates the mean plasma M5 concentrations (ng/ml) over time after two 100 mg Tramadol HCl ER Tablets formulated according to an embodiment of the present invention or after one 200 mg Tramadol HCl ER Tablet formulated according to an embodiment of the present invention following a 10 hour overnight fast.
Figure 20 illustrates the mean plasma Tramadol concentrations (ng/ml) over time after a single dose of one 200 mg Tramadol HCl ER Tablet formulated according to an embodiment of the present invention under fasting or fed conditions.
Figure 21 illustrates the mean plasma M1 concentrations (ng/ml) over time after a single dose of one 200 mg Tramadol HCl ER Tablet formulated according to an embodiment of the present invention under fasting or fed conditions.
Figure 22 illustrates the mean plasma M5 concentrations (ng/ml) over time after a single dose of one 200 mg Tramadol HCl ER Tablet formulated according to an embodiment of the present invention under fasting or fed conditions.
Figure 23 compares the LS mean change from baseline to average of weeks 1-12 in arthritis pain intensity VAS scores (primary variables) for Tramadol HCl ER Tablets and placebo.
Figure 24 compares the LS mean change from baseline to different study time points in arthritis pain intensity VAS scores (primary variables) for Tramadol HCl ER Tablets and placebo.
Figure 25 compares the LS mean changes from baseline to Week 12 for the Tramadol HCl ER Tablets and placebo for each of the secondary variables.

### DETAILED DESCRIPTION OF THE PREFERRED EMBODIMENTS

The present invention consists of a controlled release pharmaceutical composition, in one, embodiment a tablet, comprising at least one form of tramadol, wherein the pharmaceutical composition comprises a core and a coating, according to claim 1.

The core comprises at least one form of tramadol selected from the group consisting of tramadol, enantiomers thereof, pharmaceutically acceptable salts thereof, and combinations thereof, in one embodiment tramadol hydrochloride; and at least one pharmaceutically acceptable excipient, in one embodiment a lubricant, a binder and/or a filler, and optionally a glidant as well as other pharmaceutically acceptable excipients.

The at least one form of tramadol used in the present invention may be any form of tramadol conventional in the pharmaceutical art. The at least one form of tramadol used in the present invention may be tramadol. The at least one form of tramadol used in the present invention may be the individually optically active enantiomers of tramadol, such as for example, (+)-tramadol and (-)-tramadol. The at least one form of tramadol used in the present may be pharmaceutically acceptable salts of tramadol. Suitable pharmaceutically acceptable salts of tramadol for use as the at least one form of tramadol according to the present invention are those conventionally known in the art such as, for example, pharmaceutically acceptable acid addition salts. Suitable pharmaceutically acceptable acid addition salts of tramadol for use as the at least one form of tramadol according to the present invention may be the hydrochloride salt, the hydrobromide salt, the hydroiodide salt, the saccharinate salt etc. In one embodiment, the at least one form of tramadol is tramadol hydrochloride.

The at least one lubricant used in the present invention may be any lubricant conventional in the pharmaceutical art. The at least one lubricant used in the present invention may be stearic acid, magnesium stearate, glyceryl behenate, talc, mineral oil (in PEG), sodium stearyl fumarate, etc. In one embodiment, the at least one lubricant is sodium stearyl fumarate.

The at least one binder used in the present invention may be any binder conventional in the pharmaceutical art. The at least one binder used in the present invention may be a water-soluble polymer, such as modified starch, gelatin, polyvinylpyrrolidone, polyvinyl alcohol, etc. In one embodiment, the at least one binder is polyvinyl alcohol.

The at least one filler used in the present invention may be any filler conventional in the pharmaceutical art. The at least one filler used in the present invention may be lactose, microcrystalline cellulose, etc. In one embodiment, the at least one filler is microcrystalline cellulose.

The at least one glidant used in the present invention may be any glidant conventional in the pharmaceutical art. In one embodiment, the at least one glidant is colloidal silicon dioxide. The colloidal silicon dioxide may suitably be, for example, AEROSIL® as supplied by Degussa. Similar colloidal silicon dioxides are also available from other suppliers. Preferably, the colloidal silicon dioxide used is AEROSIL® 200.

The above binders, lubricants, fillers, glidants, and any other pharmaceutically acceptable excipient that may be present can further be found in the relevant literature, for example in the Handbook of Pharmaceutical Additives: An International Guide to More Than 6000 Products by Trade Name, Chemical, Function, and Manufacturer; Michael and Irene Ash (Eds.); Gower Publishing Ltd.; Aldershot, Hampshire, England, 1995.

The relative amounts of ingredients in the core are preferably as follows. The proportion of the at least one form of tramadol in the core may vary between about 70% and about 98% of the core dry weight. The proportion of the at least one lubricant in the core may vary between about 0.5% and about 10% of the core dry weight. The proportion of the at least one binder or at least one filler in the core may vary between about 1% and about 25% of the core dry weight.

The manufacturing process of the core can be as follows. The at least one form of tramadol is first granulated with the at least one binder, in one embodiment a granulator, but not necessarily a fluidized bed granulator. The at least one binder is first dissolved or dispersed in a suitable solvent, in one embodiment water. The solution or suspension of the at least one binder is then sprayed onto the at least one form of tramadol in a granulator, in one embodiment a fluidized bed granulator. For example, fluidized bed granulators manufactured by Glatt (Germany) or Aeromatic (Switzerland) can be used for this operation. An alternative process can be to use a conventional or high shear mixer to proceed granulation. If necessary, the at least one form of tramadol can be mixed with a filler, prior to the granulation step. Granules once dried can be mixed with the other pharmaceutically acceptable excipients, especially with the at least one lubricant, but also with at least one glidant and any other pharmaceutically acceptable excipient suitable to improve processing. The mixture of granules (in one embodiment with the at least one lubricant), and optionally at least one glidant is pressed into tablets. Alternatively, the at least one form of tramadol and the at least one lubricant can be mixed in a granulator, in one embodiment a fluidized bed granulator, and heated to the melting point of the at least one lubricant to form granules. This mixture can then be mixed with at least one suitable filler and compressed into tablets. Also, it is possible to mix the at least one form of tramadol and the at least one lubricant (in one embodiment polyvinyl alcohol) in a granulator, in one embodiment a fluidized bed granulator, and then to press the resulting granules into tablets. Tablets can be obtained by standard techniques, in one embodiment on a (rotary) press (for example Manesty Betapress®) fitted with suitable punches. The resulting tablets are hereinafter referred as tablet cores.

These tablet cores are then coated with the semi-permeable coating designed to achieve a controlled release of the at least one form of tramadol.

The coating comprises at least one water-insoluble, water-permeable film-forming polymer, together with at least one plasticizer and at least one water-soluble polymer.

The at least one water-insoluble, water-permeable film-forming polymer used in the present invention, may be any water-insoluble, water-permeable film-forming polymer conventional in the pharmaceutical art. The at least one water-insoluble, water-permeable film-forming polymer used in the present invention may be a cellulose ether, such as ethylcellulose, a cellulose ester, such as cellulose acetate, polyvinyl alcohol, etc. In one embodiment, the at least one water-insoluble, water-permeable film-forming polymer is ethylcellulose. The ethylcellulose may suitably be, for example, ETHOCEL® as supplied by Dow Chemical Company. Similar ethylcelluloses are also available from other suppliers. Preferably, the ethylcellulose used is ETHOCEL® PR, more preferably ETHOCEL® PR100.

The at least one plasticizer used in the present invention, may be any plasticizer conventional in the pharmaceutical art. The at least one plasticizer used in the present invention may be an ester such as a citrate ester, an oil such as castor oil, a polyalkylene glycol of various molecular weights, such as polyethylene glycol. In one embodiment, the at least one plasticizer is dibutyl sebacate.

The at least one water-soluble polymer used in the present invention, may be any water-soluble polymer conventional in the pharmaceutical art. In one embodiment, the at least one water-soluble is polyvinylpyrrolidone. The polyvinylpyrrolidone may suitably be, for example, KOLLIDON® as supplied by BASF AG. Similar polyvinylpyrrolidones are also available from other suppliers. Preferably, the polyvinylpyrrolidone used is KOLLIDON® 90F.

Other pharmaceutically acceptable excipients can be used in the coating, such as for example, acrylic acid derivatives (available from Röhm Pharma under the trade name EUDRAGIT®), pigments, etc.

The relative amounts of ingredients in the coating are preferably as follows. The proportion of the at least one water-insoluble, water-permeable polymer (in one embodiment ethylcellulose) in the coating may vary between about 20% and about 90% of the coating dry weight. The proportion of the at least one water-soluble polymer (in one embodiment polyvinylpyrrolidone) in the coating may vary between about 10% and about 75% of the coating dry weight. The proportion of the at least one plasticizer (in one embodiment dibutyl sebacate) in the coating may vary between about 5% and about 30% of the coating dry weight. The relative proportions of ingredients, notably the ratio of the at least one water-insoluble, water-permeable film-forming polymer to the at least one water-soluble polymer, can be varied depending on the desired release profile (where a more delayed release is desired, it is generally obtained with a higher amount of the at least one water-insoluble, water-permeable film-forming polymer).

The coating process can be as follows. Ethylcellulose, dibutyl sebacate and polyvinylpyrrolidone are dissolved in a solvent such as denatured alcohol using a propeller stirrer until complete dissolution is achieved. The resulting solution is sprayed onto the tablet cores, using a perforated coating pan.

The weight ratio coating/tablet core is comprised e.g. between about 1/30 and about 3/10, preferably about 1/10.

The tablet comprises an amount of the at least one form of tramadol of from about 25 mg to about 800 mg or more per tablet.

The present invention thus provides a controlled release pharmaceutical composition comprising at least one form of tramadol selected from the group consisting of tramadol, enantiomers thereof, pharmaceutically acceptable salts thereof and combinations thereof, the composition exhibiting a dissolution profile such that after 2 hours, from about 0% up to about 30% (by weight) of the at least one form of tramadol is released, after 4 hours, from about 5% to about 22% (by weight) of the at least one form of tramadol is released, after 6 hours, from about 15% to about 38% (by weight) of the at least one form of tramadol is released, after 8 hours, more than about 40% (by weight) of the at least one form of tramadol is released.

In an embodiment of the present invention, the controlled release pharmaceutical composition is an extended release tablet, the tablet comprising:
(i) a core comprising tramadol hydrochloride, polyvinyl alcohol, colloidal silicon dioxide and sodium stearyl fumarate; and
(ii) a coating comprising ethylcellulose, polyvinylpyrrolidone and dibutyl sebacate.

Further details of the preferred embodiments of the present invention are illustrated in the following examples which are understood to be non-limiting.

### EXAMPLE 1: 100 mg Tramadol HCl ER Tablets

The following 100 mg Tramadol HCl ER Tablet formulations were prepared:

**Table 1a: Tablet Core Formulation**

| **Ingredients** | **Quantity (mg)** | **%** |
|---|---|---|
| Tramadol HCl | 100.00 | 96.15 |
| Polyvinyl Alcohol | 2.00 | 1.92 |
| Colloidal Silicon Dioxide (AEROSIL® 200) | 1.00 | 0.96 |
| Sodium Stearyl Fumarate | 1.00 | 0.96 |
| Purified Water | 41.60 * | |
| ***Core Total Weight*** | 104.00 | 99.99 |

| | | |
|---|---|---|
| ** evaporated during process* | | |

### Tablet Core Preparation

Tramadol HCl and colloidal silicon dioxide were mixed and passed through a 1.0 mm screen. Polyvinyl alcohol was dissolved in purified water. The mixed tramadol HCl and colloidal silicon dioxide powder was granulated with the aqueous solution of polyvinyl alcohol in a fluidized bed granulator, Glatt GPCG1 and then dried. After granulation, the granules were blended with sodium stearyl fumarate and then passed through a 1.0 mm screen. The blend was then compressed into tablet cores using a Manesty Betapress.

**Table 1b: Coating Formulation**

| | **mg/tablet** | | |
|---|---|---|---|
| | **Lot#2159** | **Lot#2162** | **Lot#2165** |
| **Ingredients** | **Quantity (mg)** | **Quantity (mg)** | **Quantity (mg)** |
| Ethylcellulose (ETHOCEL® PR 100) | 9.20 | 9.81 | 9.54 |
| Polyvinylpyrrolidone (KOLLIDON® 90F) | 4.14 | 3.53 | 3.80 |
| Dibutyl Sebacate | 2.66 | 2.66 | 2.66 |
| Denatured Alcohol | 170.00 * | 170.00 * | 170.00 * |

| | | | |
|---|---|---|---|
| ** evaporated during process* | | | |

### Coating Preparation

The ethyl alcohol and isopropanol were weighed and mixed. Dibutyl sebacate and ethylcellulose were added to and dissolved in the ethyl alcohol and isopropyl alcohol while stirring using a propeller stirrer, Coframo RZR1. The ethylcellulose and dibutyl sebacate were allowed to dissolve completely. The polyvinylpyrrolidone was added. The solution was stirred until all components were dissolved. The solution was passed through a high pressure homogenizer, Mini DeBee 2000 with #7 nozzle, Bee International. The tablet cores were coated using the coating solution in a perforated coating pan, O'Hara Labcoat III 36" Pan, Vector LCDS.

Coating Parameters

| | |
|---|---|
| Inlet Temperature: | 48.5-49.5 °C |
| Outlet Temperature: | 38.5-39.5 °C |
| Bed Temperature | 37.5-38.5 °C |
| Spray Rate: | 300 g/min |
| Atomizing Air/Patterm: | 25/25 psi |
| Distance gun/ Bed: | 6" |
| Distance between guns: | 6" |
| Pan speed: | 12.0 rpm |

Coating Amount

| | |
|---|---|
| Diameter: | 6 mm |
| Thickness: | 4.65 mm |
| Cup Height: | 1.02 mm |
| Surface: | 112 mm² |
| Percentage: | 100 % |
| Amount: | 16 mg |

### Dissolution Method

*In vitro* dissolution studies were conducted on 100 mg Tramadol HCl ER Tablets formulated according to Lot#2159, Lot#2162 and Lot#21.65. The following dissolution conditions were used for all of the *in vitro* dissolution studies conducted herein for determining the *in vitro* dissolution profiles of Tramadol HCl ER Tablets:

| Apparatus: | USP Basket (10 mesh) |
|---|---|
| Dissolution medium: | 0.1 N HCl |
| Volume (vessels): | 900 ml |
| Bath temperature: | 37°C (± 0.5°C) |
| Wavelength: | 271 nm |
| Flow cell thickness: | 1 cm |
| Rotation speed: | 75 rpm |
| Total run time: | 900 min |
| Sampling interval: | 30 min |

**Table 2: Dissolution Profile**

| **Time (min.)** | **% Dissolved** | | |
|---|---|---|---|
| | **Lot#2159** | **Lot#2162** | **Lot#2165** |
| 0 | 0 | 0 | 0 |
| 30 | 1.1 | 0.1 | 0.3 |
| 60 | 5.7 | 0.3 | 2.0 |
| 90 | 12.8 | 1.4 | 5.1 |
| 120 | 21.3 | 2.9 | 9.1 |
| 180 | 41.6 | 7.0 | 19.8 |
| 240 | 62.4 | 12.8 | 33.4 |
| 300 | 77.8 | 20.2 | 48.7 |
| 360 | 87.3 | 29.4 | 62.7 |
| 420 | 92.6 | 40.3 | 73.5 |
| 480 | 95.9 | 50.8 | 81.7 |
| 540 | 97.5 | 59.9 | 87.2 |
| 600 | 98.7 | 67.6 | 91.1 |
| 660 | 99.2 | 73.7 | 94.1 |
| 720 | 99.6 | 78.2 | 96.0 |
| 780 | 99.9 | 81.9 | 97.2 |
| 840 | | 84.9 | 97.8 |
| 900 | | 86.9 | 98.5 |
| 960 | | 88.5 | 99.0 |

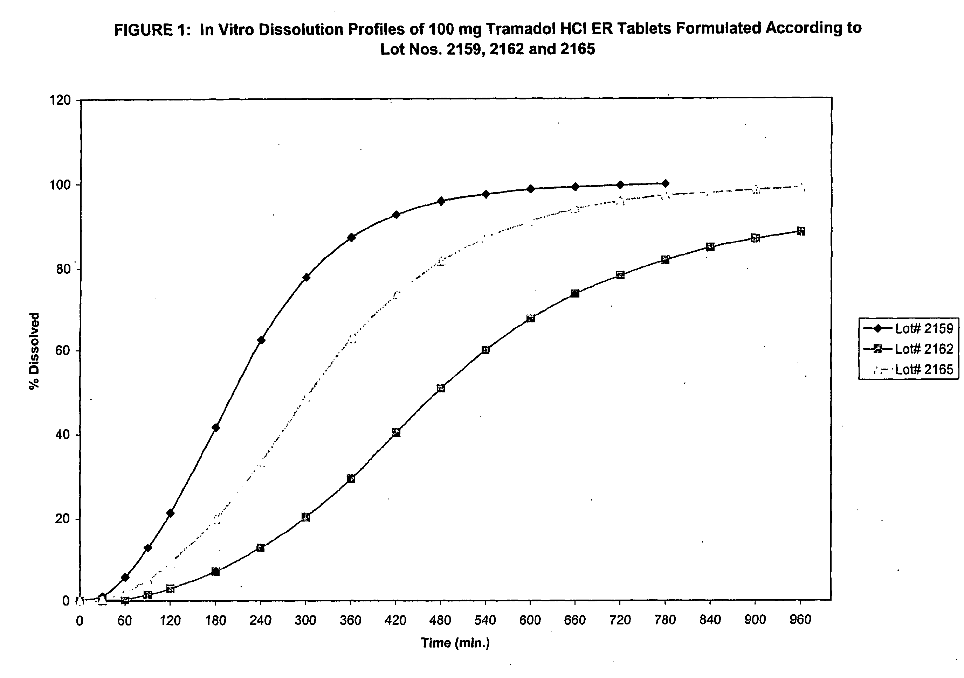

Figure 1 compares the *in vitro* dissolution profiles of 100 mg Tramadol HCl ER Tablets formulated according to Lot Nos. 2159, 2162 and 2165.

### Study No. 401 (B99-401PK-TRAP03)

A pilot four-way, single-dose, open-label, fasting, comparative bioavailability study of three formulations of Tramadol Hydrochloride Extended-Release Tablets (2x100 mg) Versus Ultram^{®} Tablets (50 mg q.i.d) in normal, healthy, Non-smoking male volunteers was conducted.

This pilot study evaluated the bioavailability of three novel Tramadol HCl Extended-Release Tablets (2x100 mg) against Ultram^{®} (Ortho-McNeil Pharmaceuticals) Tablets (50 mg q.i.d.) under fasting conditions.

This pilot study was a randomized, balanced, four-period, four-treatment, four-sequence crossover study design in sixteen (16) normal, healthy, non-smoking male volunteers and two (2) alternates.

Eighteen (18) subjects were entered into the study. Fourteen (14) subjects completed the study; there were fourteen (14) evaluable subjects. All subjects were non-smoking, between 18 and 45 years of age (inclusive), and with body weights no more than ±15% of the ideal weight for the subject's height and frame as determined by the Table of Desirable Weights for Men and Women.

The study periods were separated by a one-week washout period. Blood sampling for drug content analysis was carried out at 0.0 (pre-drug),1.0, 2.0, 3.0, 4.0, 6.0, 8.0, 10.0, 12.0, 16.0, 20.0, 24.0, 30.0, 36.0, and 48.0 hours post-drug when each test drug was administered. Blood sampling for drug content analysis was carried out at 0.0 (pre-drug), 1.0, 2.0, 3.0, 4.0, 5.0 (pre-drug), 6.0, 7.0, 8.0, 9.0, 10.0, 11.0 (pre-drug), 12.0, 13.0, 14.0, 15.0 (pre-drug), 16.0, 17.0, 18.0, 20.0, 24.0, 30.0, 36.0, and 48.0 hours post-drug when the reference drug was administered.
- Treatments: A:: Tramadol HCl ER 200 mg Tablets Lot Number: 2159
- B:: Tramadol HCl ER 200 mg Tablets Lot Number: 2162
- C:: Tramadol HCl ER 200 mg Tablets Lot Number: 2165
- D:: Ultram^{®} 50 mg Tablets Control Number: CDA 2225; Expiry Date: 4/ 01 (Ortho-McNeil Pharmaceuticals, U.S.A.)

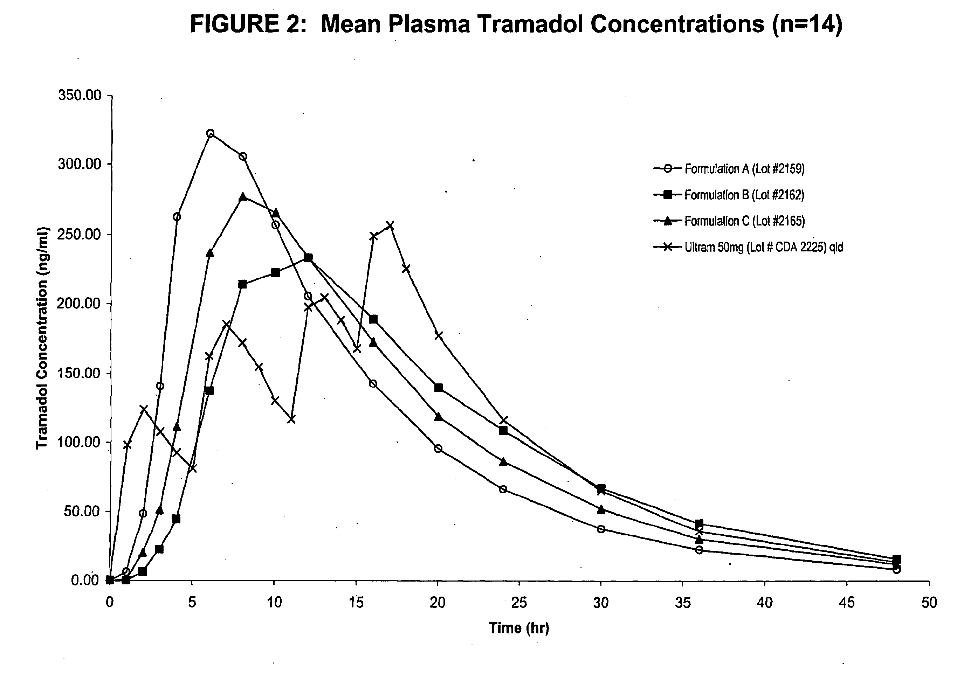

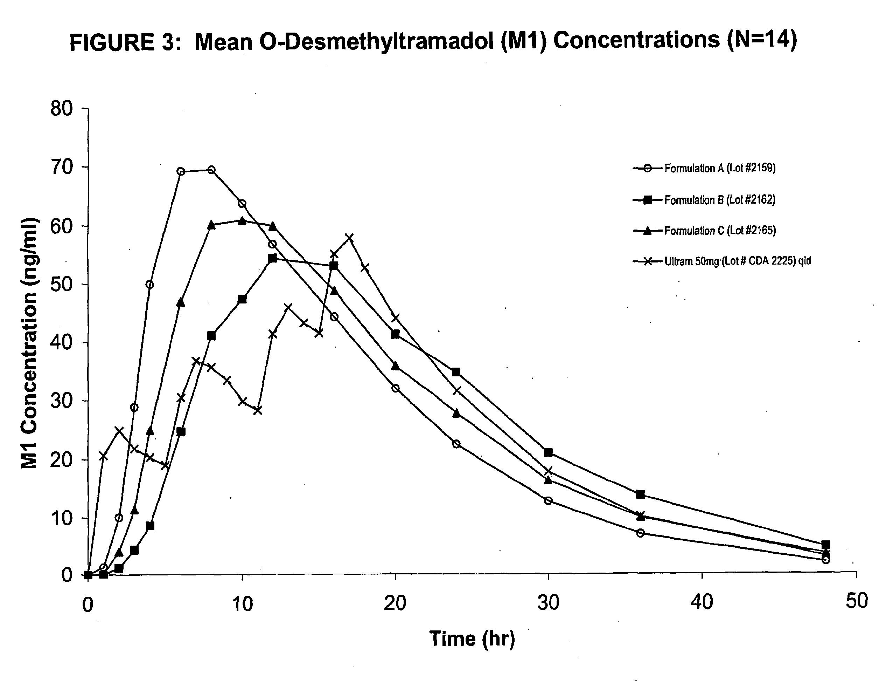

The three 100 mg extended release tramadol formulations tested (2x100 once a day) demonstrated prolonged tramadol and mono-O-desmethyltramadol plasma concentration-time profiles relative to the Ultram^{®} tablet (1 x 50mg) when administered 4 times a day (2^{nd}, 3^{rd} and 4^{th} doses at 5, 11 and 15 hours post-1^{st} dose, respectively) (See figures 2 and 3). In addition, the ER formulations yielded equivalent AUCs relative to an equivalent dose of the Ultram® immediate release tablet. The 90% geometric mean confidence intervals for AUCₜ and AUC_{∞} were within the 80%-125% range for all three test formulations. Formulations 2162 and 2165 also yielded equivalent Cₘₐₓ values versus Ultram^{®} as evidenced by 90% geometric confidence intervals within the 80-125% range. The mean pharmacokinetic parameters and 90% confidence interval for ratio of the geometric mean AUC and Cₘₐₓ are presented in Tables 3a and 3b for tramadol and in Tables 4a and 4b for O-desmethyltramadol. Table 3a also shows that overall there was no apparent difference in the ratio of metabolite (AUC_{∞} of M1/tramadol) among the ER tramadol formulations and the immediate release tablet. The half-life following Ultram^{®} treatment was slightly shorter compared to the extended release formulations.

**Table 3a: Pharmacokinetic Parameters Study 401PK (n = 14) Study 401PK Mean Pharmacokinetic Parameters for Plasma Tramadol (n = 14)**

| **Parameter** | **Lot# 2159 1x200mg** | **Lot# 2162 1x200mg** | **Lot# 2165 1x200mg** | **Ultram 50mg q.i.d** |
|---|---|---|---|---|
| | **Mean (CV %)** | **Mean (CV %)** | **Mean (CV %)** | **Mean (CV %)** |
| **AUCₜ (hr*ng/mL)** | 4796.83 (42.92) | 4663.89 (34.42) | 4827.94 (44.08) | 4915.71 (43.81) |
| **AUC_{∞} (hr*ng/mL)** | 4936.23 (45.71) | 4897.97 (38.96) | 5028.36 (46.36) | 5118.72 (47.88) |
| **Cₘₐₓ(ng/mL)** | 351.60 (28.89) | 246.46 (32.13) | 298.30 (38.34) | 284.70 (36.17) |
| **Tₘₐₓ (hr)** | 5.86 (21.02) | 9.86 (21.74) | 8.43(19.03) | 14.07 (37.03) |
| **Half-life (hr)** | 6.90 (32.10) | 7.94 (32.96) | 7.49 (37.02) | 6.73 (37.46) |
| **M1/Tramadol** | 0.29 (50.96) | 0.30 (45.62) | 0.29 (49.35) | 0.29 (52.92) |
| **MRT (hr)** | 13.70 (24.08) | 19.48 (18.19) | 16.57 (22.61) | 17.79 (19.03) |
| **Lag Time (hr)** | 0.00 (0.00) | 1.00(0.00) | 0.64 (77.35) | 0.00(0.00) |

Figure 2 illustrates the mean plasma Tramadol concentrations (ng/ml) over time following once a day Tramadol HCl ER Tablet (100 mg x 2) formulated according to Lot Nos. 2159, 2162 and 2165 vs Ultram^{®} (50 mg x 2) q.i.d.

**Table 3b: Ratio of Means & 90% Confidence Interval for Plasma Tramadol**

| | **AUC(0-t)** | | | **AUC(0-□)** | | | **Cmax** | | |
|---|---|---|---|---|---|---|---|---|---|
| | **90% CI** | **Ratio of Means** | **CV(%)** | **90% CI** | **Ratio of Means** | **CV(%)** | **90% CI** | **Ratio of Means** | **CV(%)** |
| **Formulation A (Lot # 2159)** | 90.1-105.1 | 97.3 | 11.5 | 89.2-104.4 | 96.5 | 11.7 | 114.2-140.0 | 126.5 | 15.2 |
| **Formulation B (Lot # 2162)** | 93.1-109.6 | 101 | 11.5 | 93.7-110.7 | 101.9 | 11.7 | 80.5-101.1 | 90.7 | 15.2 |
| **Formulation C (Lot # 2165)** | 94.0-109.6 | 101.5 | 11.5 | 94.0-110.1 | 101.7 | 11.7 | 99.0 -121.4 | 109.6 | 15.2 |

**Table 4a: Pharmacokinetic Parameters Study 401 (401PK) (n = 14) Study 401PK: Mean Pharmacokinetic Parameters for Plasma O-desmethyltramadol (n =12)**

| **Parameter** | **Lot# 2159 1x200mg** | **Lot# 2162 1x200mg** | **Lot# 2165 1x200mg** | **Ultram 50mg q.i.d** |
|---|---|---|---|---|
| | **Mean (CV %)** | **Mean (CV %)** | **Mean (CV %)** | **Mean (CV %)** |
| **AUCₜ (hr*ng/mL)** | 1193.78 (44.84) | 1230.54 (40.02) | 1169.03 (39.15) | 1166.74 (33.21) |
| **AUC_{∞} (hr*ng/mL)** | 1226.20 (44.26) | 1295.76 (41.06) | 1218.22 (39.68) | 1201.62 (32.42) |
| **Cₘₐₓ(ng/mL)** | 68.91 (39.65) | 56.49 (36.64) | 61.75 (39.92) | 60.72 (35.05) |
| **Tₘₐₓ (hr)** | 7.29 (23.11) | 13.29 (21.78) | 10.14 (29.41) | 16.71 (4.35) |
| **Half-life (hr)** | 7.56 (30.98) | 8.80 (36.96) | 8.16 (28.65) | 7.50 (32.79) |

Figure 3 illustrates the mean plasma Desmethyltramadol concentrations (ng/ml) following once a day Tramadol HCl ER Tablet (100 mg x 2) formulated according to Lot Nos. 2159, 2162 and 2165 vs Ultram^{®} (50 mg x 2) q.i.d.

**Table 4b: Ratio of Means & 90% Confidence Interval for Plasma O-desmethyltramadol**

| | **AUC(0-t)** | | **AUC(0-□)** | | **Cmax** | |
|---|---|---|---|---|---|---|
| | **90% CI** | **Ratio of Means** | **90% CI** | **Ratio of Means** | **90% CI** | **Ratio of Means** |
| **Formulation A (Lot # 2159)** | 87.8-109.7 | 98.1 | 87.6-108.9 | 97.7 | 98.3-124.6 | 110.7 |
| **Formulation B (Lot # 2162)** | 91.0-115.2 | 102.4 | 93.0-117.1 | 104.3 | 80.7-103.7 | 91.4. |
| **Formulation C (Lot # 2165)** | 89.0-111.2 | 99.5 | 89.7-111.6 | 100.1 | 89.4-113.3 | 100.7 |

### Study No. 99103 (B99-416PK-TRAP03)

A pilot two-way, multiple-dose, open-label, Fasting, comparative bioavailability study of Tramadol Hydrochloride Extended-Release Tablets (2x100 mg) versus Ultram^{®} in normal, healthy, non-smoking male and female volunteers was conducted.

The objective of this study was to compare the rate and extent of absorption of a new extended-release formulation of tramadol hydrochloride (2x100 mg) against Ultram^{®} (50 mg q.i.d.) under steady-state conditions in normal healthy male and female volunteers. This comparison reflects the administration of Ultram^{®} under clinical conditions.

This pilot steady-state study was a randomized, two-way crossover study design in sixteen (16) normal, healthy, non-smoking male and female volunteers and four (4) alternates (total 11 males and 9 females).

Twenty (20) subjects were entered into the study. Fifteen (15) subjects completed the study; there were fifteen (15) evaluable subjects. All subjects were non-smoking, between 18 and 45 years of age (inclusive), and with body weights no more than ±15% of the ideal weight for the subject's height and frame as determined by the Table of Desirable Weights for Men and Women. All female subjects were non-lactating, had negative pregnancy tests, and were taking an acceptable method of contraception.

The study periods were separated by a one-week washout period. Blood sampling for drug content analysis was carried out as follows for the test product (Tramadol ER tablets (2x100 mg), treatment A, Lot# 2162): Day 1 - 0.0 (pre-dose), 1.0, 2.0, 3.0, 4.0, 6.0, 8.0,10.0,12.0,16.0, 20.0, 24.0; Day 2, 3, and 4 - 0.0 (pre-dose); Day 5 - 0.0 (pre-dose),1.0, 2.0, 3.0, 4.0, 6.0, 8.0,10.0,12.0,16.0, 20.0, 24.0, 30.0, 36.0, and 48.0 hours post-drug administration.

Blood sampling for drug content analysis was carried out as follows for the reference product (Ultram^{®} 50 mg tablets q.i.d., treatment B, Lot# CDA2225): Day 1 - 0.0 (pre-dose), 1.0, 2.0, 3.0, 4.0, 5.0 (pre-dose), 6.0, 7.0, 8.0, 9.0, 10.0, 11.0 (pre-dose), 12.0,13.0,14.0,15.0 (pre-dose), 16.0,17.0,18.0,20.0 and 24.0; Days 2,3, and 4 - 0.0 (pre-dose); Day 5 - 0.0 (pre-dose), 1.0, 2.0, 3.0, 4.0, 5.0 (pre-dose), 6.0, 7.0, 8.0, 9.0, 10.0, 11.0 (pre-dose), 12.0, 13.0, 14.0, 15.0 (pre-dose), 16.0, 17.0, 18.0, 20.0, 24.0, 30.0, 36.0, and 48.0 hours post-drug administration.
- Treatments: A:: 2 Tablets of Tramadol HCl ER 100 mg Tablets (Lot# 2162-Biovail Corporation International, Canada) once a day (approximately 7 AM) for 5 consecutive days.
- B:: Ultram^{®} (Tramadol HCl 50 mg tablet, Ortho-McNeil Pharmaceutical, USA) (Lot# CDA2225) q.i.d. (approximately 7 AM, 12 PM, 6 PM and 10 PM) for 5 consecutive days.

In the current study, Lot# 2162 was compared to immediate release Ultram^{®} under multiple-dose conditions. The extended release formulation performed consistently under both single and multiple doses. The overall half-life after multiple-dose for tramadol was 7.3 hours and 6.7 hours, respectively, following Tramadol HCl ER Tablets and Ultram^{®}. Steady state levels of tramadol were achieved by the third dose (day 3 of the study) for Tramadol HCl ER Tablets, and by the fifth dose (Day 2 of the study) for Ultram^{®}. The mean pharmacokinetic data for single dose and multiple dose of tramadol and the M1 are presented in tables 5a - 5b and 6a - 6b, respectively. Steady-state bioequivalence between Tramadol HCl ER Tablets (Lot# 2162) and immediate-release Ultram® (Lot # CDA2225) was established. The 90% confidence intervals for AUC and Cₘₐₓ were within the 80-125% limits for both unchanged drug and O-desmethyltramadol. Tramadol HCl ER Tablets (Lot# 2162) given once daily exhibited lower percent fluctuation at steady state (70%) than Ultram^{®} given four times a day.

**Table 5a: Pharmacokinetic Parameters Study 99103 (416PK) (n =15) Study 416PK Mean Pharmacokinetic Parameters for Plasma Tramadol (n = 15)**

| **Parameter** | **Tramadol ER (2x00 mg) q.d.** | | **Ultram 50 mg q.i.d.** | |
|---|---|---|---|---|
| | **Day 1** | **Day 5** | **Day 1** | **Day 5** |
| **AUC_{0-□}** | 5089.010 | 7715.89 | 5000.73 | 7004.37 |
| **(ng hr/mL)** | (37.55) | (35.69) | (37.94) | (27.81) |
| **Cₘₐₓ (ng/mL)** | 365.62 | 431.58 | 348.23 | 406.95 |
| | (40.34) | (34.06) | (36.73) | (26.88) |
| **Tₘₐₓ (hr)** | 13.47 (19.82) | 12.80 | 16.00 | 15.80 |
| | | (21.13) | (10.02) | (26.23) |
| **t1/2 ₑₗ (hr)** | | 7.32 (16.41) | | 6.67 (20.24) |
| **% Fluctuation** | | 70.19 | | 81.82 |
| | | (24.19) | | (20.28) |
| | | | | |

| **Cₘᵢₙ (ng/mL)** | **Tramadol ER (2x100 mg) q.d.** | | **Ultram 50 mg q.i.d.** | |
|---|---|---|---|---|
| **Day 1** | 161.37 (70.23) | | 147.52 (49.96) | |
| **Day 2** | 213.43 (52.38) | | 178.52 (47.03) | |
| **Day 3** | 235.13 (56.41) | | 183.89 (36.33) | |
| **Day 4** | 231.44 (44.66) | | 176.41 (44.24) | |
| **Day 5** | 253.55 (46.44) | | 201.20 (26.12) | |

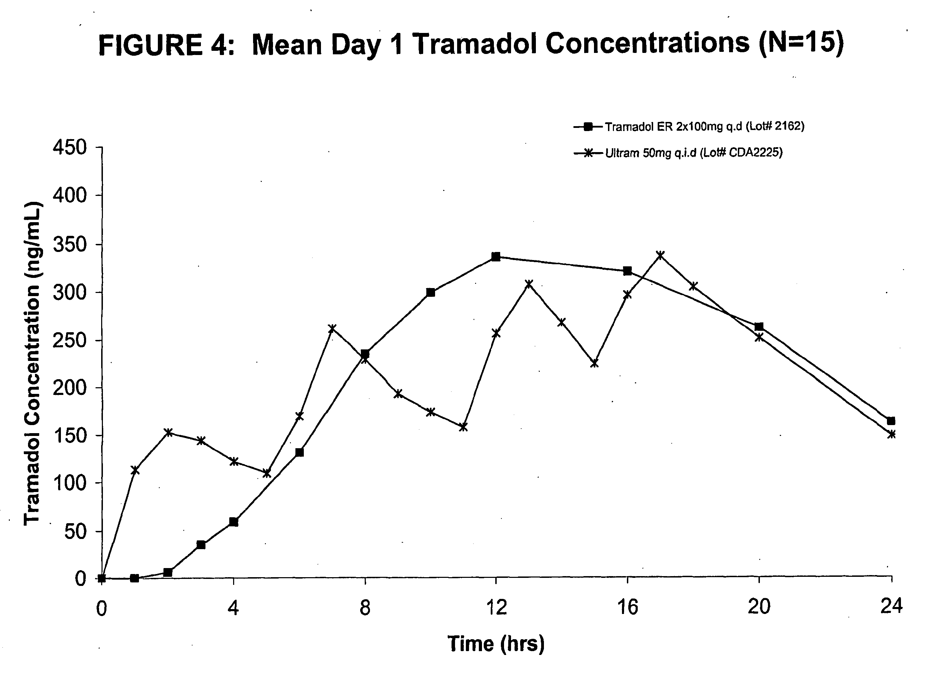

Figure 4 illustrates the mean plasma Tramadol concentrations on Day 1 following once a day Tramadol HCl ER Tablets (100 mg x 2) formulated according to Lot# 2162 for 5 Days vs. Ultram^{®} (50 mg x 2) q.i.d.

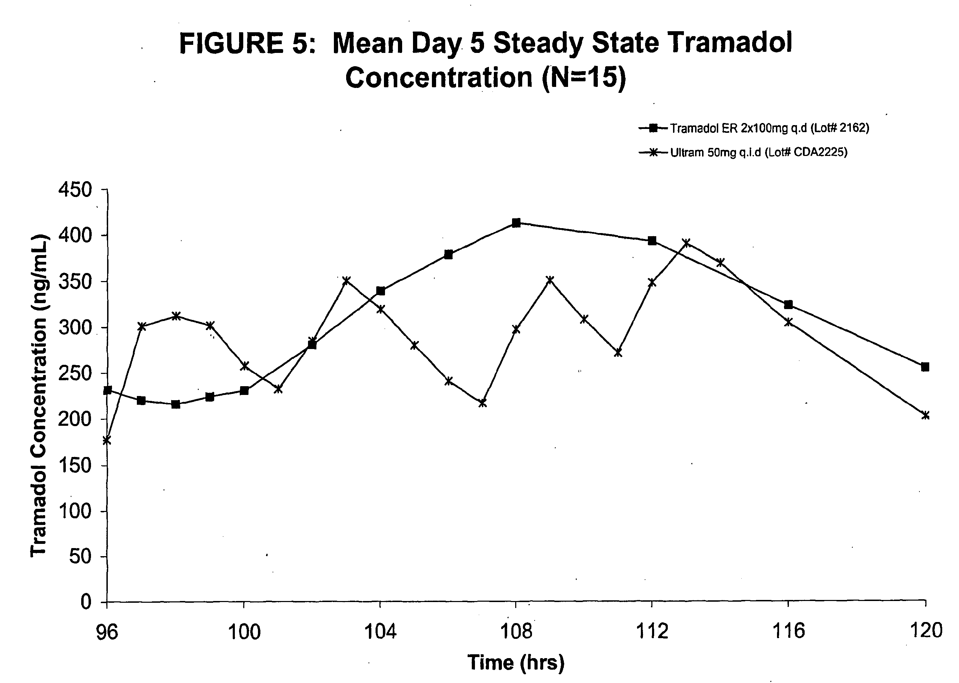

Figure 5 illustrates the mean plasma Tramadol concentrations on Day 5 following once a day Tramadol HCl ER Tablets (100 mg x 2) formulated according to Lot# 2162 for 5 Days vs. Ultram^{®} (50 mg x 2) q.i.d.

**Table 5b: Ratio of Means & 90% Confidence Interval for Plasma Tramadol**

| **Statistical Analysis (ANOVA)** | **Treatment** | **Ratio ¹** | **90 % Geometric C.I. ²** | |
|---|---|---|---|---|
| | **Comparisons** | | **Lower** | **Upper** |
| **AUC₀₋ₜ** | Tramadol HCl ER (Lot#2162) vs Ultram® (Lot# CDA2225) | 108.6% | 104.2% | 113.2% |
| **Cₘₐₓ** | Tramadol HCl ER (Lot#2162) vs Ultram® (Lot# CDA2225) | 104.9 % | 98.6% | 111.6% |

| | | | | |
|---|---|---|---|---|
| ¹Ratio of least squares means ²Calculated from log-transformed data | | | | |

**Table 6a: Pharmacokinetic Parameters Study 99103 (416PK) (n = 15) Study 416PK: Mean Pharmacokinetic Parameters for Plasma O-desmethyltramadol (n =15)**

| **Parameter** | **Tramadol ER (2x100 mg) q.d.** | | **Ultram 50 mg q.i.d.** | |
|---|---|---|---|---|
| | **Day 1** | **Day 5** | **Day 2** | **Day 5** |
| **AUC**_{**0**-□} | 1037.71 | 1550.55 | 1105.30 | 1540.17 |
| **(ng hr/mL)** | (40.22) | (37.21) | (37.74) | (39.07) |
| **Cₘₐₓ (ng/mL)** | 70.85 (39.87) | 79.75 | 72.82 | 80.97 |
| | | (36.94) | (37.02) | (41.13) |
| **Tₘₐₓ (hr)** | 14.27 (21.76) | 13.73 | 16.87 (8.34) | 13.47 |
| | | (16.39) | | (41.43) |
| t**1/2 ₑ₁ (hr)** | | 8.49 (14.28) | | 7.15 (12.75) |
| **% Fluctuation** | | 49.46 | | 55.08 |
| | | (22.71) | | (33.22) |
| | | | | |

| **Cₘᵢₙ (ng/mL)** | **Tramadol ER (2x100 mg) q.d.** | | **Ultram 50 mg q.i.d.** | |
|---|---|---|---|---|
| **Day 1** | 40.05 (45.16) | | 41.34 (38.83) | |
| **Day 2** | 54.73 (39.72) | | 49.41 (37.52) | |
| **Day 3** | 56.67 (36.46) | | 50.03 (37.94) | |
| **Day 4** | 56.55 (37.43) | | 47.35 (40.18) | |
| **Day 5** | 55.19 (38.43) | | 50.86 (39.67) | |

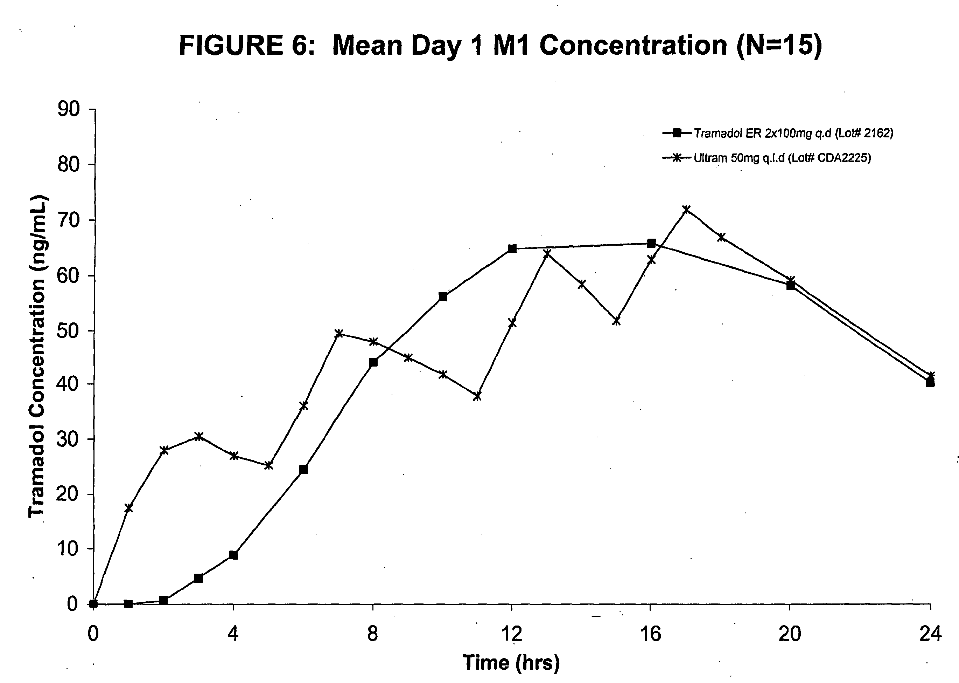

Figure 6 illustrates the mean plasma Desmethyltramadol concentrations on Day 1 following once a day Tramadol HCl ER Tablets (100 mg x 2) formulated according to Lot# 2162 for 5 Days vs. Ultram^{®} (50 mg x 2) q.i.d.

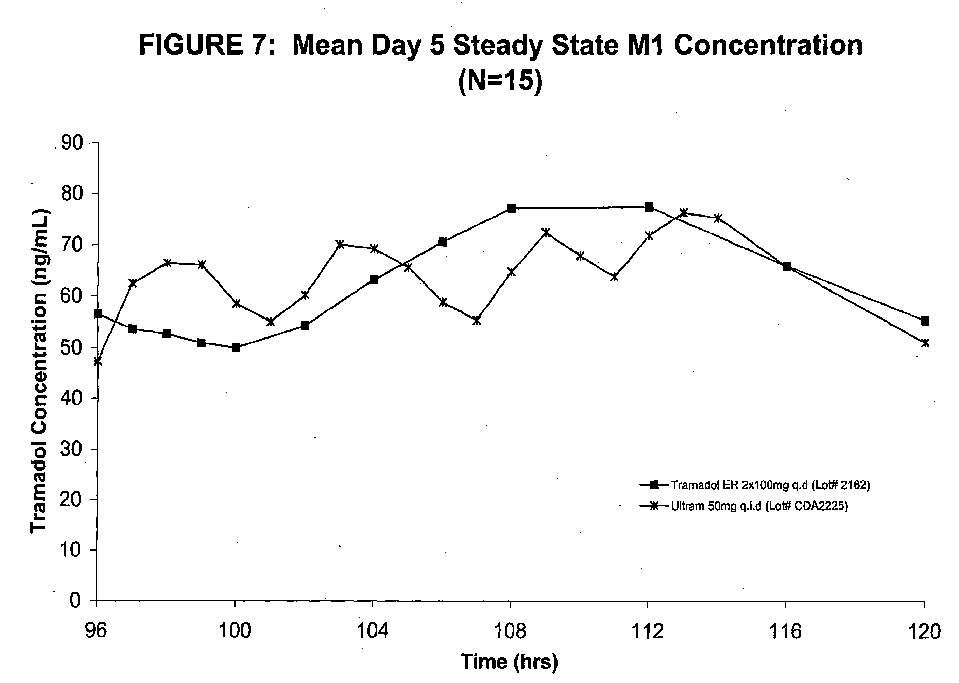

Figure 7 illustrates the mean plasma Desmethyltramadol concentrations on Day 5 following once a day Tramadol HCl ER Tablets (100 mg x 2) formulated according to Lot# 2162 for 5 Days vs. Ultram^{®} (50 mg x 2) q.i.d.

**Table 6b: Ratio of Means & 90% Confidence Interval for Plasma O-desmethyltramadol**

| **Statistical Analysis (ANOVA)** | **Treatment Comparisons** | **Ratio 1** | **90 % Geometric C.I. ²** | |
|---|---|---|---|---|
| | | | **Lower** | **Upper** |
| **AUC₀₋ₜ** | Tramadol HCl ER (Lot#2162) vs Ultram^{®} (Lot# CDA2225) | 101.5 % | 97.2% | 106.0% |
| **Cₘₐₓ** | Tramadol HCl ER (Lot#2162) vs Ultram^{®} (Lot# CDA2225) | 100.0% | 94.2% | 106.2% |

| | | | | |
|---|---|---|---|---|
| ¹Ratio of least squares means ²Calculated from log-transformed data | | | | |

### Study No. 2282 (B99-424PK-TRAP03)

A pilot three-way, multiple-dose, open-label, fasting, comparative bioavailability study of two formulations of Tramadol Hydrochloride Extended-Release Tablets (3x100 mg) administered once a day versus Ultram^{®} Tablets (2x50 mg) administered three times a day in normal, healthy, non-smoking male and female volunteers was conducted.

The objective of this study was to compare the rate and extent of absorption of two new extended-release formulations of tramadol hydrochloride (3x100 mg) administered once daily against Ultram^{®} (2x50 mg) administered three times a day under steady-state conditions in normal healthy male and female volunteers. This comparison reflects the administration of Ultram^{®} under clinical conditions.

This pilot steady-state study was a randomized, three-way crossover study design in fifteen (15) normal, healthy, non-smoking male and female volunteers and three (3) alternates (total 11 males and 9 females). Eighteen (18) subjects were entered into the study. Fourteen (14) subjects completed the study; there were fourteen (14) evaluable subjects. All subjects were non-smoking, between 18 and 45 years of age (inclusive), and with body weights no more than ±15% of the ideal weight for the subject's height and frame as determined by the Table of Desirable Weights for Men and Women. All female subjects were non-lactating, had negative pregnancy tests, and were taking an acceptable method of contraception.

The study periods were separated by a one-week washout period. Blood sampling for drug content analysis was carried out as follows for the two test products (Tramadol ER tablets (3x100 mg), treatment A (Lot# 2162) and treatment B (Lot# 2165)): Day 1 - 0.0 (pre-dose), 1.0, 2.0, 3.0, 4.0, 6.0, 8.0, 10.0, 12.0,16.0, 20.0, Day 2, 3, and 4 - 0.0 (pre-dose); Day 5 - 0.0 (pre-dose), 1.0, 2.0, 3.0, 4.0, 6.0, 8.0, 10.0, 12.0, 16.0, 20.0, 24.0, 30.0, 36.0 and 48.0 hours post-drug administration.

Blood sampling for drug content analysis was carried out as follows for the reference product (Ultram^{®} 50 mg tablets q.i.d., treatment C): Day 1 - 0.0 (pre-dose), 1.0, 2.0, 3.0, 4.0, 5.0 (pre-dose), 6.0, 7.0, 8.0, 9.0, 10.0, 11.0 (pre-dose), 12.0, 13.0, 14.0, 16.0, and 20.0 hours; Days 2, 3, and 4 - 0.0 (pre-dose); Day 5 - 0.0 (pre-dose), 1.0, 2.0, 3.0, 4.0, 5.0 (pre-dose), 6.0, 7.0, 8.0, 9.0, 10.0, 11.0 (pre-dose), 12.0, 13.0, 14.0, 16.0, 20.0, 24.0, 30.0, 36.0, and 48.0 hours post-drug administration.
- Treatments: A:: 3 Tablets of Tramadol HCl ER 100 mg Tablets (Lot# 2162 - Biovail Corporation International, Canada) once a day (approximately 7 AM) for 5 consecutive days.
- B:: 3 Tablets of Tramadol HCl ER 100 mg Tablets (Lot# 2165-Biovail Corporation International, Canada) once a day (approximately 7 AM) for 5 consecutive days.
- C:: 2 Tablets of Ultram® (Tramadol HCl 50 mg tablet, Ortho-McNeil Pharmaceutical, USA) (Lot# CDA2225) t.i.d. (approximately 7 AM, 12 PM and 6 PM) for 5 consecutive days.

The current study was undertaken to compare the two lead Tramadol HCl ER Tablet formulations (Lot# 2162 and Lot# 2165) (3x100 mg) administered once daily against Ultram^{®} (2x50 mg) administered three times a day under steady-state conditions in normal healthy male and female volunteers. This comparison reflects the administration of Ultram^{®} under clinical conditions.

The extended release formulations performed consistently both under single and multiple dose conditions. The overall half-life after multiple-dose for tramadol was 7.3 hours following Tramadol HCl ER Tablets (Lot# 2162), 6.9 hours following Tramadol HCl ER Tablets (Lot# 2165), and 6.4 hours immediate release Ultram^{®}. Steady state levels of tramadol were achieved by the third dose (day 3 of the study) for Tramadol HCl ER Tablets, (Lot# 2162 and Lot# 2165) and by the seventh dose (day 3 of the study) for Ultram^{®}. The mean pharmacokinetic data for single dose and multiple doses of tramadol and the M1 are presented in tables 7a - 7b and 8a - 8b, respectively. Steady-state bioequivalence between Tramadol HCl ER tablets (Lot# 2162 and Lot# 2165) and immediate-release Ultram^{®} (Lot # CDA2225) was established. The 90% confidence intervals for unchanged drug and O-desmethyltramadol AUC and Cₘₐₓ for Tramadol HCl ER Tablets (Lot# 2162), and the 90% confidence intervals for unchanged drug and O-desmethyltramadol AUC for Tramadol HCl ER Tablets (Lot# 2165) were within the 80-125% limits. O-desmethyltramadol Cₘₐₓ for Lot# 2165 was within the limits.

Lot# 2162 demonstrates steady-state bioequivalence versus both t.i.d. and q.i.d. administration of Ultram^{®} as evidenced by 90% C.I. values for AUC and Cₘₐₓ within 80-125 % limits for both tramadol and O-desmethyltramadol. Lot# 2162 also exhibited lower percent fluctuation versus Ultram® when given t.i.d. and q.i.d.

**Table 7a: Pharmacokinetic Parameters Study 2282 (424PK) (n = 15) Study 424PK Mean Pharmacokinetic Parameters for Plasma Tramadol (n =15)**

| **Parameter** | **Tramadol ER (2x100 mg) q.d. Lot # 2162** | | **Tramadol ER (2×100 mg) q.d. Lot # 2165** | | **Ultram 50 mg q.i.d. Lot #** | |
|---|---|---|---|---|---|---|
| | **Day 1** | **Day 5** | **Day 1** | **Day 5** | **Day 1** | **Day 5** |
| **AUC_{0-□} (ng hr/mL)** | 6407.95 (27.03) | 9849.28 (23.65) | 6977.91 (27.97) | 10116.75 (23.97) | 6854.57 (25.77) | 9611.88 (19.12) |
| **Cₘₐₓ (ng/mL)** | 457.65 (28.37) | 585.17 (21.58) | 540.76 (24.05) | 699.76 (2232) | 464.67 (23.46) | 621.66 (20.06) |
| **Tₘₐₓ (hr)** | 10.40 (20.80) | 10.90 (27.70) | 7.90 (18.6) | 8.40 (21.20) | 1220 (34.20) | 9.60 (36.10) |
| **t1/2 ₑₗ (hr)** | | 7.32(23.58) | | 6.91 (17.33) | | 6.40 (14.20) |
| **% Fluctuation** | | 84.73 (36.04) | | 125.39 (24.96) | | 114.47 (15.79) |
| | | | | | | |

| **Cₘᵢₙ (ng/mL)** | **Tramadol ER (2x100 mg) q.d. Lot # 2162** | | **Tramadol ER (2x100 mg) q.d. Lot #2165** | | **Ultram 50 mg q.i.d. Lot #** | |
|---|---|---|---|---|---|---|
| **Day 2** | 174.56 (40.90) | | 134.96 (51.01) | | 142.63 (36.88) | |
| **Day 3** | 213.73 (41.07) | | 156.63 (37.89) | | 154.99 (39.36) | |
| **Day 4** | 218.78 (44.50) | | 175.36 (46.91) | | 150.46 (32.52) | |
| **Day 5** | 250.77 (43.26) | | 186.04 (47.41) | | 166.85 (31.67) | |

**Table 7b: Ratio of Means & 90% Confidence Interval for Plasma Tramadol**

| **Statistical Analysis (ANOVA)** | **Treatment** | **Ratio ¹** | **90 % Geometric C.I. ²** | |
|---|---|---|---|---|
| | **Comparisons** | | **Lower** | **Upper** |
| **AUC₀₋ₜ** | Tramadol ER (Lot#2162) vs Ultram^{®} (Lot# CDA2225) | 101.9% | 95.4% | 108.8 % |
| | Tramadol ER (Lot# 2165) vs Ultram^{®} (Lot# CDA2225) | 104.9% | 98.2% | 112.0% |
| **Cₘₐₓ** | Tramadol ER (Lot#2162) vs Ultram^{®} (Lot# CDA2225) | 93.1% | 83.9% | 103.4% |
| | Tramadol ER (Lot#2165) vs Ultram^{®} (Lot# CDA2225) | 114.1% | 102.8% | 126.7% |

| | | | | |
|---|---|---|---|---|
| ¹Ratio of least squares means ²Calculated from log-transformed data | | | | |

**Table 8a: Pharmacokinetic Parameters Study 2282 (424PK) (n = 15) Study 424PK: Mean Pharmacokinetic Parameters for Plasma O-desmethyltramadol (n =15)**

| **Parameter** | **Tramadol ER (2×100 mg) q.d. Lot # 2162** | | **Tramadol ER (2×100 mg) q.d. Lot # 2165** | | **Ultram 50 mg q.i.d. Lot #** | |
|---|---|---|---|---|---|---|
| | **Day 1** | **Day 5** | **Day 1** | **Day 5** | **Day 1** | **Day 5** |
| **AUC_{0-□} (ng hr/mL)** | 1896.02 (26.07) | 2554.04 (26.68) | 2133.71 (32.64) | 2478.46 (32.46) | 2096.32 (24.41) | 2475.64 (25.10) |
| **Cₘₐₓ (ng/mL)** | 130.64 (30.58) | 138.37 (24.02) | 150.67 (33.05) | 145.57 (29.10) | 127.43 (2432) | 138.26 (26.73) |
| **Tₘₐₓ (hr)** | 11.60 (19.80) | 12.60 (22.00) | 9.60 (14.60) | 9.20 (18.80) | 13.20 (29.90) | 13.20 (14.30) |
| | | | | | | |

| **Cₘᵢₙ (ng/mL)** | **Tramadol ER (2×100 mg) q.d.** | | **Tramadol ER (2x100 mg) q.d.** | | **Ultram 50 mg q.i.d.** | |
|---|---|---|---|---|---|---|
| **Day 2** | 64.24 (34.32) | | 52.92 (46.34) | | 57.74 (25.59) | |
| **Day 3** | 70.48 (33.65) | | 55.29 (41.81) | | 56.61 (28.78) | |
| **Day 4** | 76.09 (41.83) | | 60.05 (44.08) | | 57.67 (28.81) | |
| **Day 5** | 76.87 (38.41) | | 59.65 (45.35) | | 58.82 (30.95) | |

**Table 8b: Ratio of Means & 90% Confidence Interval for Plasma O-desmethyltramadol**

| **Statistical Analysis (ANOVA)** | **Treatment** | **Ratio ¹** | **90 % Geometric C.I.²** | |
|---|---|---|---|---|
| | **Comparisons** | | **Lower** | **Upper** |
| **AUC₀₋ₜ** | Tramadol ER (Lot# 2162) vs Ultram^{®} (Lot# CDA2225) | 102.4% | 96.9 % | 108.1% |
| | Tramadol ER (Lot# 2165) vs Ultram^{®} (Lot# CDA2225) | 98.6% | 93.4% | 104.2% |
| **Cₘₐₓ** | Tramadol ER (Lot# 2162) vs Ultram^{®} (Lot# CDA2225) | 100.4 % | 95.0 % | 106.0% |
| | Tramadol ER (Lot# 2165) vs Ultram^{®} (Lot# CDA2225) | 105.3% | 99.7% | 111.3% |

| | | | | |
|---|---|---|---|---|
| ¹Ratio of least squares means ²Calculated from log-transformed data | | | | |

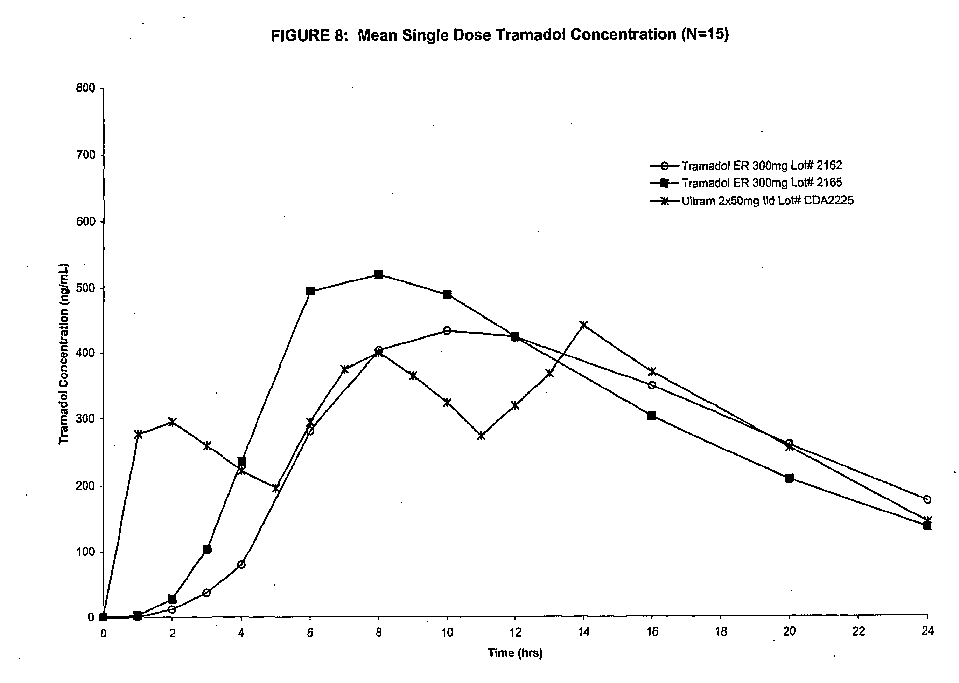

Figure 8 illustrates the mean plasma Tramadol concentrations on Day 1 following once a day Tramadol HCl ER Tablets (100 mg x 3) formulated according to Lot Nos. 2162 and 2165 for 5 Days vs. Ultram^{®} (50 mg x 2) t.i.d.

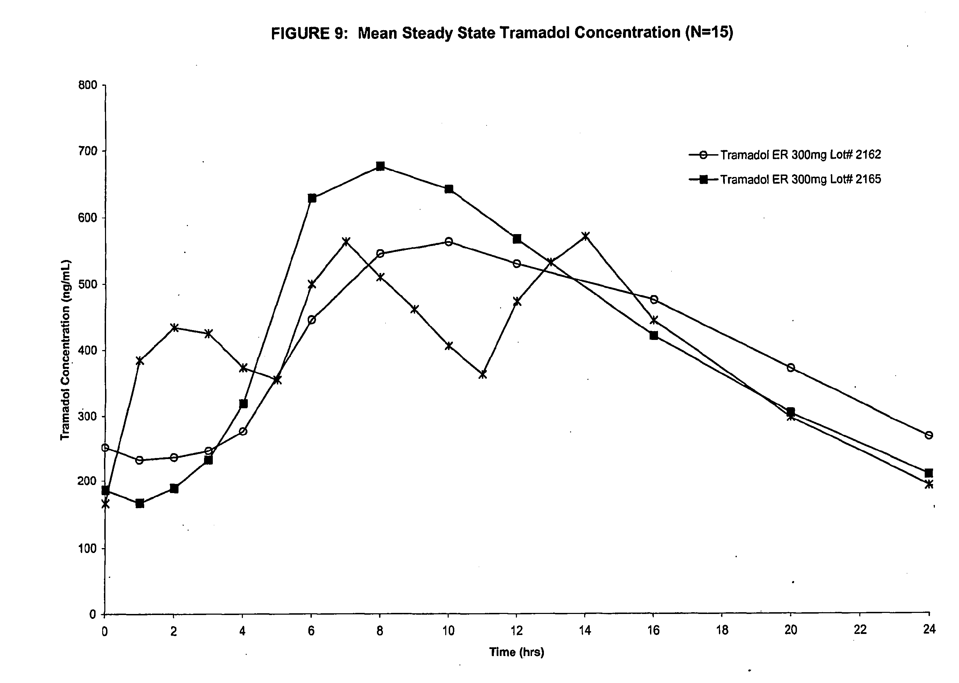

Figure 9 illustrates the mean plasma Tramadol concentrations on Day 5 following once a day Tramadol HCl ER Tablets (100 mg x 3) formulated according to Lot Nos. 2162 and 2165 for 5 Days vs. Ultram^{®} (50 mg x 2) t.i.d.

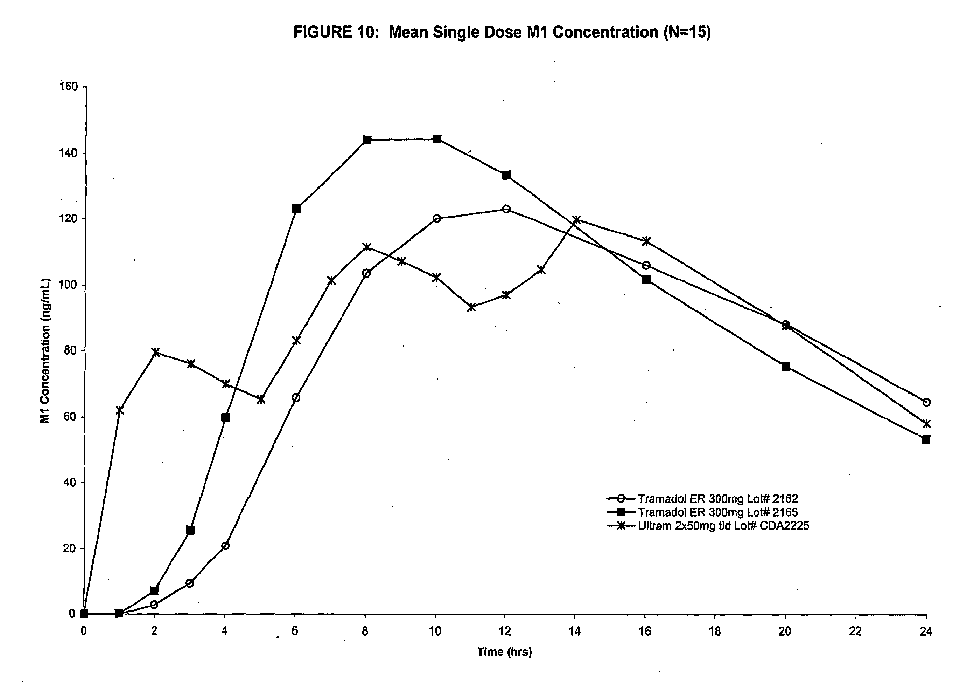

Figure 10 illustrates the mean plasma Desmethyltramadol concentrations on Day 1 following once a day Tramadol HCl ER Tablets (100 mg x 3) formulated according to Lot Nos. 2162 and 2165 for 5 Days vs. Ultram^{®} (50 mg x 2) t.i.d.

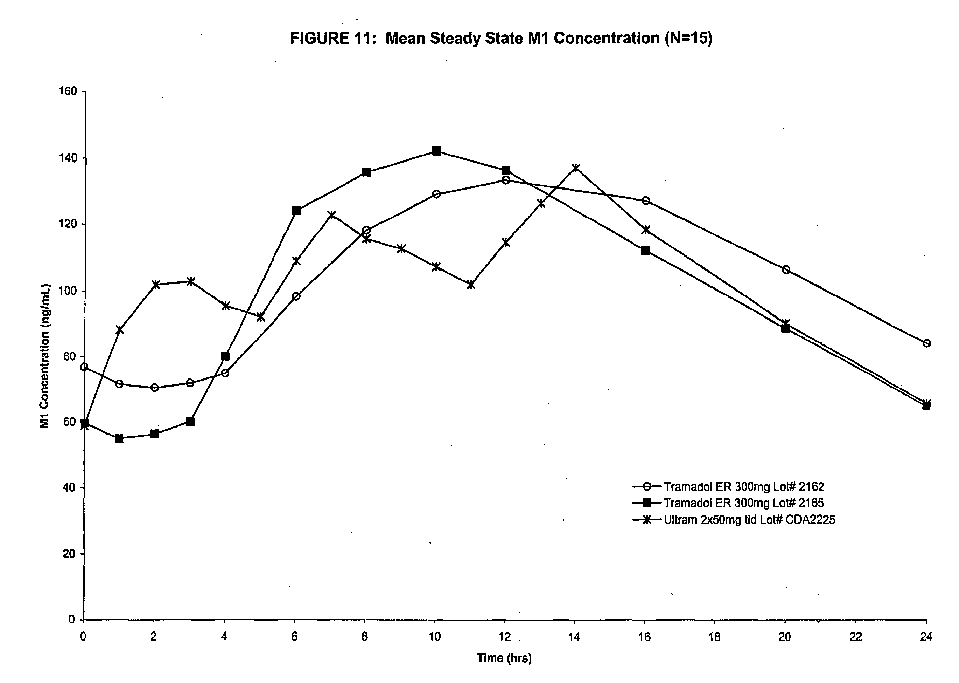

Figure 11 illustrates the mean plasma Desmethyltramadol concentrations on Day 1 following once a day Tramadol HCl ER Tablets (100 mg x 3) formulated according to Lot Nos. 2162 and 2165 for 5 Days vs. Ultram^{®} (50 mg x 2) t.i.d.

### EXAMPLE 2: 100 mg Tramadol HCl ER Tablets

The following 100 mg Tramadol HCl ER Tablet formulation was prepared: Tablet Core Formulation

The tablet core formulation was that of Example 1. The tablet core was prepared according to the process described in Example 1.

**Table 9: Coating Formulation**

| **Ingredients** | **Quantity (mg)** | **%** |
|---|---|---|
| Ethylcellulose (ETHOCEL® PR 100) | 9.73 | 73.00 (of coating polymer) |
| Polyvinylpyrrolidone (KOLLIDON® 90F) | 3.60 | 27.00 (of coating polymer) |
| Dibutyl Sebacate | 2.67 | 20.00 (of above polymer) |

| ***Total dry material:** 8.5% of the solution* | | |
|---|---|---|
| Ethyl Alcohol 200 Proof | 163.62 * | 95% (of total solvent) |
| Isopropyl Alcohol 99% | 8.61 * | 5% (of total solvent) |
| ***Coated Tablet*** | 120.00 | |

| | | |
|---|---|---|
| ** evaporated during process* | | |

The coating process was carried out with the following parameters:
- 30 psi spray pressure
- 40°C product temperature
- 5 g/min/kg spray rate

### EXAMPLE 3: 100 mg Tramadol HCl ER Tablets

The following 100 mg Tramadol HCl ER Tablet formulation was prepared: Tablet Core Formulation

The tablet core formulation was that of Example 1. Tablet Core Preparation

The tablet core was prepared according to the process described in Example 1.

**Table 10: Coating Formulation**

| | **mg/tablet** | | | |
|---|---|---|---|---|
| | **Lot#1** | **Lot#2** | **Lot#3** | **Lot#4** |
| **Ingredients** | **Quantity (mg)** | **Quantity (mg)** | **Quantity (mg)** | **Quantity (mg)** |
| Ethylcellulose (ETHOCEL® PR 100) | 9.87 | 9.87 | 9.60 | 9.60 |
| Polyvinylpyrrolidone (KOLLIDON® 90F) | 3.47 | 3.47 | 3.73 | 3.73 |
| Dibutyl Sebacate | 2.67 | 2.67 | 2.67 | 2.67 |
| Ethyl Alcohol 200 Proof | 153.94 * | 153.94 * | 153.94 * | 153.94 |
| Isopropyl Alcohol 99% USP | 8.09* | 8.09* | 8.09* | 8.09 |

| | | | | |
|---|---|---|---|---|
| * *evaporated during process* | | | | |

### Coating Preparation

The tablet core coating solution was prepared according to the process described in Example 1.

**Table 10: Coating Parameters:**

| **Parameter** | **Lot #1** | **Lot#2** | **Lot #3** | **Lot #4** |
|---|---|---|---|---|
| Inlet Temperature C° | 41-42 | 56-57 | 56-57 | 48.5-49.5 |
| Outlet Temperature C° | 32-33 | 44-45 | 44-45 | 38.5-39.5 |
| Bed Temperature C° | N/A | 45-46 | 45-46 | 37.5-38.5 |
| Spray Rate g/ min | 300 | 300-310 | 300-310 | 300 |
| Atomizing Air/Pattern Psi | 25/20 | 25/25 | 25/25 | 25/25 |
| Distance gun/Bed | 6" | 6" | 6" | 6" |
| Distance between guns | 6" | 6" | 6" | 6" |
| Pan speed rpm | 12.0 | 12.0 | 12.0 | 12.0 |

**Table 11: Dissolution Profile**

| | **% Dissolved** | | | |
|---|---|---|---|---|
| **Time (min.)** | **Lot #1** | **Lot#2** | **Lot #3** | **Lot #4** |
| | 0 | 0 | 0 | 0 |
| 120 | 0.5 | 3.34 | 7.81 | 5.13 |
| 240 | 0.85 | 7.94 | 26.68 | 15.23 |
| 360 | 1.39 | 13.06 | 50.97 | 34.94 |
| 480 | 2.43 | 20.72 | 70.16 | 54.58 |
| 600 | 4.04 | 30.15 | 83.27 | 70.10 |
| 720 | 6.89 | 41.77 | 91.40 | 81.89 |

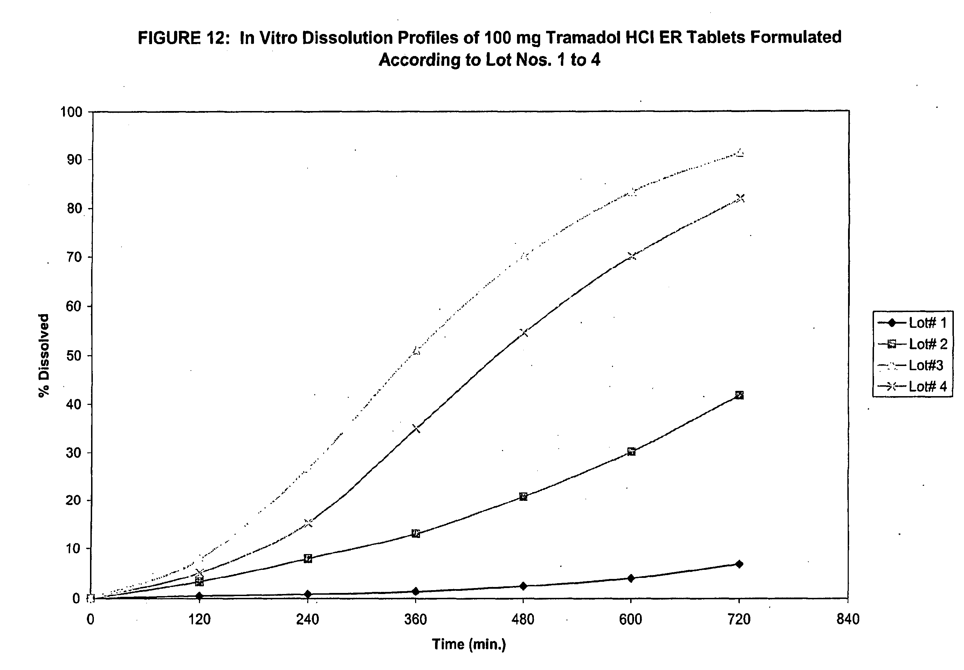

Figure 12 illustrates the *in vitro* dissolution profiles of Tramadol HCl 100 mg ER Tablets formulated according to Lot Nos.1-4.

### EXAMPLE 4: 200 mg Tramadol HCl ER Tablets

The following 200 mg Tramadol HCl ER Tablet formulation was prepared:

**Table 12a: Tablet Core Formulation**

| **Ingredients** | **Quantity (mg)** |
|---|---|
| Tramadol HCl | 200.00 |
| Polyvinyl Alcohol | 4.00 |
| Colloidal Silicon Dioxide (AEROSIL® 200) | 2.00 |
| Sodium Stearyl Fumarate | 2.00 |
| Purified Water | 83.20 * |
| ***Core Total Weight*** | ***208.00*** |

| | |
|---|---|
| ** evaporated during process* | |

### Tablet Core Preparation

The tablet core was prepared according to the process described in Example 1.

**Table 12b: Coating Formulation**

| **Ingredients** | **Quantity (mg)** |
|---|---|
| Ethylcellulose (ETHOCEL® PR100) | 12.28 |
| Polyvinylpyrrolidone (KOLLIDON® K90) | 6.05 |
| Dibutyl Sebacate NF | 3.67 |
| Ethyl Alcohol 200 Proof | 154.24* |
| Isopropyl Alcohol USP | 8.12 * |

| | |
|---|---|
| * *evaporated during process* | |

### Coating Preparation

The tablet core coating solution was prepared according to the process described in Example 1.

**Table 13: Dissolution Profile**

| **Time (min.)** | **% Dissolved** |
|---|---|
| 0 | 0 |
| 60 | 1.13 |
| 120 | 6.05 |
| 180 | 13.80 |
| 240 | 22.87 |
| 300 | 32.18 |
| 360 | 41.17 |
| 420 | 49.43 |
| 480 | 56.85 |
| 540 | 63.33 |
| 600 | 68.87 |
| 660 | 73.55 |
| 720 | 77.55 |
| 780 | 80.72 |
| 840 | 83.43 |
| 900 | 85.77 |
| 960 | 87.75 |
| 1020 | 89.20 |
| 1080 | 90.70 |
| 1140 | 91.62 |

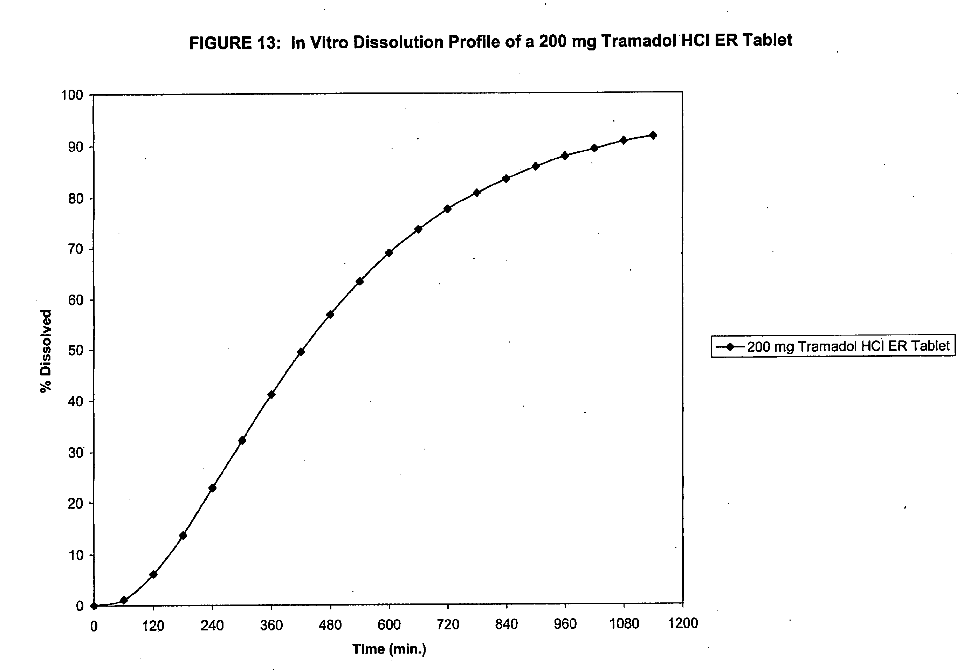

Figure 13 illustrates the *in vitro* dissolution profile of a 200 mg Tramadol HCl ER Tablet formulated according to Example 4.

### EXAMPLE 5: 200 mg Tramadol HCl Tablets

The following Tramadol HCl formulation was prepared: Tablet Core Formulation

The tablet core formulation was that of Example 4.

### Tablet Core Preparation

The tablet core was prepared according to the process described in Example 1.

**Table 14: Coating Formulation**

| **Ingredients** | **Quantity (mg)** | **%** |
|---|---|---|
| Ethylcellulose (ETHOCEL® PR 100) | 13.38 | 73.00 (of coating polymer) |
| Polyvinylpyrrolidone (KOLLIDON® 90F) | 4.95 | 27.00 (of coating polymer) |
| Dibutyl Sebacate | 3.67 | 20.00 (of above polymer) |

| *Total dry material: 9% of the solution* | | |
|---|---|---|
| Ethyl Alcohol 200 Proof | 211.32 * | 95 % (of total solvent) |
| Isopropyl Alcohol 99% | 11.12 * | 5% (of total solvent) |
| ***Coated Tablet*** | 230.00 | |

| | | |
|---|---|---|
| * *evaporated during process* | | |

The coating process was carried out with the following parameters:
- 30 psi spray pressure
- 40°C product temperature
- 5 g/min/kg spray rate

### EXAMPLE 6: 200 mg Tramadol HCl ER Tablets

The following Tramadol HCl formulation was prepared:

### Tablet Core Formulation

The tablet core formulation was that of Example 4.

### Tablet Core Preparation

The tablet core was prepared according to the process described in Example 1.

**Table 15: Coating Formulation**

| **Ingredient** | **Lot #5** | **Lot#6** | **Lot #7** |
|---|---|---|---|
| Ethylcellulose (ETHOCEL® PR 100) | 15.60 | 15.60 | 15.60 |
| Polyvinylpyrrolidone (KOLLIDON® 90F) | 6.07 | 6.07 | 6.07 |
| Dibutyl Sebacate | 4.33 | 4.33 | 4.33 |
| Ethyl Alcohol 200 Proof | 249.75* | 249.75* | 249.75* |
| Isopropyl Alcohol 99% | 13.15* | 13.15* | 13.15* |

| | | | |
|---|---|---|---|
| * *evaporated during process* | | | |

### Coating Preparation

The tablet core coating solution was prepared according to the process described in Example 1.

**Table 16: Coating Parameters**

| **Parameter** | **Lot #5** | **Lot#6** | **Lot #7** |
|---|---|---|---|
| Inlet Temperature C° | 49-50 | 50-51.5 | 50-51.5 |
| Outlet Temperature C° | 38.5-39.5 | 39.5-40.5 | 39.5-40.5 |
| Bed Temperature C° | 37.5-38.5 | 37.5-39 | 37.5-39 |
| Spray Rate g/min | 300 | 300 | 300 |
| Atomizing Air/ Pattern Psi | 25/25 | 25/25 | 25/25 |
| Distance gun/Bed | 6" | 6" | 6" |
| Distance between guns | 6" | 6" | 6" |
| Pan speed rpm | 12.0 | 12.0 | 12.0 |

### Dissolution Method

The dissolution method was performed according to the method described in Example 1.

**Table 17: Dissolution Profile**

| | **% Dissolved** | | |
|---|---|---|---|
| **Time (min.)** | **Lot #5** | **Lot#6** | **Lot #7** |
| 0 | 0 | 0 | 0 |
| 120 | 5.54 | 4.13 | 5.37 |
| 240 | 14.71 | 14.29 | 15.76 |
| 360 | 29.25 | 31.83 | 33.48 |
| 480 | 46.40 | 50.16 | 51.62 |
| 600 | N/A | 65.64 | 66.42 |
| 720 | N/A | 76.8 | 77.49 |

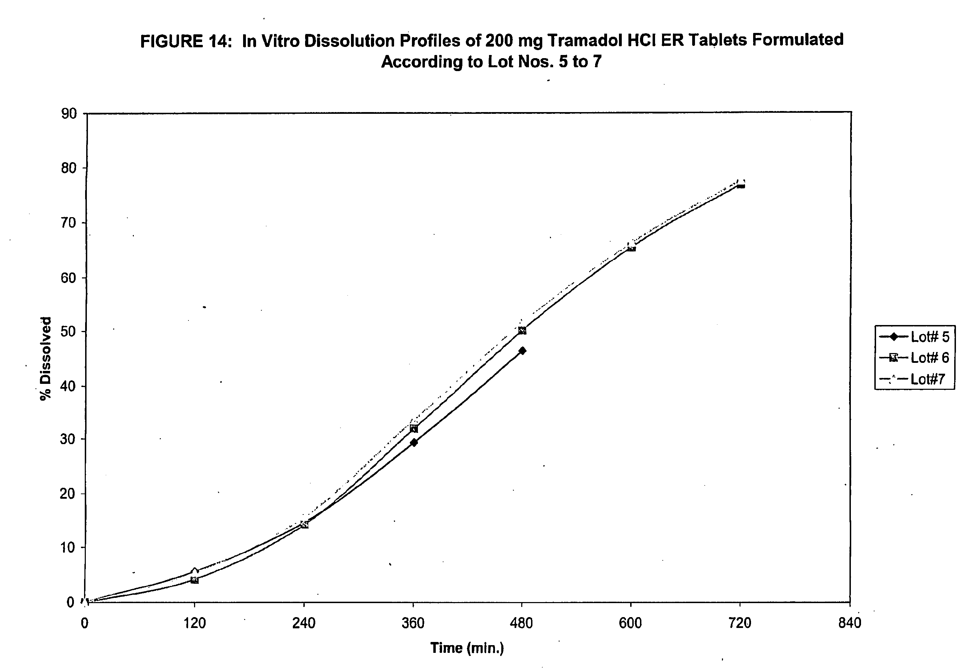

Figure 14 illustrates the *in vitro* dissolution profiles of 200 mg Tramadol HCl ER Tablets formulated according to Lot Nos. 5 to 7.

### EXAMPLE 7:

A two-way, crossover, open-label, single-dose, fasting, comparative biovailability study of Tramadol HCl Extended-Release Tablets (2 x 100 mg vs 1 x 200 mg) in normal healthy non-smoking male subjects was conducted.

### 7.1: Synopsis

Based on preliminary data from 12 completing subjects, the 200 mg strength Tramadol HCl ER Tablets are proportional to the 100 mg strength given as 2 x 100 mg.

### 7.2: Purpose of Study

This study was designed to determine the dosage strength proportionality of two strengths of Tramadol HCl ER Tablets (2 x 100 mg vs 1 x 200 mg) under single dose fasting conditions.

### 7.3: Study Design

A single-dose, open-label, two-way, two-sequence, crossover design. The treatments were separated by a one (1) week washout period. On day 1 of each period, subjects received one of the following treatments on two (2) separate occasions according to the randomization scheme
- Treatment A:: Two Tramadol HCl Extended Release 100 mg Tablets with 240 mL of water at 0.0 hour following a 10 hour overnight fast (Total Daily Dose = 200 mg)
- Treatment B:: One Tramadol HCl Extended Release 200 mg Tablet with 240 mL of water at 0.0 hour following a 10 hour overnight fast (Total Daily Dose = 200 mg)

### 7.4: Summary and Conclusions

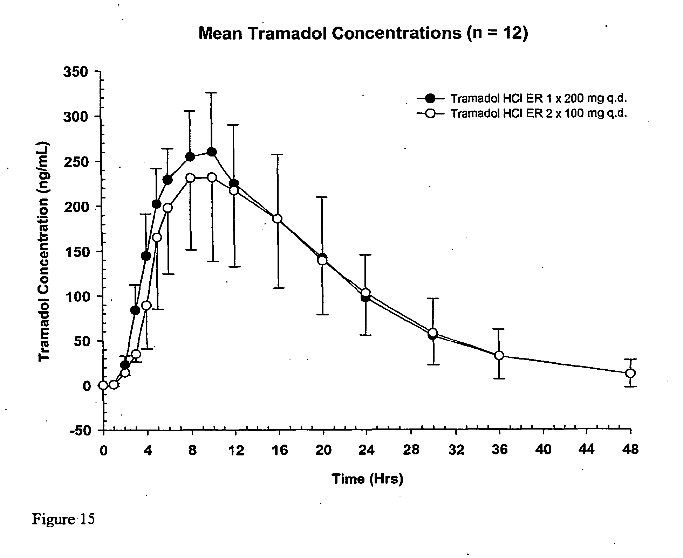

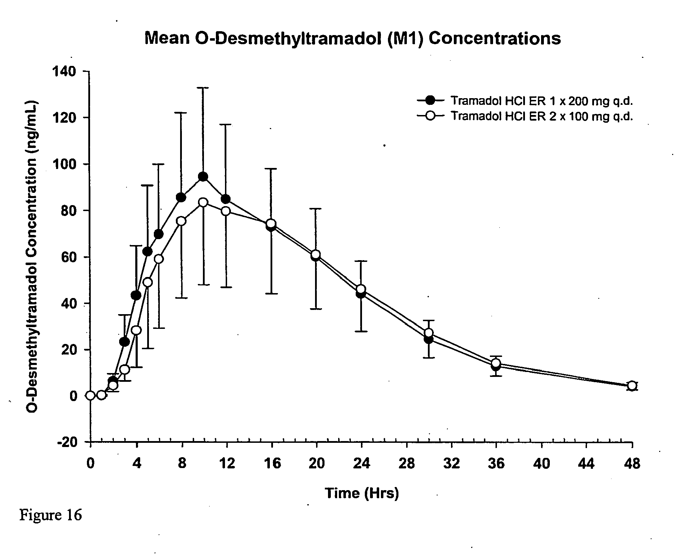

This study was intended to determine the dosage strength proportionality of two strengths of Tramadol HCl ER Tablets (2 x 100 mg vs 1 x 200 mg) under single dose fasting conditions. A total of 12 male subjects were dosed at Biovail Contract Research. Pharmacokinetic and statistical analyses were conducted with preliminary plasma data from 12 completing subjects for Tramadol, and M1 metabolite. The mean plasma concentrations vs time plots based on 12 completing subjects for tramadol, and M1 metabolite are presented in Figures 15 and 16, respectively. Individual pharmacokinetic parameters are shown in Tables 18a,18b,19a and 19b.

With all subjects, the ratio of geometric means (1 x 200 mg / 2 x 100 mg) for tramadol AUC₀₋ₜ and Cₘₐₓ were 1.11 and 1.17, respectively. The corresponding 90% confidence intervals were 97% - 125% and 103% - 133%, respectively. For the M1 metabolite, the ratio of geometric means (1 x 200 mg / 2 x 100 mg) for AUC₀₋ₜ and Cₘₐₓ were 1.05 and 1.11, respectively. The corresponding 90% confidence intervals were 96% - 116% and 102% - 121%, respectively.

In conclusion, the 200 mg strength Tramadol HCl ER Tablets were proportional to the 100 mg strength given as 2 x 100 mg since the 90 % confidence intervals for AUC₀₋ₜ and Cₘₐₓ for all analytes were found to be within 80 % -125.

**Table 18a: Tramadol PK (n=12)**

| **Cmax** | | | **Tramadol ER** | | | | |
|---|---|---|---|---|---|---|---|
| | | **200mg** | | | **2x00 mg** | | **200mg/2x100mg** |
| **Subject** | **Tmax** | **Cmax** | **In Cmax** | **Tmax** | **Cmax** | **In Cmax** | **Cmax Ratio** |
| **1** | 8 | 244.69 | 5.50 | 8 | 258.32 | 5.55 | 0.95 |
| **2** | 10 | 277.87 | 5.63 | 12 | 339.09 | 5.83 | 0.82 |
| **3** | 8 | 243.47 | 5.50 | 10 | 330.78 | 5.80 | 0.74 |
| **4** | 10 | 268.04 | 5.59 | 10 | 226.90 | 5.42 | 1.18 |
| **5** | 6 | 227.80 | 5.43 | 8 | 200.75 | 5.30 | 1.13 |
| **6** | 8 | 259.13 | 5.56 | 10 | 216.91 | 5.38 | 1.19 |
| **7** | 5 | 261.09 | 5.56 | 10 | 155.44 | 5.05 | 1.68 |
| **8** | 8 | 226.11 | 5.42 | 8 | 98.86 | 4.59 | 2.29 |
| **9** | 8 | 278.90 | 5.63 | 10 | 232.96 | 5.45 | 1.20 |
| **10** | 10 | 195.36 | 5.27 | 8 | 148.30 | 5.00 | 1.32 |
| **11** | 8 | 353.25 | 5.87 | 8 | 330.44 | 5.80 | 1.07 |
| **12** | 10 | 435.46 | 6.08 | 10 | 404.39 | 6.00 | 1.08 |
| | | | | | | | |
| **Mean** | **8.25** | **272.60** | **5.59** | **9.33** | **245.26** | **5.43** | **1.22** |
| **SD** | **1.60** | **64.10** | **0.21** | **1.30** | **90.97** | **0.41** | **0.41** |
| **CV** | **19.42** | **23.51** | **3.77** | **13.96** | **37.09** | **7.50** | **33.90** |
| **Min** | **5.00** | **195.36** | **5.27** | **8.00** | **98.86** | **4.59** | **0.74** |
| **Max** | **10.00** | **435.46** | **6.08** | **12.00** | **404.39** | **6.00** | **2.29** |
| **Geo Mean** | **8.09** | **266.71** | **5.58** | **9.25** | **228.55** | **5.42** | **1.17** |
| | | | | | | | |

| **Cmax Ratio** | | | | | | | |
|---|---|---|---|---|---|---|---|
| | | **Mean** | **Geo Mean** | | **90% C.I.** | **90% C.I. (Excluding Subject 8)** | |
| **200mg/2x100mg** | | **1.11** | **1.17(SAS)** | | **103-133** | **100-123** | |

**Table 18b: Tramadol PK (n=12)**

| **AUCt** | | **Tramadol ER** | | | |
|---|---|---|---|---|---|
| | **200mg** | | **2x100mg** | | **200mg/2x100mg** |
| **Subject** | **AUCt** | **In AUCt** | **AUCt** | **In AUCt** | **AUCt Ratio** |
| **1** | 4604.23 | 8.43 | 4446.02 | 8.40 | 1.04 |
| **2** | 6485.28 | 8.78 | 6343.27 | 8.76 | 1.02 |
| **3** | 5324.71 | 8.58 | 6067.74 | 8.71 | 0.88 |
| **4** | 6975.11 | 8.85 | 6292.41 | 8.75 | 1.11 |
| **5** | 4284.83 | 8.36 | 4045.08 | 8.31 | 1.06 |
| **6** | 3919.08 | 8.27 | 3944.65 | 8.28 | 0.99 |
| **7** | 4096.54 | 8.32 | 3521.10 | 8.17 | 1.16 |
| **8** | 3279.62 | 8.10 | 1382.53 | 7.23 | 2.37 |
| **9** | 4260.44 | 8.36 | 4423.57 | 8.39 | 0.96 |
| **10** | 2923.70 | 7.98 | 2714.23 | 7.91 | 1.08 |
| **11** | 4911.47 | 8.50 | 4882.50 | 8.49 | 1.01 |
| **12** | 8824.82 | 9.09 | 8323.14 | 9.03 | 1.06 |
| | | | | | |
| **Mean** | **4990.82** | **8.47** | **4698.85** | **8.37** | **1.14** |
| **SD** | **1687.36** | **0.32** | **1852.43** | **0.47** | **0.39** |
| **CV** | **33.81** | **3.73** | **39.42** | **5.60** | **34.34** |
| **Min** | **2923.70** | **7.98** | **1382.53** | **7.23** | **0.88** |
| **Max** | **8824.82** | **9.09** | **8323.14** | **9.03** | **2.37** |
| **Geo Mean** | **4759.37** | **8.46** | **4307.55** | **8.36** | **1.10** |
| | | | | | |

| **AUCt Radio** | | | | | |
|---|---|---|---|---|---|
| | | **Mean** | **Geo Mean** | **90% C.I.** | **90% C.I. (Excluding Subject 8)** |
| **200mg/2x100mg** | | **1.06** | **1.11(SAS)** | **97-125** | **99-107** |

Figure 15 illustrates the comparison of the mean tramadol plasma concentration-time profiles resulting from the oral administration of Tramadol HCl 100 mg ER tablets (2 x 100 mg once a day) and Tramadol HCl 200 mg ER tablets (1 x 200 mg once a day) formulated according to an embodiment of the present invention.

**Table 19a: M1 PK (n=12)**

| **Cmax** | | | **Tramadol ER** | | | | |
|---|---|---|---|---|---|---|---|
| | | **200mg** | | | **2x100 mg** | | **200mg/2x100mg** |
| **Subject** | **Tmax** | **Cmax** | **In Cmax** | **Tmax** | **Cmax** | **In Cmax** | **Cmax Ratio** |
| **1** | 10 | 104.54 | 4.65 | 12 | 110.02 | 4.70 | 0.95 |
| **2** | 10 | 98.85 | 4.59 | 12 | 105.22 | 4.66 | 0.94 |
| **3** | 10 | 96.35 | 4.57 | 10 | 97.49 | 4.58 | 0.99 |
| **4** | 20 | 23.51 | 3.16 | 16 | 25.50 | 3.24 | 0.92 |
| **5** | 12 | 75.83 | 4.33 | 20 | 66.86 | 4.20 | 1.13 |
| **6** | 10 | 142.03 | 4.96 | 10 | 118.45 | 4.77 | 1.20 |
| **7** | 10 | 110.74 | 4.71 | 10 | 84.57 | 4.44 | 1.31 |
| **8** | 10 | 102.54 | 4.63 | 8 | 58.72 | 4.07 | 1.75 |
| **9** | 8 | 127.10 | 4.84 | 10 | 135.05 | 4.91 | 0.94 |
| **10** | 10 | 93.51 | 4.54 | 10 | 90.31 | 4.50 | 1.04 |
| **11** | 10 | 139.85 | 4.94 | 10 | 107.20 | 4.67 | 1.30 |
| **12** | 16 | 30.01 | 3.40 | 16 | 27.27 | 3.31 | 1.10 |
| | | | | | | | |
| **Mean** | **11.33** | **95.40** | **4.44** | **12.00** | **85.56** | **4.34** | **1.13** |
| **SD** | **3.34** | **37.38** | **0.57** | **3.52** | **34.68** | **0.55** | **0.24** |
| **CV** | **29.47** | **39.18** | **12.91** | **29.30** | **40.53** | **12.66** | **21.07** |
| **Min** | **8.00** | **23.51** | **3.16** | **8.00** | **25.50** | **3.24** | **0.92** |
| **Max** | **20.00** | **142.03** | **4.96** | **20.00** | **135.05** | **4.91** | **1.75** |
| **Geo Mean** | **10.98** | **85.03** | **4.40** | **11.59** | **76.53** | **4.30** | **1.11** |
| | | | | | | | |

| **Cmax Ratio** | | | | | | | |
|---|---|---|---|---|---|---|---|
| | | **Mean** | **Geo Mean** | | **90% C.I.** | **90% C.I. (Excluding Subject 8)** | |
| **200mg/2x100mg** | | **1.12** | **1.11** | | **102-121** | **100-115** | |

**Table 19b: M1 PK (n=12)**

| **AUCt** | | **Tramadol ER** | | | |
|---|---|---|---|---|---|
| | **200mg** | | **2x100mg** | | **200mg/2x100mg** |
| **Subject** | **AUCt** | **In AUCt** | **AUCt** | **In AUCt** | **AUCt Ratio** |
| **1** | 2386.29 | 7.78 | 2404.81 | 7.79 | 0.99 |
| **2** | 2367.19 | 7.77 | 2244.63 | 7.72 | 1.05 |
| **3** | 2150.65 | 7.67 | 2139.59 | 7.67 | 1.01 |
| **4** | 718.62 | 6.58 | 750.69 | 6.62 | 0.96 |
| **5** | 1862.16 | 7.53 | 1773.47 | 7.48 | 1.05 |
| **6** | 2474.58 | 7.81 | 2359.88 | 7.77 | 1.0 |
| **7** | 2079.14 | 7.64 | 2137.31 | 7.67 | 0.97 |
| **8** | 1717.15 | 7.45 | 958.31 | 6.87 | 1.79 |
| **9** | 2262.49 | 7.72 | 2661.79 | 7.89 | 0.85 |
| **10** | 1657.30 | 7.41 | 1800.10 | 7.50 | 0.92 |
| **11** | 2373.89 | 7.77 | 2072.75 | 7.64 | 1.15 |
| **12** | 722.24 | 6.58 | 666.77 | 6.50 | 1.08 |
| | | | | | |
| **Mean** | **1897.64** | **7.48** | **1830.84** | **7.42** | **1.07** |
| **SD** | **611.42** | **0.44** | **674.61** | **0.48** | **0.24** |
| **CV** | **32.22** | **5.87** | **36.85** | **6.45** | **22.33** |
| **Min** | **718.62** | **6.58** | **666.77** | **6.50** | **0.85** |
| **Max** | **2474.58** | **7.81** | **2661.79** | **7.89** | **1.79** |
| **Geo Mean** | **1766.50** | **7.46** | **1676.26** | **7.41** | **1.05** |
| | | | | | |

| **AUCt Ratio** | | | | | |
|---|---|---|---|---|---|
| | | **Mean** | **Geo Mean** | **90% C.I.** | **90% C.I. (Excluding Subject 8)** |
| **200mg/2x100mg** | | **1.04** | **1.05** | **96-116** | **96-105** |

Figure 16 illustrates the comparison of the mean M1 plasma concentration-time profiles resulting from the oral administration of Tramadol HCl 100 mg ER tablets (2 x 100 mg once a day) and Tramadol HCl 200 mg ER tablets (1 x 200 mg once a day) formulated according to an embodiment of the present invention.

### EXAMPLE 8:

A two-way, crossover, open-label, single-dose, fasting, comparative biovailability study of Tramadol HCl Extended-Release Tablets (2 x 100 mg vs 1 x 200 mg) in normal healthy non-smoking male subjects was conducted.

### 8.1: Synopsis

Based on preliminary data from 23 completing subjects, the 200 mg strength Tramadol HCl ER Tablets are proportional to the 100 mg strength given as 2 x 100 mg.

### 8.2: Purpose of Study

This study was designed to determine the dosage strength proportionality of two strengths of Tramadol HCl ER Tablets (2 x 100 mg vs 1 x 200 mg) under single dose fasting conditions.

### 8.3: Study Design

A single-dose, open-label, two-way, two-sequence, crossover design. The treatments were separated by a one (1) week washout period. On day 1 of each period, subjects received one of the following treatments on two (2) separate occasions according to the randomization scheme
- Treatment A:: Two Tramadol HCl Extended Release 100 mg Tablets with 240 mL of water at 0.0 hour following a 10 hour overnight fast (Total Daily Dose = 200 mg)
- Treatment B:: One Tramadol HCl Extended Release 200 mg Tablet with 240 mL of water at 0.0 hour following a 10 hour overnight fast (Total Daily Dose = 200 mg)

### 8.4: Summary and Conclusions

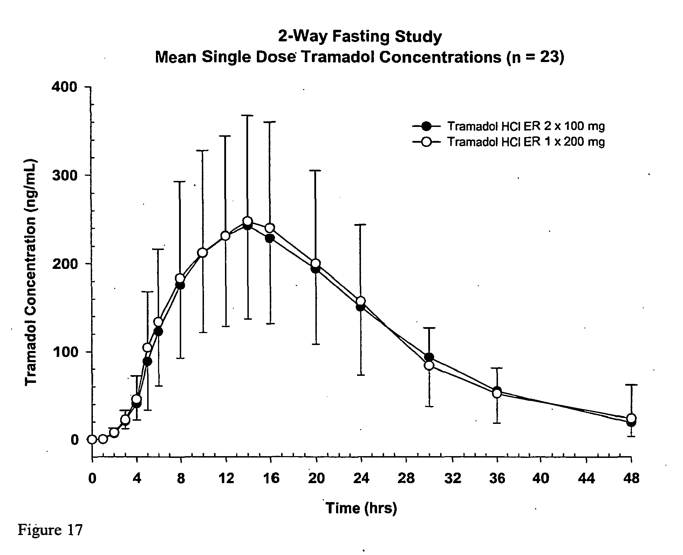

This study was intended to determine the dosage strength proportionality of two strengths of Tramadol HCl ER Tablets (2 x 100 mg vs 1 x 200 mg) under single dose fasting conditions. A total of 24 male subjects were dosed at Biovail Contract Research. Pharmacokinetic and statistical analyses were conducted with preliminary plasma data from 23 completing subjects for Tramadol, M1 and M5 metabolites. The mean plasma concentrations vs time plots based on 23 completing subjects for tramadol, M1 and M5 metabolites are presented in Figures 17, 18 and 19, respectively. Individual pharmacokinetic parameters are shown in Tables 20, 21 and 22.

With all subjects, the ratio of geometric means (1 x 200 mg / 2 x 100 mg) for tramadol AUC₀₋ₜ and Cₘₐₓ were 1.00 and 1.00, respectively. The corresponding 90% confidence intervals were 96.3 % - 104.17 % and 92.2 % - 109.12 %, respectively. For the M1 metabolite, the ratio of geometric means (1 x 200 mg / 2 x 100 mg) for AUC₀₋ₜ and Cₘₐₓ were 1.00 and 0.97, respectively. The corresponding 90% confidence intervals were 95.6 % - 104.61 % and 90.3 % - 104.39 %, respectively. For the M5 metabolite, the ratio of geometric means (1 x 200 mg / 2 x 100 mg) for AUC₀₋ₜ and Cₘₐₓ were 0.98 and 0.99, respectively. The corresponding 90% confidence intervals were 92.6 % - 104.72 % and 90.9 % - 107.25 %, respectively.

In conclusion, the 200 mg strength Tramadol HCl ER Tablets were proportional to the 100 mg strength given as 2 x 100 mg since the 90 % confidence intervals for AUC₀₋ₜ and Cₘₐₓ for all analytes were found to be within 80%-125%.

**Table 20: Summary of Pharmacokinetic Parameters for Tramadol**

| **Tramadol** | | | | | | | | | | |
|---|---|---|---|---|---|---|---|---|---|---|
| **Subject** | **B** | | | | **A** | | | | **B/ A Ratio** | |
| | **Tramadol HCl ER 1 x 200 mg** | | | | **Tramadol HCl ER 2 x 100 mg** | | | | | |
| | **AUC** | **Cmax** | **Tmax** | | **AUC** | **Cmax** | **Tmax** | | **AUC** | **Cmax** |
| **1** | 3772.72 | 125.97 | 24.00 | | 4465.15 | 207.76 | 8.00 | | 0.84 | 0.61 |
| **2** | 6989.42 | 361.35 | 14.00 | | 7563.59 | 416.83 | 12.00 | | 0.92 | 0.87 |
| **3** | 9967.41 | 440.03 | 14.00 | | 8773.22 | 374.54 | 16.00 | | 1.14 | 1.17 |
| **4** | 10503.26 | 528.11 | 16.00 | | 10727.13 | 517.12 | 14.00 | | 0.98 | 1.02 |
| **5** | 4315.08 | 244.26 | 12.00 | | 4439.38 | 209.95 | 16.00 | | 0.97 | 1.16 |
| **7** | 3539.99 | 151.93 | 16.00 | | 4128.98 | 234.12 | 8.00 | | 0.86 | 0.65 |
| **8** | 7752.67 | 368.21 | 14.00 | | 6369.64 | 369.49 | 8.00 | | 1.22 | 1.00 |
| **9** | 5413.87 | 281.50 | 14.00 | | 5561.14 | 244.18 | 16.00 | | 0.97 | 1.15 |
| **10** | 3640.04 | 162.12 | 12.00 | | 4354.42 | 179.31 | 14.00 | | 0.84 | 0.90 |
| **11** | 3442.46 | 147.72 | 20.00 | | 3394.52 | 162.28 | 16.00 | | 1.01 | 0.91 |
| **12** | 2763.51 | 190.15 | 10.00 | | 2722.78 | 150.02 | 10.00 | | 1.01 | 1.27 |
| **13** | 3746.10 | 203.07 | 8.00 | | 4084.60 | 183.27 | 16.00 | | 0.92 | 1.11 |
| **14** | 6394.67 | 311.42 | 16.00 | | 5107.43 | 239.83 | 8.00 | | 1.25 | 1.30 |
| **15** | 3093.78 | 178.86 | 12.00 | | 2806.92 | 128.49 | 16.00 | | 1.10 | 1.39 |
| **16** | 8363.96 | 465.15 | 10.00 | | 7811.02 | 369.66 | 14.00 | | 1.07 | 1.26 |
| **17** | 2410.07 | 130.62 | 14.00 | | 2647.52 | 135.74 | 14.00 | | 0.91 | 0.96 |
| **18** | 9336.37 | 469.34 | 8.00 | | 9475.49 | 409.69 | 16.00 | | 0.99 | 1.15 |
| **19** | 9125.11 | 437.92 | 14.00 | | 8440.88 | 322.16 | 14.00 | | 1.08 | 1.36 |
| **20** | 4983.63 | 209.73 | 10.00 | | 4444.82 | 226.82 | 12.00 | | 1.12 | 0.92 |
| **21** | 3337.64 | 151.75 | 12.00 | | 3818.74 | 220.36 | 12.00 | | 0.87 | 0.69 |
| **22** | 2868.24 | 164.88 | 16.00 | | 2595.66 | 163.49 | 8.00 | | 1.11 | 1.01 |
| **23** | 4233.82 | 179.29 | 16.00 | | 4310.37 | 238.80 | 16.00 | | 0.98 | 0.75 |
| **24** | 7311.78 | 329.98 | 20.00 | | 7105.46 | 307.21 | 20.00 | | 1.03 | 1.07 |
| | | | | | | | | | | |
| **Mean** | **5535.03** | **271.02** | **14.00** | | **5441.25** | **261.35** | **13.22** | | **1.01** | **1.03** |
| **SD** | **2591.09** | **128.34** | **3.86** | | **2376.20** | **104.90** | **3.45** | | **0.11** | **0.22** |
| **CV** | **46.81** | **47.36** | **27.58** | | **43.67** | **40.14** | **26.10** | | **11.3 2** | **21.60** |
| **Geo Mean** | **4992.59** | **244.00** | **13.51** | | **4979.67** | **242.83** | **12.74** | | **1.00** | **1.00** |
| **Min** | **2410.07** | **125.97** | **8.00** | | **2595.66** | **128.49** | **8.00** | | **0.84** | **0.61** |
| **Max** | **10503.26** | **528.11** | **24.00** | | **10727.13** | **517.12** | **20.00** | | **1.25** | **1.39** |

| | | | | | | | | | | |
|---|---|---|---|---|---|---|---|---|---|---|
| | **1 x 200 mg/ 2 x 100 mg Ratio** | | | | | | | | | |
| | **Means** | **Geo Means** | **LS Means %** | **90% CI** | | | | | | |
| **AUC** | **1.02** | **1.00** | **100.11** | **96.3-104.17** | | | | | | |
| **Cmax** | **1.04** | **1.00** | **100.29** | **92.2-109.12** | | | | | | |

Figure 17 illustrates the mean plasma Tramadol concentrations (ng/ml) over time after two 100 mg Tramadol HCl ER Tablets formulated according to an embodiment of the present invention or after one 200 mg Tramadol HCl ER Tablet formulated according to an embodiment of the present invention following a 10 hour overnight fast.

**Table 21: Summary of Pharmacokinetic Parameters for M1**

| **M1** | | | | | | | | | | |
|---|---|---|---|---|---|---|---|---|---|---|
| **Subject** | **B** | | | | **A** | | | | | |
| | **Tramadol HCl ER 1 x 200 mg** | | | | **Tramadol HCl ER 2 x 100 mg** | | | | **B / A Ratio** | |
| | **AUC** | **Cmax** | **Tmax** | | **AUC** | **Cmax** | **Tmax** | | **AUC** | **Cmax** |
| **1** | 1672.89 | 57.88 | 24.00 | | 2022.04 | 80.50 | 16.00 | | 0.83 | 0.72 |
| **2** | 970.95 | 43.44 | 14.00 | | 1156.80 | 54.21 | 12.00 | | 0.84 | 0.80 |
| **3** | 1774.74 | 69.27 | 20.00 | | 1789.62 | 70.05 | 20.00 | | 0.99 | 0.99 |
| **4** | 1076.19 | 48.16 | 16.00 | | 1050.38 | 44.32 | 16.00 | | 1.02 | 1.09 |
| **5** | 1979.48 | 96.47 | 14.00 | | 1657.77 | 72.52 | 14.00 | | 1.19 | 1.33 |
| **7** | 953.92 | 36.57 | 24.00 | | 1330.47 | 68.82 | 8.00 | | 0.72 | 0.53 |
| **8** | 1414.94 | 61.68 | 24.00 | | 1116.16 | 53.67 | 12.00 | | 1.27 | 1.15 |
| **9** | 2484.11 | 113.99 | 14.00 | | 2406.74 | 108.07 | 20.00 | | 1.03 | 1.05 |
| **10** | 1721.50 | 73.76 | 14.00 | | 1906.22 | 74.23 | 16.00 | | 0.90 | 0.99 |
| **11** | 1829.82 | 80.76 | 24.00 | | 1850.88 | 88.60 | 16.00 | | 0.99 | 0.91 |
| **12** | 2144.82 | 109.30 | 10.00 | | 2095.44 | 101.89 | 14.00 | | 1.02 | 1.07 |
| **13** | 2111.31 | 90.98 | 10.00 | | 2036.17 | 89.08 | 20.00 | | 1.04 | 1.02 |
| **14** | 2130.45 | 93.13 | 16.00 | | 2122.92 | 91.06 | 16.00 | | 1.00 | 1.02 |
| **15** | 2360.46 | 115.08 | 12.00 | | 2128.12 | 97.80 | 16.00 | | 1.11 | 1.18 |
| **16** | 3234.30 | 130.32 | 12.00 | | 3507.19 | 134.18 | 20.00 | | 0.92 | 0.97 |
| **17** | 1922.21 | 90.47 | 16.00 | | 1977.41 | 91.02 | 14.00 | | 0.97 | 0.99 |
| **18** | 3490.68 | 135.80 | 12.00 | | 3521.34 | 138.38 | 16.00 | | 0.99 | 0.98 |
| **19** | 1154.33 | 49.30 | 16.00 | | 991.16 | 38.21 | 20.00 | | 1.16 | 1.29 |
| **20** | 2303.91 | 96.21 | 14.00 | | 2269.27 | 110.63 | 14.00 | | 1.02 | 0.87 |
| **21** | 1853.29 | 72.48 | 16.00 | | 1983.58 | 95.39 | 12.00 | | 0.93 | 0.76 |
| **22** | 2637.81 | 153.39 | 16.00 | | 2464.84 | 144.41 | 8.00 | | 1.07 | 1.06 |
| **23** | 1970.96 | 81.98 | 24.00 | | 1870.67 | 96.76 | 16.00 | | 1.05 | 0.85 |
| **24** | 1867.23 | 89.74 | 20.00 | | 1673.09 | 78.73 | 20.00 | | 1.12 | 1.14 |
| | | | | | | | | | | |
| **Mean** | **1959.14** | **86.53** | **16.61** | | **1953.40** | **87.94** | **15.48** | | **1.01** | **0.99** |
| **SD** | **639.46** | **30.56** | **4.69** | | **647.00** | **27.94** | **3.58** | | **0.12** | **0.18** |
| **CV** | **32.64** | **35.32** | **28.23** | | **33.12** | **31.77** | **23.12** | | **12.1 1** | **18.40** |
| **Geo Mean** | **1857.57** | **81.15** | **16.00** | | **1855.19** | **83.46** | **15.03** | | **1.00** | **0.97** |
| **Min** | **953.92** | **36.57** | **10.00** | | **991.16** | **38.21** | **8.00** | | **0.72** | **0.53** |
| **Max** | **3490.68** | **153.39** | **24.00** | | **3521.34** | **144.41** | **20.00** | | **1.27** | **1.33** |

| | **1 x 200 mg / 2 x 100 mg Ratio** | | | | | | | | | |
|---|---|---|---|---|---|---|---|---|---|---|
| | **Means** | **Geo Means** | **LS Means %** | **90% CI** | | | | | | |
| **AUC** | **1.00** | **1.00** | **100.01** | **95.6-104.61** | | | | | | |
| **Cmax** | **0.98** | **0.97** | **97.06** | **90.3-104.39** | | | | | | |

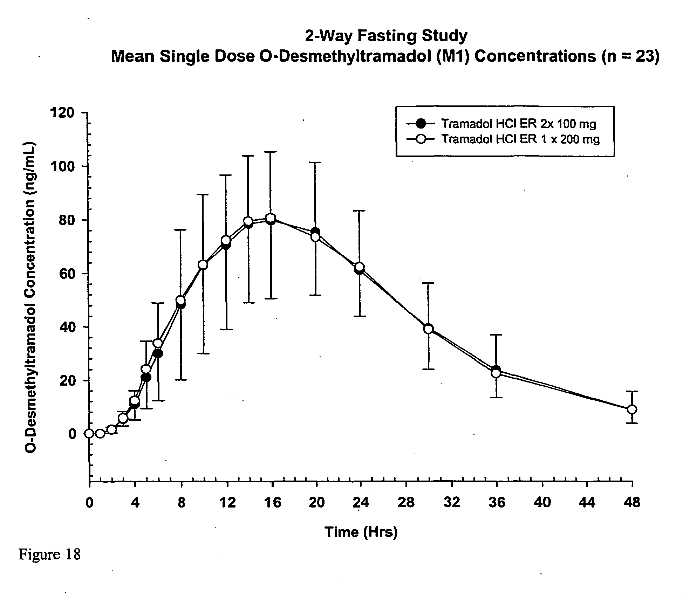

Figure 18 illustrates the mean plasma M1 concentrations (ng/ml) over time after two 100 mg Tramadol HCl ER Tablets formulated according to an embodiment of the present invention or after one 200 mg Tramadol HCl ER Tablet formulated according to an embodiment of the present invention following a 10 hour overnight fast.

**Table 22: Summary of Pharmacokinetic Parameters for M5:**

| **M5** | | | | | | | | | | |
|---|---|---|---|---|---|---|---|---|---|---|
| | **B** | | | | **A** | | | | **B / A Ratio** | |
| | **Tramadol HCl ER 1 x 200 mg** | | | | **Tramadol HCl ER 2 x 100 mg** | | | | | |
| **Subject** | **AUC** | **Cmax** | **Tmax** | | **AUC** | **Cmax** | **Tmax** | | **AUC** | **Cmax** |
| **1** | 586.48 | 19.01 | 30.00 | | 669.49 | 26.43 | 16.00 | | 0.88 | 0.72 |
| **2** | 1011.03 | 39.82 | 20.00 | | 1156.49 | 43.27 | 16.00 | | 0.87 | 0.92 |
| **3** | 479.82 | 18.04 | 20.00 | | 606.97 | 21.98 | 24.00 | | 0.79 | 0.82 |
| **4** | 597.78 | 23.35 | 24.00 | | 709.78 | 25.47 | 24.00 | | 0.84 | 0.92 |
| **5** | 638.62 | 28.06 | 12.00 | | 652.44 | 28.32 | 16.00 | | 0.98 | 0.99 |
| **7** | 505.59 | 18.07 | 20.00 | | 794.87 | 34.53 | 14.00 | | 0.64 | 0.52 |
| **8** | 1105.91 | 49.06 | 24.00 | | 1022.61 | 39.84 | 14.00 | | 1.08 | 1.23 |
| **9** | 765.91 | 32.10 | 14.00 | | 860.60 | 35.68 | 20.00 | | 0.89 | 0.90 |
| **10** | 986.85 | 41.59 | 14.00 | | 973.22 | 38.01 | 16.00 | | 1.01 | 1.09 |
| **11** | 492.42 | 20.37 | 24.00 | | 565.44 | 24.25 | 16.00 | | 0.87 | 0.84 |
| **12** | 1566.17 | 75.38 | 16.00 | | 1481.53 | 71.45 | 14.00 | | 1.06 | 1.06 |
| **13** | 1044.20 | 43.60 | 16.00 | | 974.90 | 39.62 | 20.00 | | 1.07 | 1.10 |
| **14** | 843.42 | 35.36 | 16.00 | | 809.01 | 33.16 | 16.00 | | 1.04 | 1.07 |
| **15** | 967.94 | 46.14 | 14.00 | | 867.23 | 36.72 | 16.00 | | 1.12 | 1.26 |
| **16** | 987.92 | 44.60 | 20.00 | | 1019.98 | 38.32 | 20.00 | | 0.97 | 1.16 |
| **17** | 630.21 | 29.71 | 16.00 | | 607.88 | 25.47 | 20.00 | | 1.04 | 1.17 |
| **18** | 1889.26 | 66.43 | 14.00 | | 1865.13 | 67.08 | 20.00 | | 1.01 | 0.99 |
| **19** | 813.41 | 31.21 | 20.00 | | 584.26 | 19.99 | 20.00 | | 1.39 | 1.56 |
| **20** | 841.90 | 34.50 | 14.00 | | 640.29 | 27.74 | 14.00 | | 1.31 | 1.24 |
| **21** | 829.00 | 33.19 | 16.00 | | 940.72 | 42.90 | 14.00 | | 0.88 | 0.77 |
| **22** | 617.32 | 33.20 | 16.00 | | 639.87 | 37.09 | 10.00 | | 0.96 | 0.90 |
| **23** | 908.93 | 38.57 | 24.00 | | 759.38 | 40.38 | 16.00 | | 1.20 | 0.96 |
| **24** | 743.29 | 33.02 | 20.00 | | 717.33 | 30.83 | 20.00 | | 1.04 | 1.07 |
| | | | | | | | | | | |
| **Mean** | **863.19** | **36.28** | **18.43** | | **866.06** | **36.02** | **17.22** | | **1.00** | **1.01** |
| **SD** | **334.21** | **14.20** | **4.51** | | **308.56** | **12.52** | **3.45** | | **0.17** | **0.22** |
| **CV** | **38.72** | **39.15** | **24.47** | | **35.63** | **34.76** | **20.04** | | **16.5 9** | **21.38** |
| **Geo Mean** | **812.38** | **33.87** | **17.94** | | **825.19** | **34.29** | **16.88** | | **0.98** | **0.99** |
| **Min** | **479.82** | **18.04** | **12.00** | | **565.44** | **19.99** | **10.00** | | **0.64** | **0.52** |
| **Max** | **1889.26** | **75.38** | **30.00** | | **1865.13** | **71.45** | **24.00** | | **1.39** | **1.56** |

| | | | | | | | | | | |
|---|---|---|---|---|---|---|---|---|---|---|
| | **1 x 200 mg/ 2 x 100 mg Ratio** | | | | | | | | | |
| | **Means** | **Geo Means** | **LS Means %** | **90% CI** | | | | | | |
| **AUC** | **1.00** | **0.98** | **98.45** | **92.6-104.72** | | | | | | |
| **Cmax** | **1.01** | **0.99** | **98.77** | **90.9-107.25** | | | | | | |

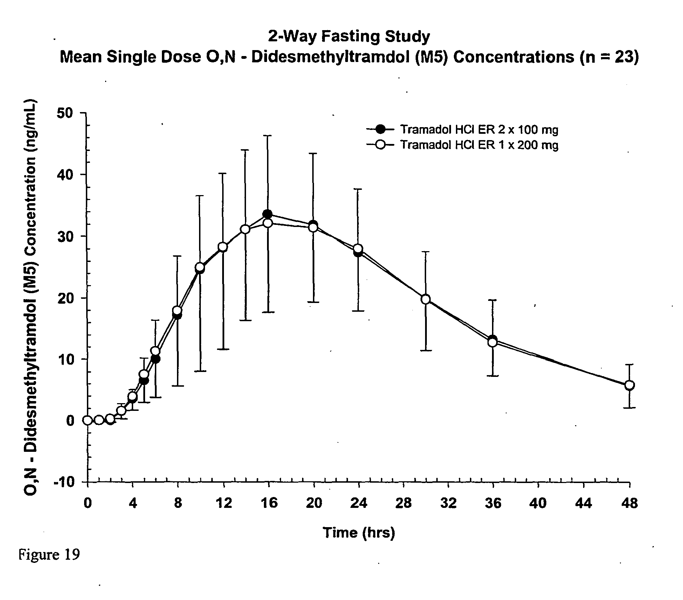

Figure 19 illustrates the mean plasma M5 concentrations (ng/ml) over time after two 100 mg Tramadol HCl ER Tablets formulated according to an embodiment of the present invention or after one 200 mg Tramadol HCl ER Tablet formulated according to an embodiment of the present invention following a 10 hour overnight fast.

### EXAMPLE 9:

A two-way, crossover, open-label, single-dose, food effect, comparative biovailability study of Tramadol HCl Extended-Release 200 mg Tablets in normal healthy non-smoking male subjects was conducted.

### 9.1: Synopsis

Based on preliminary data from 20 completing subjects, the presence of food significantly decreased the rate and extent of absorption of tramadol, M1 and M5 metabolites of Tramadol HCl ER tablets 200 mg following single dose administration.

### 9.2: Purpose of Study

This study was designed to evaluate the effect of food on Tramadol HCl ER 200 mg Tablets following single dose administration.

### 9.3: Study Design

A single-dose, open-label, two-way, two-sequence, crossover design. The treatments were separated by a one (1) week washout period. On day 1 of each period, subjects received one of the following treatments following a 10 hour overnight fast on two (2) separate occasions according to the randomization scheme
- Treatment A:: One Tramadol HCl Extended Release 200 mg Tablet with 240 mL of water at 0.0 hour within 5 minutes of complete ingestion of a high fat content breakfast.
- Treatment B:: One Tramadol HCl Extended Release 200 mg Tablet with 240 mL of water at 0.0 hour following a 10 hour overnight fast.

### 9.4: Summary and Conclusions

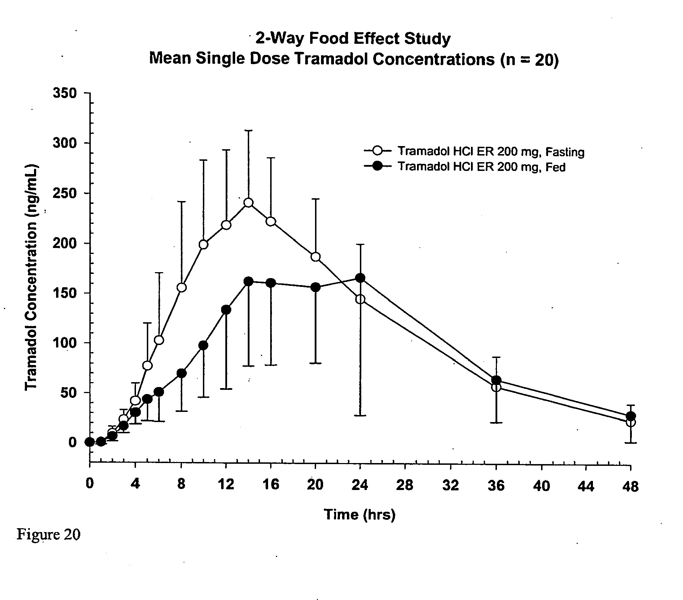

This study was intended to evaluate the effect of food on Tramadol HCl ER 200 mg Tablets following single dose administration. A total of 24 male subjects were dosed at Biovail Contract Research. Pharmacokinetic and statistical analyses were conducted with preliminary plasma data from 22 completing subjects. The mean plasma concentrations vs time plots based on 20 completing subjects for tramadol, M1 and M5 metabolites are presented in Figures 20, 21 and 22, respectively. Individual pharmacokinetic parameters are shown in Tables 23, 24 and 25.

With all subjects, the ratio of geometric means (Fed / Fasting) for tramadol AUC₀₋ₜ and Cₘₐₓ were 0.76 and 0.73, respectively. For the M1 metabolite, the ratio of geometric means (Fed / Fasting) for AUC₀₋ₜ and Cₘₐₓ were 0.75 and 0.76, respectively. For the M5 metabolite, the ratio of geometric means (Fed / Fasting) for AUC₀₋ₜ and Cₘₐₓ were 0.73 and 0.73, respectively.

When data analysis was carried out in the absence of subject #12 and #18, the ratio of geometric means for tramadol AUC₀₋ₜ and Cₘₐₓ were 0.79 and 0.73, respectively. For the M1 metabolite, the ratio of geometric means (Fed / Fasting) for AUC₀₋ₜ and Cₘₐₓ were 0.78 and 0.76, respectively. For the M5 metabolite, the ratio of geometric means (Fed / Fasting) for AUC₀₋ₜ and Cₘₐₓ were 0.75 and 0.72, respectively.

Based on the results, it was concluded that a significant food effect was observed for Tramadol HCl ER 200 mg Tablets. In the presence of food, the rate and extent of absorption of tramadol and its metabolites resulting from a single dose of Tramadol HCl ER 200 mg Tablet were significantly lower when compared to administration without food.

**Table 23: Summary of Pharmacokinetic Parameters for Tramadol**

| **Tramadol** | | | | | | | | | | |
|---|---|---|---|---|---|---|---|---|---|---|
| | | | | | | | | | | |
| | **Tramadol ER 200 mg, Fed** | | | | **Tramadol ER 200 mg, Fasting** | | | | **Fed / Fasting** | |
| **Subject** | **AUC** | **Cmax** | **Tmax** | | **AUC** | **Cmax** | **Tmax** | | **AUC** | **Cmax** |
| **1** | 3944.30 | 149.33 | 24.00 | | 4013.96 | 205.64 | 14.00 | | 0.98 | 0.73 |
| **2** | 4078.64 | 139.34 | 24.00 | | 6969.14 | 355.35 | 14.00 | | 0.59 | 0.39 |
| **3** | 6818.20 | 251.76 | 20.00 | | 6939.38 | 342.62 | 10.00 | | 0.98 | 0.73 |
| **4** | 2021.34 | 72.15 | 24.00 | | 4218.92 | 234.01 | 14.00 | | 0.48 | 0.31 |
| **5** | 2330.34 | 142.73 | 20.00 | | 2848.84 | 140.20 | 16.00 | | 0.82 | 1.02 |
| **6** | 4636.19 | 225.35 | 20.00 | | 5154.28 | 340.84 | 12.00 | | 0.90 | 0.66 |
| **7** | 1901.40 | 128.41 | 14.00 | | 3554.14 | 173:63 | 16.00 | | 0.53 | 0.74 |
| **8** | 4585.55 | 137.09 | 14.00 | | 5674.75 | 263.32 | 14.00 | | 0.81 | 0.52 |
| **9** | 7727.96 | 320.14 | 14.00 | | 7827.31 | 290.33 | 20.00 | | 0.99 | 1.10 |
| **10** | 3783.07 | 170.80 | 14.00 | | 4258.28 | 203.68 | 16.00 | | 0.89 | 0.84 |
| **11** | 7079.58 | 263.39 | 14.00 | | 7252.23 | 285.04 | 14.00 | | 0.98 | 0.92 |
| **13** | 3342.06 | 171.38 | 16.00 | | 3019.69 | 138.26 | 12.00 | | 1.11 | 1.24 |
| **15** | 4424.02 | 190.87 | 16.00 | | 4935.58 | 255.62 | 14.00 | | 0.90 | 0.75 |
| **16** | 7296.94 | 328.00 | 16.00 | | 6632.83 | 342.80 | 12.00 | | 1.10 | 0.96 |
| **17** | 1070.08 | 43.56 | 4.00 | | 8377.65 | 384.17 | 10.00 | | 0.13 | 0.11 |
| **20** | 4209.33 | 215.06 | 14.00 | | 5340.01 | 288.25 | 12.00 | | 0.79 | 0.75 |
| **21** | 3451.99 | 173.23 | 14.00 | | 3886.46 | 205.50 | 10.00 | | 0.89 | 0.84 |
| **22** | 3937.39 | 155.94 | 16.00 | | 3596.43 | 152.88 | 14.00 | | 1.09 | 1.02 |
| **23** | 4539.31 | 200.08 | 20.00 | | 5569.65 | 244.83 | 16.00 | | 0.82 | 0.82 |
| **24** | 8472.81 | 669.76 | 24.00 | | 6400.85 | 312.84 | 16.00 | | 1.32 | 2.14 |
| | | | | | | | | | | |
| **Mean** | **4482.52** | **207.42** | **17.10** | | **5323.52** | **257.99** | **13.80** | | **0.85** | **0.83** |
| **SD** | **2042.83** | **130.14** | **4.96** | | **1647.23** | **75.37** | **2.50** | | **0.26** | **0.41** |
| **CV** | **45.57** | **62.74** | **29.02** | | **30.94** | **29.21** | **18.15** | | **30.9 3** | **49.48** |
| **Geo Mean** | **3999.75** | **178.85** | **16.16** | | **5076.49** | **246.69** | **13.59** | | **0.79** | **0.73** |
| **Min** | **1070.08** | **43.56** | **4.00** | | **2848.84** | **138.26** | **10.00** | | **0.13** | **0.11** |
| **Max** | **8472.81** | **669.76** | **24.00** | | **8377.65** | **384.17** | **20.00** | | **1.32** | **2.14** |

| | | | | | | | | | | |
|---|---|---|---|---|---|---|---|---|---|---|
| | **Fed/ Fasting Ratio** | | | | **Fed / Fasting Ratio (Including Subject #12 and #18 who vomited during post-dose)** | | | | | |
| | | **Geo Means** | **LS Means %** | | | **Geo Means** | **LS Means %** | | | |
| **AUC** | | **0.79** | **78.93** | | **AUC** | **0.76** | **75.62** | | | |
| **Cmax** | | **0.73** | **72.27** | | **Cmax** | **0.73** | **72.38** | | | |

Figure 20 illustrates the mean plasma Tramadol concentrations (ng/ml) over time after a single dose of one 200 mg Tramadol HCl ER Tablet formulated according to an embodiment of the present invention under fasting or fed conditions.

**Table 24: Summary of Pharmacokinetic Parameters for M1:**

| **M1** | | | | | | | | | | |
|---|---|---|---|---|---|---|---|---|---|---|
| | **Tramadol ER 200 mg, Fed** | | | | **Tramadol ER 200 mg, Fasting** | | | | **Fed/ Fasting** | |
| **Subject** | **AUC** | **Cmax** | **Tmax** | | **AUC** | **Cmax** | **Cmax** | | **AUC** | **Cmax** |
| **1** | 1921.05 | 75.67 | 24.00 | | 2116.84 | 90.06 | 20.00 | | 0.91 | 0.84 |
| **2** | 1278.59 | 45.95 | 24.00 | | 2158.62 | 86.81 | 20.00 | | 0.59 | 0.53 |
| **3** | 1159.73 | 42.64 | 20.00 | | 1183.17 | 46.50 | 10.00 | | 0.98 | 0.92 |
| **4** | 542.48 | 19.92 | 24.00 | | 1223.87 | 58.17 | 14.00 | | 0.44 | 0.34 |
| **5** | 1894.84 | 117.77 | 20.00 | | 1982.93 | 96.08 | 20.00 | | 0.96 | 1.23 |
| **6** | 1887.04 | 91.91 | 20.00 | | 1761.13 | 105.91 | 12.00 | | 1.07 | 0.87 |
| **7** | 1130.02 | 60.88 | 16.00 | | 1901.17 | 77.45 | 16.00 | | 0.59 | 0.79 |
| **8** | 1674.77 | 51.91 | 24.00 | | 2013.76 | 75.57 | 16.00 | | 0.83 | 0.69 |
| **9** | 1511.07 | 52.51 | 24.00 | | 1487.99 | 53.84 | 24.00 | | 1.02 | 0.98 |
| **10** | 1322.65 | 54.89 | 16.00 | | 1702.91 | 70.92 | 16.00 | | 0.78 | 0.77 |
| **11** | 1335.58 | 51.01 | 20.00 | | 1286.31 | 46.10 | 24.00 | | 1.04 | 1.11 |
| **13** | 2269.80 | 100.16 | 16.00 | | 1796.92 | 70.38 | 14.00 | | 1.26 | 1.42 |
| **15** | 1018.82 | 46.97 | 20.00 | | 1168.38 | 52.96 | 16.00 | | 0.87 | 0.89 |
| **16** | 1270.79 | 56.22 | 16.00 | | 1382.61 | 71.49 | 16.00 | | 0.92 | 0.79 |
| **17** | 183.63 | 5.70 | 24.00 | | 1555.20 | 63.67 | 14.00 | | 0.12 | 0.09 |
| **20** | 2275.30 | 101.30 | 20.00 | | 2439.33 | 116.53 | 14.00 | | 0.93 | 0.87 |
| **21** | 1257.39 | 60.38 | 20.00 | | 1339.50 | 64.90 | 12.00 | | 0.94 | 0.93 |
| **22** | 1749.84 | 66.63 | 20.00 | | 2118.35 | 79.93 | 24.00 | | 0.83 | 0.83 |
| **23** | 2201.99 | 91.33 | 24.00 | | 2577.88 | 107.78 | 20.00 | | 0.85 | 0.85 |
| **24** | 1373.21 | 77.04 | 24.00 | | 1575.86 | 65.39 | 20.00 | | 0.87 | 1.18 |
| | | | | | | | | | | |
| **Mean** | **1462.93** | **63.54** | **20.80** | | **1738.64** | **75.02** | **17.10** | | **0.84** | **0.84** |
| **SD** | **543.09** | **27.58** | **3.07** | | **419.16** | **20.30** | **4.18** | | **0.25** | **0.29** |
| **CV** | **37.12** | **43.40** | **14.76** | | **24.11** | **27.07** | **24.44** | | **29.5 6** | **34.89** |
| **Geo Mean** | **1311.89** | **55.00** | **20.57** | | **1691.12** | **72.48** | **16.61** | | **0.78** | **0.76** |
| **Min** | **183.63** | **5.70** | **16.00** | | **1168.38** | **46.10** | **10.00** | | **0.12** | **0.09** |
| **Max** | **2275.30** | **117.77** | **24.00** | | **2577.88** | **116.53** | **24.00** | | **1.26** | **1.42** |

| | | | | | | | | | | |
|---|---|---|---|---|---|---|---|---|---|---|
| | **Fed/ Fasting Ratio** | | | | **Fed / Fasting Ratio (Including Subject #12 and #18 who vomited during post-dose)** | | | | | |
| | | **Geo Means** | **LS Means** % | | | **Geo Means** | **LS Means** % | | | |
| **AUC** | | **0.78** | **77.89** | | **AUC** | **0.75** | **75.27** | | | |
| **Cmax** | | **0.76** | **76.07** | | **Cmax** | **0.76** | **76.25** | | | |

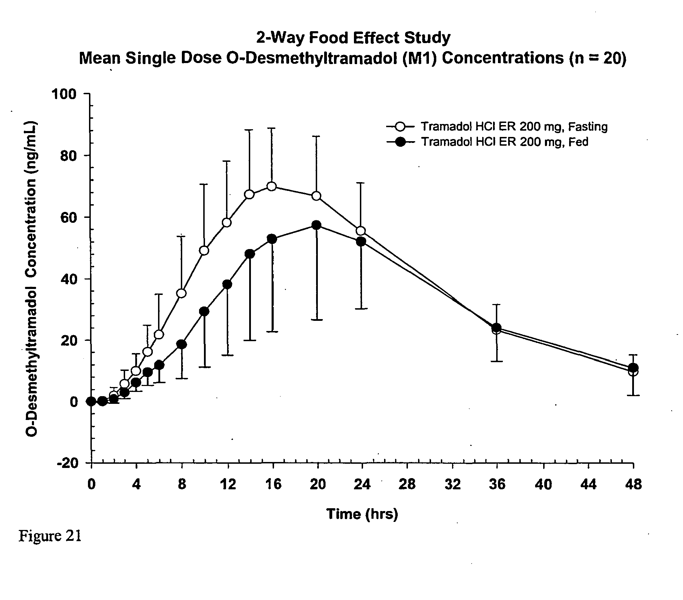

Figure 21 illustrates the mean plasma M1 concentrations (ng/ml) over time after a single dose of one 200 mg Tramadol HCl ER Tablet formulated according to an embodiment of the present invention under fasting or fed conditions.

**Table 25: Summary of Pharmacokinetic Parameters for M5:**

| **M5** | | | | | | | | | | |
|---|---|---|---|---|---|---|---|---|---|---|
| | **Tramadol ER 200 mg, Fed** | | | | **Tramadol ER 200 mg, Fasting** | | | | **Fed/ Fasting** | |
| **Subject** | **AUC** | **Cmax** | **Tmax** | | **AUC** | **Cmax** | **Tmax** | | **AUC** | **Cmax** |
| **1** | 748.53 | 27.50 | 20.00 | | 897.15 | 39.14 | 20.00 | | 0.83 | 0.70 |
| **2** | 511.56 | 16.27 | 24.00 | | 915.79 | 36.24 | 20.00 | | 0.56 | 0.45 |
| **3** | 658.62 | 21.94 | 24.00 | | 678.13 | 22.05 | 24.00 | | 0.97 | 1.00 |
| **4** | 536.16 | 20.64 | 36.00 | | 1281.12 | 58.25 | 20.00 | | 0.42 | 0.35 |
| **5** | 371.79 | 21.38 | 20.00 | | 406.02 | 17.27 | 20.00 | | 0.92 | 1.24 |
| **6** | 851.56 | 38.97 | 20.00 | | 732.06 | 40.97 | 12.00 | | 1.16 | 0.95 |
| **7** | 298.02 | 16.33 | 16.00 | | 535.16 | 21.77 | 16.00 | | 0.56 | 0.75 |
| **8** | 764.80 | 21.96 | 24.00 | | 1100.54 | 38.46 | 24.00 | | 0.69 | 0.57 |
| **9** | 1066.70 | 31.87 | 24.00 | | 1054.70 | 33.20 | 24.00 | | 1.01 | 0.96 |
| **10** | 547.25 | 21.77 | 20.00 | | 674.38 | 26.28 | 16.00 | | 0.81 | 0.83 |
| **11** | 582.46 | 22.11 | 24.00 | | 565.57 | 19.86 | 24.00 | | 1.03 | 1.11 |
| **13** | 446.45 | 19.72 | 16.00 | | 399.37 | 16.12 | 14.00 | | 1.12 | 1.22 |
| **15** | 629.68 | 26.27 | 20.00 | | 746.10 | 30.50 | 16.00 | | 0.84 | 0.86 |
| **16** | 943.41 | 33.73 | 20.00 | | 1024.16 | 44.47 | 16.00 | | 0.92 | 0.76 |
| **17** | 41.47 | 1.49 | 24.00 | | 341.34 | 12.37 | 14.00 | | 0.12 | 0.12 |
| **20** | 939.16 | 36.75 | 20.00 | | 1125.74 | 52.09 | 14.00 | | 0.83 | 0.71 |
| **21** | 599.05 | 26.91 | 20.00 | | 617.38 | 26.32 | 14.00 | | 0.97 | 1.02 |
| **22** | 674.58 | 23.94 | 20.00 | | 782.20 | 28.83 | 16.00 | | 0.86 | 0.83 |
| **23** | 555.68 | 21.00 | 24.00 | | 755.53 | 30.14 | 20.00 | | 0.74 | 0.70 |
| **24** | 644.69 | 24.87 | 24.00 | | 826.43 | 31.25 | 20.00 | | 0.78 | 0.80 |
| | | | | | | | | | | |
| **Mean** | **620.58** | **23.77** | **22.00** | | **772.94** | **31.28** | **18.20** | | **0.81** | **0.80** |
| **SD** | **236.47** | **8.13** | **4.21** | | **259.24** | **11.96** | **3.89** | | **0.25** | **0.28** |
| **CV** | **38.10** | **34.21** | **19.11** | | **33.54** | **38.23** | **21.36** | | **30.6 3** | **35.05** |
| **Geo Mean** | **542.98** | **21.07** | **21.67** | | **728.60** | **29.09** | **17.81** | | **0.75** | **0.72** |
| **Min** | **41.47** | **1.49** | **16.00** | | **341.34** | **12.37** | **12.00** | | **0.12** | **0.12** |
| **Max** | **1066.70** | **38.97** | **36.00** | | **1281.12** | **58.25** | **24.00** | | **1.16** | **1.24** |

| | | | | | | | | | | |
|---|---|---|---|---|---|---|---|---|---|---|
| | **Fed/ Fasting Ratios** | | | | **Fed / Fasting Ratios (Including Subject #12 and #18 who vomited during post-dose)** | | | | | |
| | | **Geo Means** | **LS Means %** | | | **Geo Means** | **LS Means %** | | | |
| **AUC** | | **0.75** | **74.62** | | **AUC** | **0.73** | **73.24** | | | |
| **Cmax** | | **0.72** | **72.23** | | **Cmax** | **0.73** | **73.45** | | | |

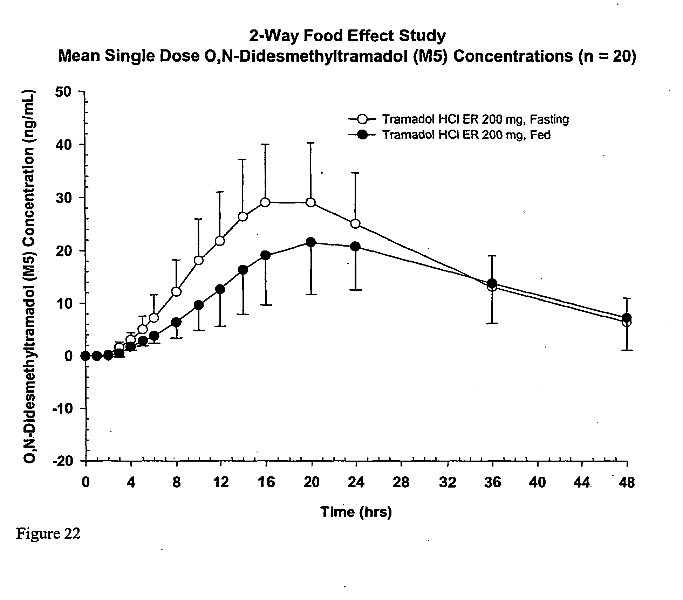

Figure 22 illustrates the mean plasma M5 concentrations (ng/ml) over time after a single dose of one 200 mg Tramadol HCl ER Tablet formulated according to an embodiment of the present invention under fasting or fed conditions.

### EXAMPLE 10: Tramadol ER Osteoarthritis Study

### 10.1: Overall Study Design and plan

A 12-week, multi-center double blind, randomized, dose-titration, parallel-group comparison of the efficacy and safety of Tramadol ER tablets and placebo in the treatment of osteoarthritis of the knee was conducted. Approximately 245 patients from 18 to 75 years of age with moderate to severe pain associated with Functional Class I-III osteoarthritis of the knee were planned for study enrollment to ensure that a minimum of 140 patients completed the study. After signing the informed consent, patients who met the inclusion and exclusion criteria at screening entered a 2 to 7 day washout period during which all analgesic use was discontinued. At the start of the first week of the study (Baseline, Visit 2), eligible patients who reported pain intensity ≥ 40 mm on a visual analog scale (VAS) in the index knee joint were randomly assigned to either Tramadol ER tablets or placebo.

Patients assigned to Tramadol ER tablets were initiated on 100 mg QD and maintained on their dose for at least 3 days. On Day 4, and for the remainder of the week (until their return to the clinic for Visit 3), patients were permitted to have their dose increased to 200 mg QD, based upon the tolerability of side effects. Beginning at Visit 3, patients must have been maintained on a minimum Tramadol ER tablet dose of 200 mg QD, and the dose titrated upwards if required based upon the adequacy of pain relief and tolerability of side effects. Patients randomized to the placebo group underwent sham dose increases. Further dose escalation and de-escalation was permitted provided that a minimum dose of 200 mg QD was maintained from Week 1 (Visit 3) to Week 12 (Visit 7). In patients with pain unresponsive to appropriate dosage adjustments, or with unacceptable side effects, treatment was discontinued and alternate analgesia therapy initiated, as appropriate. Patients returned for efficacy and safety evaluations at Week 1 (Visit 3), Week 2 (Visit 4), Week 4 (Visit 5), Week 8 (Visit 6) and Week 12 (Visit 7) or at Early Termination.

### 10.2: Efficacy Variables

The primary measure of efficacy was the Arthritis Pain Intensity VAS (visual analog scale) Score from patient visits. The arthritis VAS is the most commonly used, validated tool to assess pain intensity and one recommended by FDA to evaluate the analgesic potential of a drug product.

Pain was also assessed as a secondary measure of efficacy using the WOMAC Osteoarthritis Index. The WOMAC is a validated, internationally recognized and widely used multidimensional instrument for assessing response to therapy in osteoarthritis. It assesses pain, joint stiffness and physical function, the three major bothersome symptoms in osteoarthritis. In addition, patients and physicians provided a Global assessment of disease and patients recorded their response on a sleep questionnaire as other secondary measures of efficacy.

### 10.3: Results

A total of 246 patients were randomized and evaluable for safety. Of these, 219 were evaluable for the intent-to-treat (ITT) population. The ITT population included all safety evaluable patients who had primary efficacy information recorded at the baseline visit (Visit 2) and at the Week 1 visit (Visit 3), the first primary efficacy variable collection point on treatment. The ITT population also included all patients who dropped out before the Week 1 visit due to lack of treatment efficacy. The mean daily dose of Tramadol ER following the flexible dosing regimen was approximately 300 mg. The median age of patients who enrolled was 61 years and the median duration of osteoarthritis was 10 years.

Tramadol ER produced statistically significant and clinically meaningful reductions in pain intensity associated with osteoarthritis of the knee compared to placebo for the primary efficacy variable and all secondary variables evaluated.

### 10.4: Response to Primary Variable

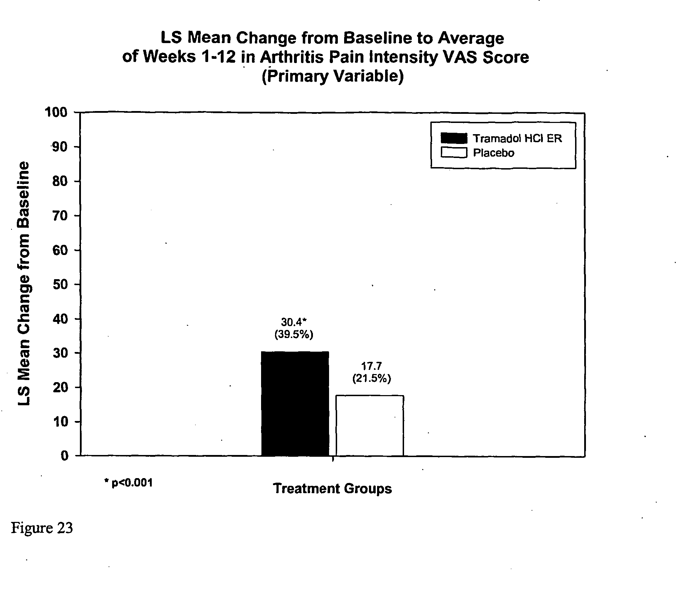

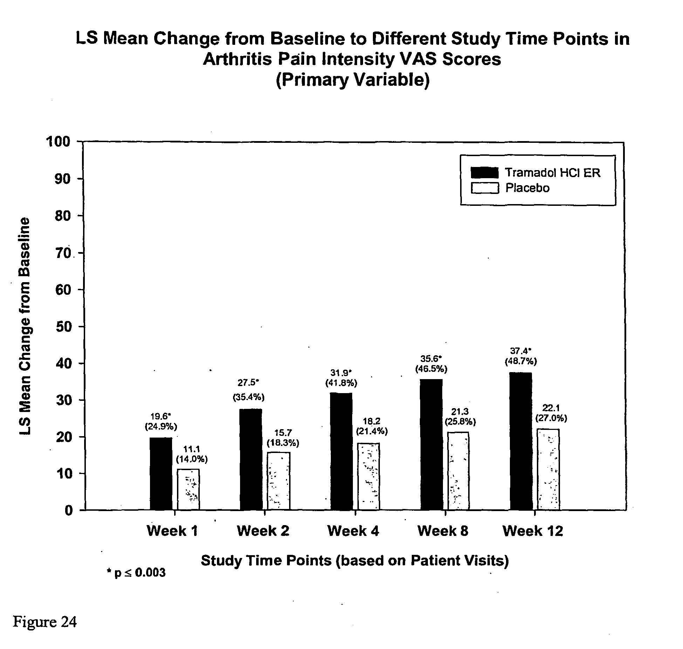

Figure 23 compares the LS mean change from baseline in VAS score for Tramadol ER and placebo based upon the average of Weeks 1-12. On the primary endpoint (LS Mean change from baseline over 12 weeks), there was a 39.5% (30.4 mm) and 21.5% (17.7 mm) change from baseline in the arthritis pain intensity VAS in the Tramadol ER and placebo groups, respectively (LS Mean Difference 12.7 mm, p< 0.001). Figure 24 shows the weekly LS mean changes from baseline for the two treatment groups. Treatment differences emerged at the first return visit (Week 1) when patients were receiving either a 100 mg or 200 mg dose of Tramadol ER (change from baseline 24.8% [19.6 mm] vs. 14.0% [11.1 mm], LS mean difference 8.5 mm, p=0.003). At the end of the second week of treatment, the response to Tramadol ER increased relative to placebo (change from baseline 35.7% [27.4 mm] vs. 19.3% [15.7 mm], LS mean difference 11.7 mm, p< 0.001). By Week 12, the response to Tramadol ER (LS mean change from baseline) was 48.6% [37.4 mm] while that for placebo was 27.0% [22.1 mm]. The LS Mean difference was 15.3 mm, p< 0.001).

### 10.5: Response on the Secondary Variables

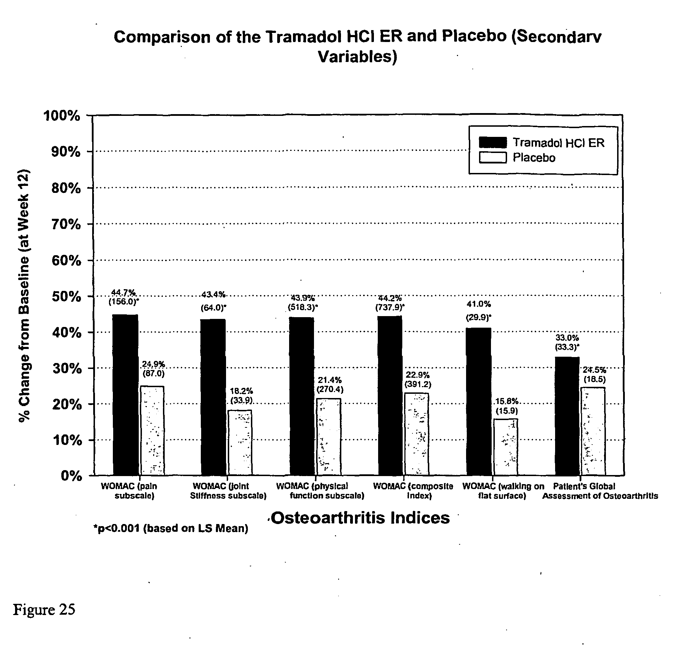

Figure 25 compares the LS mean changes from baseline to Week 12 for the Tramadol ER and placebo for each of the secondary variables.

### 10.5.1: WOMAC Subscales

Results on the three dimensions of the WOMAC, namely pain, stiffness and physical function were similar to the main findings. Tramadol ER tablets was significantly better than placebo in improving pain, stiffness and physical function on the WOMAC.

At Week 12, on the WOMAC Pain Subscale, Tramadol ER was significantly different from placebo (change from baseline 44.6% [155.9 mm] vs. 24.8% [86.9 mm], LS mean difference on 0-100 mm Scale 13.8 mm, p< 0.001).

At Week 12, on the WOMAC Stiffness Subscale, Tramadol ER was significantly different from placebo (change from baseline 43.4% [63.9 mm] vs. 18.1% [33.8 mm], LS mean difference on 0-100 mm Scale 15.0 mm, p< 0.001).

At Week 12, on the WOMAC Physical Function Subscale, Tramadol ER was significantly different from placebo (change from baseline 43.8% [518.3 mm] vs. 21.3% [270.4 mm], LS mean difference on 0-100 mm Scale 14.6 mm, p< 0.001).

At Week 12, on the WOMAC Composite Score, Tramadol ER was significantly different from placebo (change from baseline 42.2% [737.9 mm] vs. 22.8% [391.2 mm], LS mean difference on 0-100 mm Scale 14.4 mm, p< 0.001). 3.5.2: WOMAC Pain Walking on a Flat Surface

In the past, some studies have utilized one item from the WOMAC pain subscale as the primary endpoint. Since some of the questions on the WOMAC subscale relate to walking "up" or "down stairs" (and some areas of the country preferred by the elderly have few stairs, e.g., Arizona, New Mexico, Florida, etc), the WOMAC pain subscale question, "Pain Walking on a Flat Surface" is preferred by some biometricians.

At Week 12, on the WOMAC Pain Walking on Flat Surface, Tramadol ER was significantly different from placebo (change from baseline 40.9% [29.9 mm] vs.15.7% [15.9 mm], LS Mean Difference 14 mm, p< 0.001). This is also shown in Figure 25.

### 10.5.3: Patient Global Assessment of Osteoarthritis

At Week 12, on the Patient Global Assessment of Arthritis, Tramadol ER was significantly different from placebo (change from baseline 33.0% [33.2 mm] vs. 24.4% [18.5 mm], LS Mean Difference 14.7 mm, p< 0.001).

### 10.6: Safety Results

This was a placebo-controlled study without a positive control. Consequently, direct comparison with data on ULTRAM® product are not possible. However, indirect comparisons with the ULTRAM® product package insert are possible. Table 1 provides data on the cumulative incidence of adverse events on ULTRAM® in chronic non-malignant pain. The 90-day comparison is the most appropriate, given the 12 weeks duration of the present study. Adverse events were qualitatively similar. However, the incidence of the most commonly observed adverse events were generally lower for Tramadol ER after up to 90 days compared to only 7 days of treatment with ULTRAM®.

| | | | |
|---|---|---|---|
| **Table 26. Cumulative Incidence of Adverse Reactions for ULTRAM in Chronic Trials of Nonmalignant Pain (N = 427). Data on Tramadol HCl ER from the 12-Week OA Study (N=124) are Provided in Parenthesis for Comparison** | | | |

| | **Up to 7 Days** | **Up to 30 Days** | **Up to 90 Days** |
|---|---|---|---|
| Dizziness/Vertigo | 26% | 31% | 33% (33%) |
| Nausea | 24% | 34% | 40% (24.2%) |
| Constipation | 24% | 38% | 46% (25.8%) |
| Headache | 18% | 26% | 32% (14.5%) |
| Somnolence | 16% | 23% | 25% (8.1%) |
| Vomiting | 9% | 13% | 17% (6.5%) |
| Pruritus | 8% | 10% | 11% (6.5%) |
| "CNS Stimulation" ¹ | 7% | 11% | 14% (TBD) |
| Asthenia | 6% | 11% | 12% (TBD) |
| Sweating | 6% | 7% | 9% (4%) |
| Dyspepsia | 5% | 9% | 13% (1.6%) |
| Dry Mouth | 5% | 9% | 10% (1.6%) |
| Diarrhea | 5% | 6% | 10% (9.7%) |
| ¹ "CNS Stimulation" is a composite of nervousness, anxiety, agitation, tremor, spasticity, euphoria, emotional lability and hallucinations. | | | |

### 10.7: Conclusion:

Tramadol ER produced statistically significant and clinically meaningful reductions in pain associated with osteoarthritis compared to placebo following a flexible dosing regimen in which the once-daily tablet formulation was titrated upward or downward over 12 weeks in doses of 200mg, 300mg or 400mg based upon adequacy of pain relief and tolerability of side effects. The mean daily dose of Tramadol ER was estimated to be about 300mg. The primary efficacy variable was pain relief as measured on a visual analog scale (VAS). Secondary measures of efficacy were the pain intensity, stiffness and physical function subscales of WOMAC Osteoarthritis Index, the Patient's and Physician's Global Assessment of Arthritis, patient withdrawal due to inadequate pain relief, and patient assessment of sleep.

At the end of the first week of treatment and at all subsequent weeks, Tramadol ER was statistically superior to placebo in reducing pain. The magnitude pain improvement (change from baseline) for the Tramadol ER cohort increased weekly throughout the 12 weeks of therapy (25% at Week 1 and 47% at Week 12). By Week 12, patient's treated with Tramadol reported a clinically important 15 mm difference in mean pain relief score compared to placebo. This difference was highly significant (p< 0.001). Based upon the average of Weeks 1 through 12, the Tramadol ER treated patients achieved a highly statistically significant and clinically meaningful 14 mm difference in mean pain relief score compared to placebo (p < 0.001). The results for the secondary variables paralleled those of the primary with all results statistically significant in favor or Tramadol ER.

The adverse events reported were qualitatively similar to that for Ultram. However, the incidence was generally lower for Tramadol ER than for that previously reported for ULTRAM®.

The results of this 12 week placebo controlled study demonstrate that Tramadol ER, when given at a dose of 200 to 400 mg QD, results in significant improvements in the cardinal symptoms of osteoarthritis, namely pain, stiffness and physical function. The safety profile of Tramadol ER is consistent with that for ULTRAM®, although the frequency of some adverse events appears to be lower than historical controls.

### 10.8: Clinical Implications

In the present study, using conventional endpoints for evaluating efficacy, Tramadol ER demonstrated a 30.4 mm and 37.4 mm change from baseline in Arthritis Pain Intensity VAS, when expressed as a mean over 12 weeks (primary endpoint) and at the 12-Week time point, respectively. However, consistent with most such studies, the placebo treatment demonstrates a 17.7 mm and 22.1 mm change from baseline in Arthritis Pain Intensity VAS, when expressed as a mean over 12 weeks (primary endpoint) and at the 12-Week time point, respectively. Consequently, the actual treatment difference (response on Tramadol ER less response on placebo) is a 12.7 mm and 15.3 mm change from baseline in Arthritis Pain Intensity VAS, when expressed as a mean over 12 weeks (primary endpoint) and at the 12-Week time point, respectively.

A close examination of the time course and magnitude of the pharmacological response (on the primary and secondary variables) following treatment with Tramadol ER indicates that this is a clear drug effect: the magnitude of the response increases over time and all of the effects (pain, stiffness, physical function, patient global) are directionally consistent and generally of comparable size.

There are two available benchmarks for determining the robustness of the analgesic response to Tramadol ER in osteoarthritis. One approach involves using the perspectives from the academic rheumatology community. The other involves using the results of pivotal studies in osteoarthritis from recently approved and commercially successful drugs.

In a consensus development (3-round Delphi exercise) involving academic rheumatologists, a 15 mm treatment difference in patients overall assessment of pain was considered to be the minimum clinically important difference (MCID) for clinical trial purposes (Bellamy N, Carette S, Ford PM et al. Osteoarthritis Antirheumatic Drug Trials. II. Tables for calculating sample size for clinical trials. J Rheumatol 1992;19:444-50; Bellamy N, Carette S, Ford PM et al. Osteoarthritis Antirheumatic Drug Trials. III. Delta for Clinical Trials - Results of a Consensus Development (Delphi) Exercise. J Rheumatology 1992; 19:3, 451-457). However, in a recently published study, the minimum clinically perceptible improvement (MCPI) on the three dimensions of WOMAC pain, stiffness and physical function subscale scores (expressed using a 0-100 mm scale) were 9.7, 9.3 and 10 mm, respectively (Beaton DE, Bombardier C, Katz J et al. Looking for important change/difference in studies of responsiveness. J Rheumatol 2001;28;400-405.).

Data from other approved analgesics for which the NDA contained pivotal clinical trials in osteoarthritis were obtained under from the FDA under Freedom of Information (FOI). Although a direct comparison with other drugs is not possible and no drugs exist in the Tramadol class (combined serotonergic, noardrenergic and opioidergic effects), data on other analgesics provide a context for the results of the Tramadol ER study.

Rofecoxib (VIOXX®) is approved for the treatment of osteoarthritis at a daily dose of 12.5 mg; with the comment that some patients may derive a benefit from an increase in does to 25 mg per day (maximum dose). The efficacy studies ' with rofecoxib in osteoarthritis were 6 weeks in duration. The WOMAC variable "pain walking on a flat surface" was used as the primary endpoint. In most cases, the LS mean change from baseline over 6-weeks formed the basis of comparison. The mean difference between rofecoxib 12.5 mg and placebo was 14.3 mm (Study No. 029),12.4 mm (Study No. 033), 15.4 mm (Study No. 040) and 9.0 (Study No. 058). Similarly, the mean difference between the positive control and placebo was 13.5 mm (Ibuprofen, 2400 mg, Study No. 033), 14.6 mm (Ibuprofen, 2400 mg, Study No. 040) and 10 mm (Nabumetone [Relafen®], 1500 mg, Study No. 058).

Celecoxib (CELEBREX®) is approved for the treatment of osteoarthritis at a daily dose of 200 mg. Pivotal clinical trials were placebo controlled studies of either 6 or 12 weeks duration and used naproxen as the positive control. Study No. 020 and 054 served as pivotal clinical trials and Studies 040 and 087 were placebo controlled evaluations of celecoxib 100 mg BID vs. 200 mg QD. There were multiple primary endpoints in each of the studies, including Patients Assessment of Arthritis Pain VAS. The LS mean difference in pain VAS change from baseline between celecoxib 100 mg BID and placebo was 8.0 mm (12 weeks; Study No. 020), 12.2 mm (12 weeks; Study No. 054),13.9 mm (6 weeks; Celecoxib 100 mg BID; Study No. 040) and 13.1 mm (6 weeks; Celecoxib 200 mg QD; Study No. 040), 6.2 mm (6 weeks; Celecoxib 100 mg BID; Study No. 087) and 8.5 mm (6 weeks; Celecoxib 100 mg BID; Study No. 087). Similarly, the mean difference between the positive control and placebo was 7.6 mm (Naproxen 500 mg BID, Study No. 020) and 11.2 mm (Naproxen 500 mg BID, Study No. 054).

The results of the present study demonstrate that Tramadol ER at an approximate dose of 300 mg QD (range 200 to 400 mg) provides a robust analgesic response in OA. The magnitude of the response is at least equal, if not superior to that of NSAIDs and COX-2 inhibitors. With its advantage of once daily dosing, Tramadol ER will be an important addition to the therapeutic armamentarium of clinicians treating chronic pain.

### EXAMPLE 11:

A double-blind, placebo-controlled, parallel-group comparison of the efficacy and safety of 200 mg and 300 mg Tramadol HCl Extended-Release Tablets to placebo in the treatment of chronic low
back pain was conducted.

### 11.1: Study Overview

A multicenter, multiple-dose, randomized, placebo-controlled, parallel-group study involving a minimum of 360 patients designed to compare the analgesic efficacy and safety of extended release tramadol (Tramadol ER) 300 mg and 200 mg orally (PO) once-daily (QD) to placebo in patients with chronic low back pain.

### 11.2: Subjects

Patients with chronic (≥ 6 months) low back pain requiring daily treatment with an analgesic.

### 11.3: Deign

An open-label run-in period followed by a double-blind, randomized, multiple-dose, placebo-controlled study. Patients may roll-over into an ongoing 1-year open-label extension study.

### 11.4: Treatment Regimen:

A 3-week, open-label run-in period, including 2 weeks of dose titration on Tramadol ER, beginning with 100 mg, to attain a tolerable dose of 300 mg QD, followed by 1 week on a stable maintenance dose of Tramadol ER 300 mg QD. Following the run-in period, patients were randomized to one of the following double-blind treatments:
Tramadol ER 300 mg QD at 8:00 A.M.;
Tramadol ER 200 mg QD at 8:00 A.M.;
Placebo QD at 8:00 A.M.

### 11.5: Enrollment Period

5 months

### 11.6: Treatment Period

At Screening (Visit 1), eligible patients underwent laboratory testing, and then entered a 2 to 7 day washout period during which all analgesic use was discontinued. At Visit 2 (Week -3), eligible patients who reported a pain intensity of ≥40 mm on a visual analog scale entered a 3-week, open-label run-in period. Patients were initiated on Tramadol ER 100 mg QD and maintained at their dose for at least 3 days. On Day 4, and for the remainder of the week (until their return to the clinic for Visit 3, Week -2), patients had their dose increased to 200 mg QD, based on the tolerability of side effects. Beginning at Visit 3 (Week - 2), patients were maintained on a minimum Tramadol ER dose of 200 mg QD, and the dose titrated upwards (i.e. to 300 mg QD) based on the tolerability of side effects. Beginning at Visit 4 (Week -1), patients escalated their Tramadol ER dose to 300 mg QD and maintained that dose for 1 week. No further dose adjustments were permitted during the remainder of the run-in period. In patients with pain unresponsive to appropriate dosage adjustments, or with unacceptable side effects, treatment was discontinued and alternate analgesic therapy initiated, as appropriate. Eligible patients receiving Tramadol ER 300 mg QD at the end of the 3-week run-in period were entered into a 12-week, double-blind, randomized study. At Visit 5 (Week 0), patients were randomly assigned to receive Tramadol ER 300 mg QD, Tramadol ER 200 mg QD, or placebo QD. Study medication dosing occurred daily at 8:00 A.M. No dose adjustments were permitted in the double-blind period. Patients unable to tolerate the double-blind study medication and those with unacceptable pain control were dropped from the study. Patients returned for efficacy and safety evaluations at Week 1 (Visit 6), Week 2 (Visit 7), Week 4 (Visit 8), Week 8 (Visit 9), and Week 12 (Visit 10), or Early Termination. Study medication was discontinued at Visit 10 and patients were treated with nonopioid analgesics until they returned to the clinic after 1 week for a post-study medication visit (Visit 11, Week 13). Patients were contacted by telephone between Visit 10 and Visit 11 to ensure that they were not taking opioid analgesics, including tramadol. Visit 11 was scheduled earlier than 1 week after Visit 10, in the event that patient pain could not be managed with nonopioid analgesics, and intervention with opioid analgesics or tramadol was necessary. At Visit 11, patients completed assessments for physical dependence and adverse events.

### 11.7: No. of Centers

30 centers

### 11.8: No. of Subjects

Approximately 600 patients were enrolled to provide a minimum of 360 patients (120 patients per treatment).

### 11.9: Efficacy

At each visit, patients were asked to rate their current pain intensity and their pain intensity since their previous visit, using a visual analog scale (VAS), and they provided an overall global assessment of study medication. The primary efficacy measure was the patient's pain intensity VAS score since the previous visit. Secondary measures included the patient's current pain intensity, the patient's global assessment, the Roland Disability Index, the patient's assessment of sleep and premature study termination due to inefficacy.

### 11.10: Safety

Safety was assessed at each visit by vital signs (heart rate, respiratory rate, supine or sitting and standing blood pressure) and a non-directed adverse events questionnaire. At Screening and at each visit, including Early Termination, patients were evaluated for the occurrence of syncope, orthostasis, dizziness, drop attacks and flushing (vasodilation). Adverse events were monitored throughout the study. Physical examination was performed at Screening (Visit 1) and at the Final Visit (Visit 11), or at Early Termination. Clinical laboratory testing was performed at Screening (Visit 1), at Week -3 (Visit 2), at Baseline (Week 0, Visit 5), at Week 1 (Visit 6), and at Week 12 (Visit 10) or Early Termination. In females of childbearing potential, serum pregnancy tests were performed at Screening (Visit 1), at Week -3 (Visit 2), at Baseline (Week 0, Visit 5), at Week 1 (Visit 6), at Week 4 (Visit 8), at Week 8 (Visit 9), and at Week 12 (Visit 10) or Early Termination. If the Screening (Visit 1) serum pregnancy results were not available from the central laboratory at the start of the run-in period (Visit 2), then the site obtained a urine pregnancy test locally. EKGs were performed at Screening (Visit 1), at Baseline (Week 0, Visit 5) and at Week 12 (Visit 10) or at Early Termination. The Addiction Research Center Inventory (ARCI) Short-form was completed by patients at each visit following the Screening Visit. The Physical Dependence Questionnaire was completed by patients at the start of the run-in period (Visit 2), at Week 12 (Visit 10), and at Week 13 (Visit 11) or at Early Termination.

## Claims

1. A modified release pharmaceutical composition in the form of a tablet for oral administration, suitable for once daily dosing comprising:
(i) a core comprising said at least one form of tramadol selected from the group consisting of tramadol, racemic mixtures thereof, enantiomers thereof, pharmaceutically acceptable salts thereof and combinations thereof and at least one pharmaceutically acceptable excipient; and
(ii) a modified release coating comprising at least one water-insoluble, water-permeable film-forming polymer, at least one plasticizer and at least one water-soluble polymer, wherein the proportion of the at least one water-insoluble, water-permeable film-forming polymer varies from about 20% to about 90% of the coating dry weight, the proportion of the at least one plasticizer varies from about 5% to about 30% of the coating dry weight, and the proportion of the at least one water-soluble polymer varies from about 10% to about 75% of the coating dry weight; and
wherein the composition provides a delayed release of the at least one form of tramadol.

2. A modified release pharmaceutical composition according to claim 1 wherein the composition, when orally administered to a patient, induces a statistically significant lower mean fluctuation index in the plasma than an immediate release composition of the at least one form of tramadol while maintaining bioavailability substantially equivalent to that of the immediate release composition.

3. A modified release pharmaceutical composition according to claim 1 or 2, wherein the composition, when orally administered to a patient, produces a mean maximum plasma concentration (Cₘₐₓ) of the at least one form of tramadol that is lower than that produced by an immediate release pharmaceutical composition of the at least one form of tramadol, and the area under the concentration-time curve (AUC) and the mean minimum plasma concentration (Cₘᵢₙ) are substantially equivalent to that of the immediate release pharmaceutical composition.

4. A modified release pharmaceutical composition according to any one of claims 1 to 3, wherein the composition, when orally administered to a patient, produces a mean maximum plasma concentration (Cₘₐₓ) of the at least one form of tramadol and an area under a plasma concentration vs. time curve (AUC) within the range of from about -20% to about +25% of that produced by an immediate release pharmaceutical composition of the at least one form of tramadol.

5. The pharmaceutical composition according to any preceding claim wherein the at least one form of tramadol is tramadol hydrochloride and the immediate release pharmaceutical composition is the subject of the United States Food and Drug Administration Approved New Drug Application number N20281.

6. A modified release pharmaceutical composition according to any preceding claim, comprising at least one form of tramadol selected from the group consisting of tramadol, enantiomers thereof, pharmaceutically acceptable salts thereof and combinations thereof, wherein the composition exhibits an *in vitro* dissolution profile (measured using the USP Basket Method at 75 rpm in 900 ml 0.1 N HCl at 37° C) such that after 2 hours, from about 0% up to about 30% (by weight) of the at least one form of tramadol is released,
after 4 hours, from about 5% to about 55% (by weight) of the at least one form of tramadol is released, after 12 hours, more than about 50% (by weight) of the at least one form of tramadol is released, and after 24 hours, more than about 80% (by weight) of the at least one form of tramadol is released.

7. A modified release pharmaceutical composition according to claim 6, the composition exhibiting an *in vitro* dissolution profile (measured using the USP Basket Method at 75 rpm in 900 ml 0.1 N HCl at 37° C) such that after 2 hours, from about 0% up to about 30% (by weight) of the at least one form of tramadol is released, after 4 hours, from about 5% to about 22% (by weight) of the at least one form of tramadol is released, after 6 hours, from about 15% to about 38% (by weight) of the at least one form of tramadol is released, after 8 hours, more than about 40% (by weight) of the at least one form of tramadol is released.

8. The modified release pharmaceutical composition of claim 7 wherein the composition exhibits an *in vitro* dissolution profile (measured using the USP Basket Method at 75 rpm in 900 ml 0.1 N HCl at 37° C) such that after 2 hours, from about 2% to about 10% of the at least one form of tramadol is released, after 4 hours, from about 12% to about 20% of the at least one form of tramadol is released, after 6 hours, from about 30% to about 38% of the at least one form of tramadol is released, after 8 hours, from about 48% to about 56% of the at least one form of tramadol is released, after 10 hours, from about 64% to about 72% of the at least one form of tramadol is released, and after 12 hours, more than about 76% of the at least one form of tramadol is released.

9. A modified release pharmaceutical composition according to any preceding claim wherein the pharmaceutical composition, when orally administered to a patient, provides a mean maximum plasma concentration (Cₘₐₓ) of the at least one form of tramadol from about 80 ng/ml to about 500 ng/ml.

10. A modified release pharmaceutical composition according to any preceding claim, wherein the pharmaceutical composition, when orally administered to a patient, provides a time to mean maximum plasma concentration (Tₘₐₓ) of the at least one form of tramadol ranging from about 4 hours to about 14 hours.

11. A modified release pharmaceutical composition according to any preceding claim wherein the pharmaceutical composition, when orally administered to a patient, provides a plasma concentration time curve with an area under the curve ranging from about 1000 ng. hr/ml to about 10000 ng. hr/ml.

12. The modified release pharmaceutical composition according to any preceding claim, wherein the at least one water-insoluble, water-permeable film-forming polymer is ethylcellulose.

13. The modified release pharmaceutical composition according to any preceding claim, wherein the at least one water-soluble polymer is polyvinylpyrrolidone.

14. The modified release pharmaceutical composition according to any preceding claim, wherein the at least one plasticizer is dibutyl sebacate.

15. The modified release pharmaceutical composition according to any preceding claim, wherein the at least one water-insoluble, water-permeable film-forming polymer is ethylcellulose, the at least one water-soluble polymer is polyvinylpyrrolidone and the at least one plasticizer is dibutyl sebacate.

16. The modified release pharmaceutical composition according to any preceding claim, wherein the at least one pharmaceutically acceptable excipient in the core is selected from the group consisting of at least one lubricant, at least one binder, at least one glidant and combinations thereof.

17. The modified release pharmaceutical composition of claim 17, wherein the at least one lubricant is sodium stearyl fumarate, the at least one binder is polyvinyl alcohol, and the at least one glidant is colloidal silicon dioxide.

18. A modified release pharmaceutical composition according to any preceding claim comprising:
(i) a core comprising tramadol hydrochloride, polyvinyl alcohol, colloidal silicon dioxide and sodium stearyl fumarate; and
(ii) a coating comprising ethylcellulose, polyvinylpyrrolidone and dibutyl sebacate.

19. The modified release pharmaceutical composition according to any preceding claim, wherein the at least one form of tramadol is tramadol hydrochloride and wherein the tramadol hydrochloride is present in an amount of from about 50 mg to about 400 mg.

20. The modified release pharmaceutical composition according to any preceding claim, wherein the composition is in the form of a tablet.

21. The composition according to any preceding claim, the composition having a delayed release whereby drug release is substantially prevented in the upper part of the gastrointestinal tract.

22. The composition according to any preceding claim, the composition having a delayed release that results in a plasma concentration of the at least one form of tramadol of about 0 ng/ml at 1 hr.

## Patentansprüche

1. Eine pharmazeutische Zusammensetzung mit modifizierter Freisetzung in Form einer Tablette zur oralen Verabreichung, die sich zur einmal täglichen Dosierung eignet, umfassend:
(i) einen Kern, der die wenigstens eine Form von Tramadol, ausgewählt aus der Gruppe, bestehend aus Tramadol, racemischen Mischungen davon, Enantiomeren davon, pharmazeutisch annehmbaren Salzen davon und Kombinationen davon, und wenigstens einen pharmazeutisch annehmbaren Hilfsstoff enthält, und
(ii) eine Beschichtung für eine modifizierte Freisetzung, die wenigstens ein wasserunlösliches wasserpermeables filmbildendes Polymer, wenigstens ein Plastifizierungsmittel und wenigstens ein wasserlösliches Polymer enthält, wobei der Anteil an dem wenigstens einen wasserunlöslichen wasserpermeablen filmbildenden Polymer von etwa 20% bis etwa 90% des Trockengewichts der Beschichtung variiert, wobei der Anteil an dem wenigstens einen Plastifizierungsmittel von etwa 5% bis etwa 30% des Trockengewichts der Beschichtung variiert, und wobei der Anteil an dem wenigstens einen wasserlöslichen Polymer von etwa 10% bis etwa 75% des Trockengewichts der Beschichtung variiert, und
wobei die Zusammensetzung für eine verzögerte Freisetzung der wenigstens einen Form von Tramadol sorgt.

2. Eine pharmazeutische Zusammensetzung mit modifizierter Freisetzung gemäß Anspruch 1, wobei die Zusammensetzung, wenn sie oral an einen Patienten verabreicht wird, einen statistisch signifikant niedrigeren mittleren Fluktuationsindex im Plasma hervorruft als eine Zusammensetzung mit sofortiger Freisetzung der wenigstens einen Form von Tramadol, wobei eine Bioverfügbarkeit entsprechend im Wesentlichen der der Zusammensetzung mit sofortiger Freisetzung beibehalten wird.

3. Eine pharmazeutische Zusammensetzung mit modifizierter Freisetzung gemäß Anspruch 1 oder 2, wobei die Zusammensetzung, wenn sie oral an einen Patienten verabreicht wird, eine mittlere maximale Plasmakonzentration (Cₘₐₓ) der wenigstens einen Form von Tramadol erzeugt, die niedriger ist als diejenige, die durch eine pharmazeutische Zusammensetzung mit sofortiger Freisetzung der wenigstens einen Form von Tramadol erzeugt wird, und wobei die Fläche unter der Konzentrations-Zeit-Kurve (AUC) und die mittlere minimale Plasmakonzentration (Cₘᵢₙ) im Wesentlichen denen der pharmazeutischen Zusammensetzung mit sofortiger Freisetzung entsprechen.

4. Eine pharmazeutische Zusammensetzung mit modifizierter Freisetzung gemäß einem der Ansprüche 1 bis 3, wobei die Zusammensetzung, wenn sie oral an einen Patienten verabreicht wird, eine mittlere maximale Plasmakonzentration (Cₘₐₓ) der wenigstens einen Form von Tramadol und eine Fläche unter einer Plasmakonzentrations-Zeit-Kurve (AUC) erzeugt, die innerhalb des Bereichs von etwa -20% bis etwa +25% der von einer pharmazeutischen Zusammensetzung mit sofortiger Freisetzung der wenigstens einen Form von Tramadol erzeugten Werte liegen.

5. Die pharmazeutische Zusammensetzung gemäß einem vorhergehenden Anspruch, wobei die wenigstens eine Form von Tramadol Tramadol-Hydrochlorid ist und die pharmazeutische Zusammensetzung mit sofortiger Freisetzung der Gegenstand der United-States-Food-and-Drug-Administration-Approved-New-Drug-Application-Nummer N20281 ist.

6. Eine pharmazeutische Zusammensetzung mit modifizierter Freisetzung gemäß einem vorhergehenden Anspruch, umfassend wenigstens eine Form von Tramadol, ausgewählt aus der Gruppe, bestehend aus Tramadol, Enantiomeren davon, pharmazeutisch annehmbaren Salzen davon und Kombinationen davon, wobei die Zusammensetzung ein *In-vitro*-Auflösungsprofil (gemessen mit Hilfe des USP-Basket-Verfahrens bei 75 U/Minute in 900 ml 0,1N HCl bei 37°C) aufweist, das derart ist, dass nach 2 Stunden etwa 0% bis etwa 30% (bezogen auf das Gewicht) der wenigstens einen Form von Tramadol freigesetzt wurden, nach 4 Stunden etwa 5% bis etwa 55% (bezogen auf das Gewicht) der wenigstens einen Form von Tramadol freigesetzt wurden, nach 12 Stunden mehr als etwa 50% (bezogen auf das Gewicht) der wenigstens einen Form von Tramadol freigesetzt wurden, und nach 24 Stunden mehr als etwa 80% (bezogen auf das Gewicht) der wenigstens einen Form von Tramadol freigesetzt wurden.

7. Eine pharmazeutische Zusammensetzung mit modifizierter Freisetzung gemäß Anspruch 6, wobei die Zusammensetzung ein *In-vitro*-Auflösungsprofil (gemessen mit Hilfe des USP-Basket-Verfahrens bei 75 U/Minute in 900 ml 0,1N HCl bei 37°C) aufweist, das derart ist, dass nach 2 Stunden etwa 0% bis etwa 30% (bezogen auf das Gewicht) der wenigstens einen Form von Tramadol freigesetzt wurden, nach 4 Stunden etwa 5% bis etwa 22% (bezogen auf das Gewicht) der wenigstens einen Form von Tramadol freigesetzt wurden, nach 6 Stunden etwa 15% bis etwa 38% (bezogen auf das Gewicht) der wenigstens einen Form von Tramadol freigesetzt wurden, nach 8 Stunden mehr als etwa 40% (bezogen auf das Gewicht) der wenigstens einen Form von Tramadol freigesetzt wurden.

8. Die pharmazeutische Zusammensetzung mit modifizierter Freisetzung nach Anspruch 7, wobei die Zusammensetzung ein *In-vitro*-Auflösungsprofil (gemessen mit Hilfe des USP-Basket-Verfahrens bei 75 U/Minute in 900 ml 0,1N HCl bei 37°C) aufweist, das derart ist, dass nach 2 Stunden etwa 2% bis etwa 10% der wenigstens einen Form von Tramadol freigesetzt wurden, nach 4 Stunden etwa 12% bis etwa 20% der wenigstens einen Form von Tramadol freigesetzt wurden, nach 6 Stunden etwa 30% bis etwa 38% der wenigstens einen Form von Tramadol freigesetzt wurden, nach 8 Stunden etwa 48% bis etwa 56% der wenigstens einen Form von Tramadol freigesetzt wurden, nach 10 Stunden etwa 64% bis etwa 72% der wenigstens einen Form von Tramadol freigesetzt wurden, und nach 12 Stunden mehr als etwa 76% der wenigstens einen Form von Tramadol freigesetzt wurden.

9. Eine pharmazeutische Zusammensetzung mit modifizierter Freisetzung gemäß einem vorhergehenden Anspruch, wobei die pharmazeutische Zusammensetzung, wenn sie oral an einen Patienten verabreicht wird, eine mittlere maximale Plasmakonzentration (Cₘₐₓ der wenigstens einen Form von Tramadol von etwa 80 ng/ml bis etwa 500 ng/ml bereitstellt.

10. Eine pharmazeutische Zusammensetzung mit modifizierter Freisetzung gemäß einem vorhergehenden Anspruch, wobei die pharmazeutische Zusammensetzung, wenn sie oral an einen Patienten verabreicht wird, einen Zeitraum bis zur mittleren maximalen Plasmakonzentration (Tₘₐₓ) der wenigstens einen Form von Tramadol im Bereich von etwa 4 Stunden bis etwa 14 Stunden bereitstellt.

11. Eine Zusammensetzung mit modifizierter Freisetzung gemäß einem vorhergehenden Anspruch, wobei die pharmazeutische Zusammensetzung, wenn sie oral an einen Patienten verabreicht wird, eine Plasmakonzentrations-Zeit-Kurve mit einer Fläche unter der Kurve im Bereich von etwa 1000 ng.h/ml bis etwa 10000 ng.h/ml bereitstellt.

12. Die pharmazeutische Zusammensetzung mit modifizierter Freisetzung gemäß einem vorhergehenden Anspruch, wobei das wenigstens eine wasserunlösliche wasserpermeable filmbildende Polymer Ethylcellulose ist.

13. Die pharmazeutische Zusammensetzung mit modifizierter Freisetzung gemäß einem vorhergehenden Anspruch, wobei das wenigstens eine wasserlösliche Polymer Polyvinylpyrrolidon ist.

14. Die pharmazeutische Zusammensetzung mit modifizierter Freisetzung gemäß einem vorhergehenden Anspruch, wobei das wenigstens eine Plastifizierungsmittel Dibutylsebacat ist.

15. Die pharmazeutische Zusammensetzung mit modifizierter Freisetzung gemäß einem vorhergehenden Anspruch, wobei das wenigstens eine wasserunlösliche wasserpermeable filmbildende Polymer Ethylcellulose ist, das wenigstens eine wasserlösliche Polymer Polyvinylpyrrolidon ist und das wenigstens eine Plastifizierungsmittel Dibutylsebacat ist.

16. Die pharmazeutische Zusammensetzung mit modifizierter Freisetzung gemäß einem vorhergehenden Anspruch, wobei der wenigstens eine pharmazeutisch annehmbare Hilfsstoff in dem Kern ausgewählt ist aus der Gruppe, bestehend aus wenigstens einem Gleitmittel, wenigstens einem Bindemittel, wenigstens einem Fließregulierungsmittel und Kombinationen davon.

17. Die pharmazeutische Zusammensetzung mit modifizierter Freisetzung gemäß Anspruch 17, wobei das wenigstens eine Gleitmittel Natriumstearylfumarat ist, das wenigstens eine Bindemittel Polyvinylalkohol ist und das wenigstens eine Fließregulierungsmittel kolloidales Siliciumdioxid ist.

18. Eine pharmazeutische Zusammensetzung mit modifizierter Freisetzung gemäß einem vorhergehenden Anspruch, umfassend:
(i) einen Kern, der Tramadol-Hydrochlorid, Polyvinylalkohol, kolloidales Siliciumdioxid und Natriumstearylfumarat enthält, und
(ii) eine Beschichtung, die Ethylcellulose, Polyvinylpyrrolidon und Dibutylsebacat enthält.

19. Die pharmazeutische Zusammensetzung mit modifizierter Freisetzung gemäß einem vorhergehenden Anspruch, wobei die wenigstens eine Form von Tramadol Tramadol-Hydrochlorid ist, und wobei das Tramadol-Hydrochlorid in einer Menge von etwa 50 mg bis etwa 400 mg vorliegt.

20. Die pharmazeutische Zusammensetzung mit modifizierter Freisetzung gemäß einem vorhergehenden Anspruch, wobei die Zusammensetzung in Form einer Tablette vorliegt.

21. Die Zusammensetzung gemäß einem vorhergehenden Anspruch, wobei die Zusammensetzung eine verzögerte Freisetzung besitzt, wodurch die Arzneimittelfreisetzung im Wesentlichen im oberen Teil des Gastrointestinaltraktes verhindert wird.

22. Die Zusammensetzung gemäß einem vorhergehenden Anspruch, wobei die Zusammensetzung eine verzögerte Freisetzung besitzt, die zu einer Plasmakonzentration der wenigstens einen Form von Tramadol von etwa 0 ng/ml bei 1 Stunde führt.

## Revendications

1. Composition pharmaceutique à libération modifiée sous forme de comprimé pour l'administration orale, appropriée pour une administration une fois par jour, comprenant:
(i) un noyau refermant ladite au moins une forme de tramadol choisie dans le groupe constitué par le tramadol, les mélanges racémiques de celui-ci, ses énantiomères, leurs sels acceptables sur le plan pharmaceutique et leurs combinaisons, et au moins un excipient acceptable sur le plan pharmaceutique; et
(ii) un enrobage à libération modifiée renfermant au moins un polymère filmogène insoluble dans l'eau, perméable à l'eau, au moins un plastifiant et au moins un polymère soluble dans l'eau,
où la proportion dudit au moins un polymère filmogène insoluble dans l'eau, perméable à l'eau varie entre environ 20% et environ 90% du poids sec de l'enrobage, la proportion dudit au moins un plastifiant varie entre environ 5% et environ 30% du poids sec de l'enrobage, et la proportion dudit au moins un polymère soluble dans l'eau varie entre environ 10% et environ 75% du poids sec de l'enrobage; et
où la composition permet une libération retardée de ladite au moins une forme de tramadol.

2. Composition pharmaceutique à libération modifiée selon la revendication 1, où la composition induit, lorsqu'elle est administrée par voie orale à un patient, un indice de fluctuation moyenne statistiquement beaucoup plus faible dans le plasma qu'une composition à libération immédiate de ladite au moins une forme de tramadol tout en gardant une biodisponibilité sensiblement équivalente à celle de la composition à libération immédiate.

3. Composition pharmaceutique à libération modifiée selon la revendication 1 ou 2, où la composition produit, lorsqu'elle est administrée par voie orale, une concentration plasmatique maximale moyenne (Cₘₐₓ) de ladite au moins une forme de tramadol qui est inférieure à celle produite par une composition pharmaceutique à libération immédiate de ladite au moins une forme de tramadol, et l'aire sous la courbe de concentration en fonction du temps (AUC) et la concentration plasmatique minimale moyenne (Cₘᵢₙ) sont sensiblement équivalentes à celle de la composition pharmaceutique à libération immédiate.

4. Composition pharmaceutique à libération modifiée selon l'une quelconque des revendications 1 à 3, où la composition produit, lorsqu'elle est administrée par voie orale à un patient, une concentration plasmatique maximale moyenne (Cₘₐₓ) de ladite au moins une forme de tramadol et une aire sous la courbe de concentration plasmatique en fonction du temps (AUC) dans l'intervalle d'environ -20% à environ +25% de celles produites par une composition pharmaceutique à libération immédiate de ladite au moins une forme de tramadol.

5. Composition pharmaceutique selon l'une quelconque des revendications précédentes, dans laquelle ladite au moins une forme de tramadol est le chlorhydrate de tramadol et la composition pharmaceutique à libération immédiate est l'objet de la United States Food and Drug Administration Approved New Drug Application numéro N20281.

6. Composition pharmaceutique à libération modifiée selon l'une quelconque des revendications précédentes, comprenant au moins une forme de tramadol choisie dans le groupe constitué par le tramadol, ses énantiomères, leurs sels acceptables sur le plan pharmaceutique et leurs combinaisons, où la composition présente un profil de dissolution in vitro (mesuré à l'aide de la méthode utilisant un support-panier de l'USP à 75 tours/min dans 900 ml de HCl 0,1N à 37°C) tel qu'au bout de 2 heures, environ 0% à environ 30% (en poids) de ladite au moins une forme de tramadol est libérée, au bout de 4 heures, environ 5% à environ 55% (en poids) de ladite au moins une forme de tramadol est libérée, au bout de 12 heures, plus d'environ 50% (en poids) de ladite au moins une forme de tramadol est libérée, et au bout de 24 heures, plus d'environ 80% (en poids) de ladite au moins une forme de tramadol est libérée.

7. Composition pharmaceutique à libération modifiée selon la revendication 6, ladite composition présentant un profil de dissolution in vitro (mesuré à l'aide de la méthode utilisant un support-panier de l'USP à 75 tours/min dans 900 ml de HCl 0,1N à 37°C) tel qu'au bout de 2 heures, environ 0% à environ 30% (en poids) de ladite au moins une forme de tramadol est libérée, au bout de 4 heures, environ 5% à environ 22% (en poids) de ladite au moins une forme de tramadol est libérée, au bout de 6 heures, environ 15% à environ 38% (en poids) de ladite au moins une forme de tramadol est libérée, et au bout de 8 heures, plus d'environ 40% (en poids) de ladite au moins une forme de tramadol est libérée.

8. Composition pharmaceutique à libération modifiée selon la revendication 7, ladite composition présentant un profil de dissolution in vitro (mesuré à l'aide de la méthode utilisant un support-panier de l'USP à 75 tours/min dans 900 ml de HCl 0,1N à 37°C) tel qu'au bout de 2 heures, environ 2% à environ 10% de ladite au moins une forme de tramadol est libérée, au bout de 4 heures, environ 12% à environ 20% de ladite au moins une forme de tramadol est libérée, au bout de 6 heures, environ 30% à environ 38% de ladite au moins une forme de tramadol est libérée, au bout de 8 heures, environ 48% à environ 56% de ladite au moins une forme de tramadol est libérée, au bout de 10 heures, environ 64% à environ 72% de ladite au moins une forme de tramadol est libérée, et au bout de 12 heures, plus d'environ 76% de ladite au moins une forme de tramadol est libérée.

9. Composition pharmaceutique à libération modifiée selon l'une quelconque des revendications précédentes, où ladite composition pharmaceutique procure, lorsqu'elle est administrée par voie orale à un patient, une concentration plasmatique maximale moyenne (Cₘₐₓ) de ladite au moins une forme de tramadol d'environ 80 ng/ml à environ 500 ng/ml.

10. Composition pharmaceutique à libération modifiée selon l'une quelconque des revendications précédentes, où ladite composition pharmaceutique procure, lorsqu'elle est administrée par voie orale à un patient, un temps jusqu'à la concentration plasmatique maximale moyenne (Tₘₐₓ₎ de ladite au moins une forme de tramadol allant de 4 heures environ à 14 heures environ.

11. Composition pharmaceutique à libération modifiée selon l'une quelconque des revendications précédentes, où la composition pharmaceutique permet d'obtenir, lorsqu'elle est administrée par voie orale à un patient, une courbe de la concentration plasmatique en fonction du temps avec une aire sous la courbe allant d'environ 1000 ng.h/ml à environ 10 000 ng.h/ml.

12. Composition pharmaceutique à libération modifiée selon l'une quelconque des revendications précédentes, dans laquelle ledit au moins un polymère filmogène insoluble dans l'eau, perméable à l'eau est l'éthylcellulose.

13. Composition pharmaceutique à libération modifiée selon l'une quelconque des revendications précédentes, dans laquelle ledit au moins un polymère soluble dans l'eau est la polyvinylpyrrolidone.

14. Composition pharmaceutique à libération modifiée selon l'une quelconque des revendications précédentes, dans laquelle ledit au moins un plastifiant est le sébaçate de dibutyle.

15. Composition pharmaceutique à libération modifiée selon l'une quelconque des revendications précédentes, dans laquelle ledit au moins un polymère filmogène insoluble dans l'eau, perméable à l'eau est l'éthylcellulose, ledit au moins un polymère soluble dans l'eau est la polyvinylpyrrolidone et ledit au moins un plastifiant est le sébaçate de dibutyle.

16. Composition pharmaceutique à libération modifiée selon l'une quelconque des revendications précédentes, dans laquelle ledit au moins un excipient acceptable sur le plan pharmaceutique présent dans le noyau est choisi dans le groupe constitué par au moins un lubrifiant, au moins un liant, au moins un agent de glissement et leurs combinaisons.

17. Composition pharmaceutique à libération modifiée selon la revendication 17, dans laquelle ledit au moins un lubrifiant est le stéarylfumarate de sodium, ledit au moins un liant est l'alcool polyvinylique, et ledit au moins un agent de glissement est le dioxyde de silicium colloïdal.

18. Composition pharmaceutique à libération modifiée selon l'une quelconque des revendications précédentes, comprenant:
(i) un noyau renfermant du chlorhydrate de tramadol, de l'alcool polyvinylique, du dioxyde de silicium colloïdal et du stéarylfumarate de sodium; et
(ii) un enrobage renfermant de l'éthylcellulose, de la polyvinylpyrrolidone et du sébaçate de dibutyle.

19. Composition pharmaceutique à libération modifiée selon l'une quelconque des revendications précédentes, dans laquelle ladite au moins une forme de tramadol est le chlorhydrate de tramadol et dans laquelle le chlorhydrate de tramadol est présent en une quantité d'environ 50 mg à environ 400 mg.

20. Composition pharmaceutique à libération modifiée selon l'une quelconque des revendications précédentes, où la composition se présente sous forme de comprimé.

21. Composition selon l'une quelconque des revendications précédentes, ladite composition ayant une libération retardée ce qui fait que la libération du médicament est substantiellement empêchée dans la partie supérieure du tractus gastro-intestinal.

22. Composition selon l'une quelconque des revendications précédentes, ladite composition ayant une libération retardée qui se traduit par une concentration plasmatique de ladite au moins une forme de tramadol d'environ 0 ng/ml après 1 h.
